# EUROPEAN PATENT APPLICATION

(11) **EP 3 909 951 A1**
(43) Date of publication of application: **17.11.2021**
(21) Application number: 20739024.6
(22) Date of filing: 07.01.2020
(51) Int. Cl.: C07D 455/06, A61K 31/4375, A61P 31/12, A61P 31/20

(54) **DIHYDROISOQUINOLINE COMPOUND**

(30) Priority: 08.01.2019 CN 201910016095
(71) Applicant: Suzhou Ark Biopharmaceutical Co., Ltd, Suzhou, Jiangsu 215123 (CN)
(72) Inventor: PENG, Cheng, Suzhou, Jiangsu 215123 (CN); ZHANG, Shaoyun, Suzhou, Jiangsu 215123 (CN); LAI, Xiaogang, Suzhou, Jiangsu 215123 (CN); GONG, Chaojun, Suzhou, Jiangsu 215123 (CN); HAN, Jian, Suzhou, Jiangsu 215123 (CN); XU, Baolin, Suzhou, Jiangsu 215123 (CN); ZOU, Gang, Suzhou, Jiangsu 215123 (CN); LI, Danbin, Suzhou, Jiangsu 215123 (CN); YUAN, Haiqing, Suzhou, Jiangsu 215123 (CN); WU, JIM Zhen, Suzhou, Jiangsu 215123 (CN)
(74) Representative: Perani & Partners S.p.A.
(86) International application number: PCT/CN2020/070633
(87) International publication number: WO 2020/143604

(57) **Abstract**

The present disclosure provides a series of dihydroisoquinoline compounds or a pharmaceutically acceptable salt, ester, isomer, solvate, hydrate, prodrug, or isotopically labeled compounds thereof, having the structure of general formula I: (I). The compounds have an extremely strong activity for inhibiting Hepatitis B surface antigen and have an extremely strong activity for inhibiting Hepatitis B virus DNA. In addition, the compounds feature high bioavailability and can exhibit efficacy with a low dosage, which reduce the potential toxicity of the compounds.

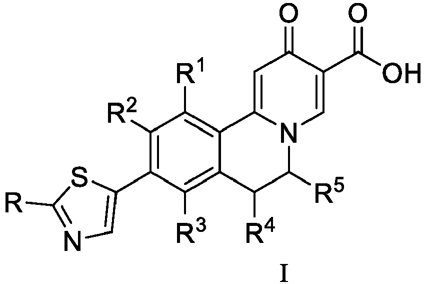

## Description

### CROSS-REFERENCE TO RELATED APPLICATION

The present disclosure claims the priority of the patent application of the invention named "DIHYDROISOQUINOLINE COMPOUND" (Application No. 201910016095.9) filed in China on January 8, 2019, the content of which is incorporated herein by reference in its entirety.

### TECHNICAL FIELD

The present disclosure belongs to the field of medicinal chemistry, specifically relates to a thiazole ring-substituted dihydroisoquinoline compound and a mixture or composition comprising the dihydroisoquinoline compound, especially a thiazole ring-substituted dihydroisoquinoline compound for preventing and treating hepatitis B virus infection and a mixture or composition comprising the dihydroisoquinoline compound.

### BACKGROUND

Hepatitis B virus (HBV) is one of the most common pathogens in the world. Viral hepatitis B is an infectious disease caused by hepatitis B virus (HBV), spread via blood and body fluid and mainly causing liver damage. It is a serious public health issue and poses a great threat to human health. According to statistics, approximately one-third of the world's population has been infected with hepatitis B virus, among which 350 to 500 million people are chronic hepatitis B virus (HBV) carriers. Hepatitis B virus infection may not only cause acute hepatitis, but may also result in chronic hepatitis, induce liver cirrhosis and eventually lead to liver cancer. HBV may infect human body via a variety of ways, thus causing the spread of liver disease(s). The number of deaths caused by acute or chronic hepatitis B virus infection is approximately 800,000 each year.

Although a safe and effective HBV vaccine has been launched and the widespread neonatal vaccination has also brought about the dramatic drop of the infection rate and even the incidence rate of liver cancer, many infected patients cannot be diagnosed and treated in time and the global burden of HBV-associated disease is still heavy due to factors such as insufficient coverage rate of vaccine and economic constraints in some regions. In recent years, great progress has been achieved in the treatment and prevention of viral hepatitis B with the in-depth research on the suppression of hepatitis B virus infection and the application of new diagnosis and treatment. However, a complete cure of hepatitis B virus infection has not yet been achieved, and there remains tremendous medical demand.

Hepatitis B virus is a circular double-stranded DNA virus comprising some single-stranded regions, its genome is about 3.2 kb in length, and hepatitis B virus belongs to hepadnavividae. The nucleotide sequence heterogeneity of the whole HBV gene is approximately 8%. Taking a nucleotide sequence heterogeneity of the S gene region of 4.2% as a standard, the genotypes of HBV may be classified into 8 types, i.e., A to H. The distribution of the genotypes of HBV has racial and geographic differences. Genotypes B and C are mainly distributed in Asia, genotypes A and D are often distributed in Africa, Europe and the Indian Peninsula, genotype E is distributed in West Africa, genotype F is distributed in Central America and South America, and genotype G is often distributed in France, Germany and North America. The genome of hepatitis B virus comprises four open reading frames encoding the core protein, polymerase, membrane protein and X protein. The infection of hepatitis B virus relies on the sodium ion-taurocholic acid-cotransporter (NTCP) receptor on liver cell membrane. After a liver cell is infected with hepatitis B virus, partially double-stranded viral DNA is transported to the cell nucleus and forms a covalently closed circular DNA (cccDNA), and cccDNA is the transcription template of the pregenomic RNA (pgRNA) and the subgenomic RNA of hepatitis B virus. The core protein encapsidates pgRNA and polymerase together for replication (Lamontagne RJ, et al. Hepatoma Res 2016; 2: 163-86).

Hepatitis B virus has a 7 nm-thick outer membrane protein layer comprising phospholipids, which is referred to as hepatitis B surface antigen (HBsAg). The membrane proteins are classified into three types (i.e., large, middle, small HBsAg). They are encoded by the S gene of HBV while having different transcription start sites. The C-terminal of the S region is the same. The membrane proteins are integrated into the endoplasmic reticulum membrane under the guidance of the N-terminal signal sequence. On the one hand, the membrane proteins will become an important structural protein of a mature hepatitis B virus particle; on the other hand, the membrane proteins will form spherical or filamentous subviral particles (SVPs). In the serum of the patient, the content of subviral particles is usually more than 1000 times that of mature virus particles. In the serological test, hepatitis B surface antigen being positive for more than 6 months is a sign of chronic HBV infection. Although the subviral particles are not infectious, they exert an important impact on the immune response of the host. Hepatitis B surface antigen may inhibit the activation of monocytes in the innate immune system (Vanlandschoot P et al, J Gen Virol. 2002 Jun; 83(Pt 6): 1281-9), destroy the function of dendritic cells (Marjoleine L et al, Immunology. 2009 Feb; 126(2): 280-289), and destroy the function of natural killer cells (Yang Y et al, Int Immunopharmacol. 2016 Sep; 38: 291-7). Persistent exposure to hepatitis B surface antigen and other viral antigens also results in the loss of the function of HBV-specific T cells, thereby causing the immune tolerance of the host to hepatitis B virus (Carolina B et al, J Virol. 2007 Apr; 81(8): 4215-4225). Surface antigen clearance in hepatitis B patients is an important biomarker for clinical diagnosis and treatment. Serological clearance of hepatitis B surface antigen is considered as a clinical cure for viral hepatitis B.

Drugs currently approved by the FDA for clinical use include interferons and nucleoside drugs. Interferons include ordinary interferon and pegylated interferons. Nucleoside drugs include lamivudine (Heptodin), adefovir (Hepsera), entecavir (Baraclude) and telbivudine (Sebivo), and tenofovir (Viread). Interferon is capable of activating immune cells and inducing the production of a variety of antiviral factors by regulating the host immune system to achieve the control of HBV. Interferon may also improve the immunity of the body while resisting the virus infection, its effect is relatively long-lasting, and the incidence rate of liver cirrhosis and liver cancer may also be reduced. The disadvantages of interferon lie in that, the antiviral effect is not strong and is accompanied by many side effects, and may cause symptoms such as flu-like symptoms, muscle pain, thrombocytopenia, hair loss and depression. Long-term interferon therapy is only capable of achieving hepatitis B surface antigen clearance in 2.25% of the population in Western countries and 0.43% of the population in Asian countries each year (CM Chun et al, Antivir Ther. 2010; 15(2): 133-43). Nucleoside drugs are capable of inhibiting the synthesis of HBV DNA. Lamivudine was once used as a drug for treating AIDS. It is absorbed rapidly after oral administration, and is capable of significantly inhibiting the replication of HBV and thus reducing the virus load in total. Lamivudine has strong effects with safety and few adverse reactions, however, the long-term administration may result in drug resistance. The therapeutic effect of adefovir is superior to that of lamivudine. Adefovir has good antiviral effect and is still effective for patients with lamivudine resistance. Adefovir has good tolerance and safety, however, the long-term use will cause nephrotoxicity. Long-term treatment with nucleoside drugs is only capable of enabling hepatitis B surface antigen clearance in 0.5% to 1% of the patients each year (E Loggi et al, Dig Liver Dis. 2015 Oct; 47(10): 836-41).

In summary, since HBV exists and replicates in liver cells persistently and HBV will continue to differentiate and mutate during treatment process, current antiviral drugs are not yet capable of eliminating HBV completely. In order to suppress the replication of the virus, the majority of the patients need long-term treatment, which brings huge economic and social burden to patients suffering from viral hepatitis B and society. There is an enormous market demand for the treatment of viral hepatitis B. Hepatitis B surface antigen clearance may break the host's immune tolerance to hepatitis B virus and restore the host's immune response to HBV, thereby achieving the purpose of treating viral hepatitis B and providing a new direction for the treatment of viral hepatitis B. Accordingly, the development of a hepatitis B surface antigen inhibitor will exert an important impact on the treatment of viral hepatitis B and bring enormous economic and social benefits.

The applicant of the present disclosure reported a dihydroisoquinoline compound in WO2018/130152A1, which may be used for treating and preventing hepatitis B virus infection. However, the activity of the compound disclosed in this patent application is not very high, and a relatively large dose is required to achieve relatively good efficacy in preventing and treating viral hepatitis B. Therefore, in the field of preventing and treating viral hepatitis B, there is an urgent need to provide a compound that has higher activity and is capable of achieving the purpose of preventing and treating viral hepatitis B at a lower dose.

### SUMMARY

### Problems to be solved by the disclosure

The present disclosure aims to provide a novel thiazole ring-substituted dihydroisoquinoline compound. This type of compound has an extremely strong activity for inhibiting hepatitis B surface antigen while having an extremely strong activity for inhibiting HBV DNA. In addition, this type of compound has relatively high bioavailability and is capable of exerting drug efficacy at a relatively low dose, thereby reducing the potential toxicity of the compound.

### Means for solving the problems

In order to achieve the above aim, the present disclosure provides a compound of general formula I, a pharmaceutically acceptable salt thereof, an ester thereof, an isomer thereof, a solvate thereof, a hydrate thereof, a prodrug thereof, or an isotopically-labeled compound thereof, and the compound of general formula I has a structure as follows: 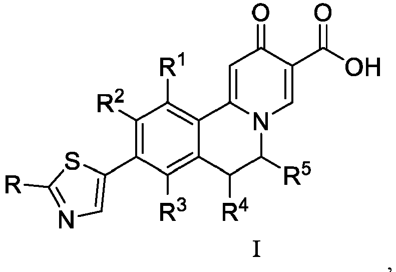 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one fluorine, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one fluorine, and C₃₋₇ cycloalkyl;
R is a substituted or unsubstituted group, said group is C₁₋₆ alkyl, amino, C₁₋₆ alkoxy, C₃₋₇ cycloalkoxy, C₆₋₁₀ aryloxy, C₃₋₇ cycloalkyl, 3- to 7-membered heterocycloalkyl, C₆₋₁₀ aryl, C₅₋₁₀ heteroaryl, or carbamoyl, and R is not an unsubstituted methyl.

Preferably, said R is a group with substitution, said group is C₁₋₆ alkyl, amino, C₁₋₆ alkoxy, C₃₋₇ cycloalkoxy, C₆₋₁₀ aryloxy, C₃₋₇ cycloalkyl, 3- to 7-membered heterocycloalkyl, C₆₋₁₀ aryl, C₅₋₁₀ heteroaryl, or carbamoyl, and said substitution refers to being substituted with one or more of the following groups: hydroxy, hydroxy-C₁₋₆ alkylene, halogen, C₁₋₆ alkyl, C₁₋₆ alkoxy, C₃₋₇ cycloalkoxy, C₁₋₆ alkoxy-C₁₋₆ alkylene, and C₁₋₆ alkoxy substituted with at least one halogen atom.

Preferably, said R is a group with substitution, said group is C₁₋₆ alkyl, amino, C₁₋₆ alkoxy, C₃₋₇ cycloalkoxy, C₃₋₇ cycloalkyl, 3- to 7-membered heterocycloalkyl, C₅₋₁₀ heteroaryl, or carbamoyl, and said substitution refers to being substituted with one or more of the following groups: hydroxy, hydroxy-C₁₋₆ alkylene, halogen, C₁₋₃ alkyl, C₁₋₃ alkoxy, C₁₋₃ alkoxy-C₁₋₆ alkylene, C₃₋₇ cycloalkoxy, and C₁₋₃ alkoxy substituted with at least one halogen atom.

Preferably, the compound of general formula I has a structure of general formula 1-1: 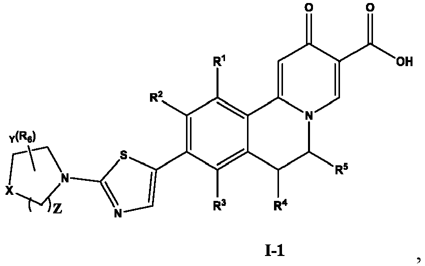 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl;
X is O, N, S, SO, SO₂, or C(R₆)₂;
Y is 0, 1,2, 3, 4 or 5;
Z is 0, 1 or 2; and
each R₆ is independently selected from hydrogen, deuterium, hydroxy, cyano, amino, C₁₋₆ alkylamino, halogen, C₁₋₆ alkyl, C₁₋₆ alkyl substituted with at least one halogen atom, C₁₋₆ alkyl substituted with hydroxy, or C₁₋₆ alkoxy.

Preferably, said X is O or C(R₆)₂; Y is 0, 1 or 2; and R₆ is independently selected from hydrogen, deuterium, hydroxy, cyano, amino, methylamino, ethylamino, fluorine, chlorine, methyl, ethyl, n-propyl, isopropyl, methoxy, ethoxy, and methyl, ethyl, n-propyl and isopropyl which are each independently substituted with at least one fluorine, chlorine or hydroxy.

Preferably, the compound of general formula I has a structure of general formula 1-2: 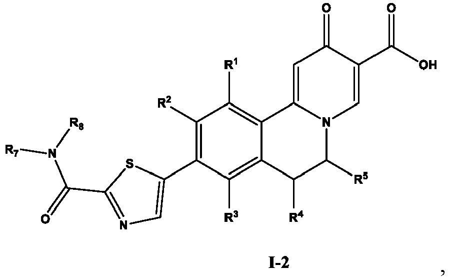 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl; and
R₇ and Rs are each independently selected from hydrogen, deuterium, C₁₋₆ alkyl, and C₁₋₆ alkyl substituted with at least one halogen atom, C₁₋₆ alkoxy or hydroxy.

Preferably, said R₇ and Rs are each independently selected from hydrogen, deuterium, methyl, ethyl, n-propyl, isopropyl, methoxyethyl, and methyl, ethyl, n-propyl and isopropyl substituted with at least one fluorine, chlorine or hydroxy.

Preferably, the compound of general formula I has a structure of general formula 1-3: 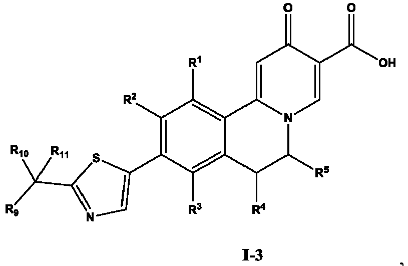 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl; and
R₉, R₁₀ and R₁₁ are each independently selected from hydrogen, deuterium, C₁₋₆ alkyl, hydroxy, C₁₋₆ alkyl substituted with at least one halogen atom or hydroxy, C₁₋₆ alkoxy unsubstituted or substituted with at least one halogen atom, and C₃₋₇ cycloalkoxy unsubstituted or substituted with at least one halogen atom or C₁₋₆ alkyl, or any two of R₉, R₁₀ and R₁₁ may form a substituted or unsubstituted C₃₋₇ cycloalkyl, and R₉, R₁₀ and R₁₁ are not hydrogen at the same time.

Preferably, R₉, R₁₀ and R₁₁ are each independently selected from hydrogen, deuterium, methyl, ethyl, n-propyl, isopropyl, hydroxy, methoxy, ethoxy, cyclopropoxy, and methyl, ethyl, n-propyl, isopropyl or C₁₋₃ alkoxy substituted with at least one fluorine, chlorine or hydroxy, and the substituted C₃₋₇ cycloalkyl refers to the one substituted with at least one halogen atom, C₁₋₆ alkyl or C₁₋₆ alkoxy.

Preferably, said R is a substituted or unsubstituted group, said group is piperidinyl, pyrrolidinyl, azetidinyl, morpholinyl, or C₁₋₃ alkyl.

Preferably, said R is a substituted or unsubstituted group, said group is piperidinyl, azetidinyl, C₁₋₆ alkoxy, cyclobutyl, propyl, C₁₋₃ alkyl, or carbamoyl.

Preferably, said R is substituted or unsubstituted piperidin-1-yl, substituted or unsubstituted azetidin-1-yl, cyclopropyl, substituted or unsubstituted cyclobutyl, propyl, or methyl or ethyl substituted with C₁₋₆ alkoxy, wherein the substituted piperidin-1-yl refers to the one substituted with at least one hydroxy-C₁₋₆ alkylene, C₁₋₆ alkyl or hydroxy, preferably substituted with hydroxy-C₁₋₆ alkylene or substituted with both C₁₋₆ alkyl and hydroxy; the substituted azetidin-1-yl refers to the one substituted with at least one halogen atom or C₁₋₆ alkoxy; and the substituted cyclobutyl refers to the one substituted with at least one halogen atom, C₁₋₆ alkyl or C₁₋₆ alkoxy.

Preferably, said R is substituted piperidin-1-yl, the substituted piperidin-1-yl refers to the one substituted with at least one hydroxy-C₁₋₃ alkylene, C₁₋₆ alkoxy, C₁₋₃ alkyl or hydroxy, preferably substituted with hydroxy-C₁₋₃ alkylene or substituted with both C₁₋₃ alkyl and hydroxy.

Preferably, said R is substituted or unsubstituted azetidin-1-yl, the substituted azetidin-1-yl refers to the one substituted with 1 to 3 halogen atoms or C₁₋₃ alkoxy.

Preferably, said R is methyl, ethyl or isopropyl substituted with C₁₋₆ alkoxy, preferably methyl, ethyl or isopropyl substituted with methoxy, ethoxy, propoxy or isopropoxy.

Preferably, R¹ is any one of hydrogen, deuterium, fluorine, chlorine, bromine, methyl, ethyl, isopropyl, tert-butyl, methylamino, ethylamino, methoxy, ethoxy, and isopropoxy;
R² is any one of hydrogen, deuterium, fluorine, chlorine, bromine, methyl, ethyl, isopropyl, tert-butyl, trifluoromethyl, trifluoromethylmethyl, cyclopropyl, cyclopentyl, methylamino, ethylamino, methoxy, ethoxy, isopropoxy, pyrrolidinyl, and morpholinyl;
R³ is any one of hydrogen, deuterium, fluorine, chlorine, bromine, methyl, ethyl, and cyano;
R⁴ is any one of hydrogen, deuterium, methyl, and ethyl; and
R⁵ is any one of hydrogen, deuterium, methyl, ethyl, isopropyl, butyl, sec-butyl, isobutyl, tert-butyl, trifluoromethyl, trifluoromethylmethyl, and cyclopropyl.
Preferably, R¹ is any one of hydrogen, deuterium, halogen, and C₁₋₆ alkoxy;
R² is any one of hydrogen, deuterium, halogen, C₁₋₆ alkylamino, and C₁₋₆ alkoxy;
R³ is any one of hydrogen, deuterium, and halogen;
R⁴ is hydrogen or deuterium; and
R⁵ is any one of hydrogen, deuterium, C₁₋₆ alkyl, C₁₋₆ alkyl substituted with at least one fluorine, and C₃₋₇ cycloalkyl.
Preferably, R¹ is hydrogen or deuterium;
R² is any one of hydrogen, deuterium, halogen, and C₁₋₆ alkoxy;
R³ is hydrogen or deuterium;
R⁴ is hydrogen or deuterium;
R⁵ is any one of hydrogen, deuterium, methyl, ethyl, isopropyl, butyl, sec-butyl, isobutyl, tert-butyl, trifluoromethyl, trifluoromethylmethyl, and cyclopropyl; and
R is a group with or without substitution, said group is piperidinyl, azetidinyl, morpholinyl, amino, cyclopropyl, propyl, C₁₋₃ alkyl, C₁₋₃ alkoxy, cyclobutyl, or carbamoyl, and said substitution refers to being substituted with one or more of the following groups: hydroxy, hydroxy-C₁₋₆ alkylene, halogen, C₁₋₃ alkyl, C₁₋₃ alkoxy, C₁₋₃ alkoxy-C₁₋₆ alkylene, C₁₋₃ alkoxy substituted with at least one halogen atom, and C₃₋₇ cycloalkoxy.

Preferably, the compound of general formula I is any one of the following compounds:
6-tert-butyl-9-[2-(4-hydroxypiperidin-1-yl)thiazol-5-yl]-10-methoxy-2-oxo-6,7-dihydro-2*H*-pyrido[2,1-*α*]isoquinoline-3-carboxylic acid;
6-tert-butyl-9-{2-[4-(hydroxymethyl)piperidin-1-yl]thiazol-5-yl}-10-methoxy-2-oxo-6,7-dihydro-2*H*-pyrido[2,1-*α*]isoquinoline-3-carboxylic acid;
9-[2-(azetidin-1-yl)thiazol-5-yl]-6-tert-butyl-10-methoxy-2-oxo-6,7-dihydro-2*H-*pyrido[2,1-*α*]isoquinoline-3-carboxylic acid;
6-tert-butyl-9-[2-(3,3-difluoroazetidin-1-yl)thiazol-5-yl]-10-methoxy-2-oxo-6,7-dihydro-2*H*-pyiido[2,1-*α*]isoquinoline-3-carboxylic acid;
6-tert-butyl-10-methoxy-9-(2-morpholinothiazol-5-yl)-2-oxo-6,7-dihydro-2*H*-pyrido[2,1-*α*]isoquinoline-3-carboxylic acid;
6-tert-butyl-9-[2-(dimethylamino)thiazol-5-yl]-10-methoxy-2-oxo-6,7-dihydro-2*H-*pyrido[2,1-*α*]isoquinoline-3-carboxylic acid;
6-tert-butyl-9-[2-(4-hydroxy-4-methylpiperidin-1-yl)thiazol-5-yl]-10-methoxy-2-oxo-6,7-dihydro-2*H*-pyrido[2,1-*α*]isoquinoline-3-carboxylic acid;
6-tert-butyl-10-methoxy-9-[2-(4-methoxypiperidin-1-yl)thiazol-5-yl]-2-oxo-6,7-dihydro-2*H*-pyiido[2,1-*α*]isoquinoline-3-carboxylic acid;
6-tert-butyl-10-methoxy-9-[2-(3-methoxyazetidin-1-yl)thiazol-5-yl]-2-oxo-6,7-dihydro-2*H*-pyiido[2,1-*α*]isoquinoline-3-carboxylic acid;
6-tert-butyl-10-methoxy-9-[2-(methoxymethyl)thiazol-5-yl]-2-oxo-6,7-dihydro-2*H-*pyrido[2,1-*α*]isoquinoline-3-carboxylic acid;
6-tert-butyl-9-[2-(3-fluoroazetidin-1-yl)thiazol-5-yl]-10-methoxy-2-oxo-6,7-dihydro-2*H-*pyrido[2,1-*α*]isoquinoline-3-carboxylic acid;
6-tert-butyl-10-methoxy-9-(2-methoxythiazol-5-yl)-2-oxo-6,7-dihydro-2*H*-pyrido[2,1-*α*]isoquinoline-3-carboxylic acid;
6-tert-butyl-9-[2-(ethoxy)thiazol-5-yl]-10-methoxy-2-oxo-6,7-dihydro-2*H*-pyrido[2,1-*α*]isoquinoline-3-carboxylic acid;
6-tert-butyl-9-[2-(ethoxymethyl)thiazol-5-yl]-10-methoxy-2-oxo-6,7-dihydro-2*H-*pyrido[2,1-*α*]isoquinoline-3-carboxylic acid;
6-tert-butyl-10-methoxy-9-[2-(1-methoxyethyl)thiazol-5-yl]-2-oxo-6,7-dihydro-2*H-*pyrido[2,1-*α*]isoquinoline-3-carboxylic acid;
6-tert-butyl-9-[2-(1-hydroxyethyl)thiazol-5-yl]-10-methoxy-2-oxo-6,7-dihydro-2*H-*pyrido[2,1-*α*]isoquinoline-3-carboxylic acid;
6-tert-butyl-10-methoxy-9-[2-(2-methoxypropan-2-yl)thiazol-5-yl]-2-oxo-6,7-dihydro-2*H*-pyiido[2,1-*α*]isoquinoline-3-carboxylic acid;
6-tert-butyl-10-methoxy-2-oxo-9-(2-propylthiazol-5-yl)-6,7-dihydro-2*H*-pyrido[2,1-*α*] isoquinoline-3-carboxylic acid;
6-tert-butyl-10-methoxy-9-[2-(methylcarbamoyl)thiazol-5-yl]-2-oxo-6,7-dihydro-2*H-*pyrido[2,1-*α*]isoquinoline-3-carboxylic acid;
6-tert-butyl-9-[2-(ethylcarbamoyl)thiazol-5-yl]-10-methoxy-2-oxo-6,7-dihydro-2*H-*pyrido[2,1-*α*]isoquinoline-3-carboxylic acid;
6-tert-butyl-10-methoxy-9-[2-(2-methoxyethoxy)thiazol-5-yl]-2-oxo-6,7-dihydro-2*H-*pyrido[2,1-*α*]isoquinoline-3-carboxylic acid;
6-tert-butyl-10-methoxy-9-{2-[(2-methoxyethyl)carbamoyl]thiazol-5-yl}-2-oxo-6,7-dihydro-2*H*-pyrido[2,1-*α*]isoquinoline-3-carboxylicacid;
6-tert-butyl-9-{2-[(difluoromethoxy)methyl]thiazol-5-yl}-10-methoxy-2-oxo-6,7-dihydro-2*H*-pyrido[2,1-*α*]isoquinoline-3-carboxylic acid;
6-tert-butyl-9-[2-(cyclopropoxymethyl)thiazol-5-yl]-10-methoxy-2-oxo-6,7-dihydro-2*H-*pyrido[2,1-*α*]isoquinoline-3-carboxylic acid;
6-tert-butyl-9-[2-(3,3-difluorocyclobutyl)thiazol-5-yl}-10-methoxy-2-oxo-6,7-dihydro-2*H*-pyiido[2,1-*α*]isoquinoline-3-carboxylic acid;
6-tert-butyl-9-[2-(3-fluorocyclobutyl)thiazol-5-yl]-10-methoxy-2-oxo-6,7-dihydro-2*H-*pyrido[2,1-*α*]isoquinoline-3-carboxylic acid; and
6-tert-butyl-10-methoxy-9-[2-(3-methoxycyclobutyl)thiazol-5-yl]-2-oxo-6,7-dihydro-2*H-*pyrido[2,1-*α*]isoquinoline-3-carboxylic acid.

The present disclosure further provides a pharmaceutical composition, comprising any aforementioned compound of general formula I, or a pharmaceutically acceptable salt thereof, an ester thereof, an isomer thereof, a solvate thereof, a hydrate thereof, a prodrug thereof, or an isotopically-labeled compound thereof.

The present disclosure further provides a pharmaceutical formulation, comprising any aforementioned compound of general formula I, or a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, said formulation is any one of a tablet, a capsule, an injection, a granule, a pulvis, a suppository, a pill, a cream, a paste, a gel, a powder, an oral solution, an inhalant, a suspension, a dry suspension, a patch, and a lotion.

The present disclosure further provides any aforementioned compound of general formula I, or a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation, which is used for preventing and treating viral hepatitis B.

The present disclosure further provides any aforementioned compound of general formula I, or a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation for use in the prevention and/or treatment of viral hepatitis B.

The present disclosure further provides use of any aforementioned compound of general formula I, or a pharmaceutically acceptable salt thereof, an ester thereof, a hydrate thereof, a solvate thereof, a stereoisomer thereof, a tautomer thereof, cis-trans isomers thereof, an isotopically-labeled compound of general formula I, or a prodrug thereof, or the aforementioned pharmaceutical composition, or the aforementioned pharmaceutical formulation in the preparation of a drug for preventing and/or treating viral hepatitis B.

The present disclosure further provides a method for preventing and/or treating viral hepatitis B, comprising the following step of administering a therapeutically effective amount of any aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation to a patient in need thereof.

The present disclosure further provides a pharmaceutical combination, comprising any aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation, and at least one additional therapeutic agent for viral hepatitis B.

The present disclosure further provides a method for preventing and/or treating viral hepatitis B, comprising the following step of administering a therapeutically effective amount of any aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation, and at least one additional therapeutic agent for viral hepatitis B to a patient in need thereof.

### Advantageous effects of the disclosure

The novel thiazole ring-substituted dihydroisoquinoline compound of the present disclosure has an extremely strong activity for inhibiting hepatitis B surface antigen while having an extremely strong activity for inhibiting HBV DNA. In addition, this type of compound has relatively high bioavailability and is capable of exerting drug efficacy at a relatively low dose, thereby reducing the potential toxicity of the compound.

### DETAILED DESCRIPTION

In order to describe the content of the present disclosure more clearly, all terms involved in this disclosure are defined as follows.

First, the present disclosure provides a compound of general formula I, a pharmaceutically acceptable salt thereof, an ester thereof, an isomer thereof, a solvate thereof, a hydrate thereof, a prodrug thereof, or an isotopically-labeled compound thereof, and the compound of general formula I has the structure as follows: 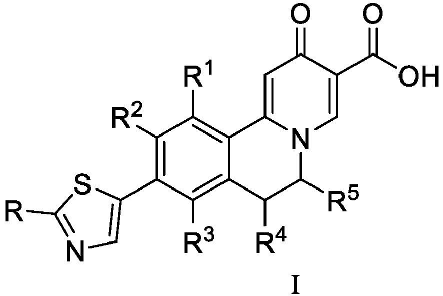 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one fluorine, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one fluorine, and C₃₋₇ cycloalkyl; and
R is a substituted or unsubstituted group, said group is C₁₋₆ alkyl, amino, C₁₋₆ alkoxy, C₃₋₇ cycloalkoxy, C₆₋₁₀ aryloxy, C₃₋₇ cycloalkyl, 3- to 7-membered heterocycloalkyl, C₆₋₁₀ aryl, C₅₋₁₀ heteroaryl, or carbamoyl, and R is not an unsubstituted methyl.

The term "C₁₋₆ alkyl", alone or in combination, refers to a saturated linear or branched alkyl containing 1 to 6 carbon atoms, in particular 1 to 4 carbon atoms, including methyl, ethyl, propyl, isopropyl, butyl, sec-butyl, isobutyl, tert-butyl, n-pentyl, pentan-2-yl, pentan-3-yl, 2-methylbutan-2-yl, 3-methylbutan-2-yl, 3-methylbutan-1-yl, 2-methylbutan-1-yl, n-hexyl, hexan-2-yl, hexan-3-yl, 2-methylpentan-2-yl, 3-methylpentan-2-yl, 4-methylpentan-2-yl, 3-methylpentan-3-yl, 2-methylpentan-3-yl, 2,3-dimethylbutan-2-yl, 3,3,-dimethylbutan-2-yl, and the like. Preferably, "C₁₋₆ alkyl" is any one of methyl, ethyl, n-propyl, isopropyl, and tert-butyl. Similarly, the term "C₁₋₃ alkyl", alone or in combination, refers to a saturated linear or branched alkyl containing 1 to 3 carbon atoms, including methyl, ethyl, propyl, isopropyl, and the like.

The term "C₃₋₇ cycloalkyl", alone or in combination, refers to a saturated cycloalkyl having 3 to 7 carbon atoms, in particular 3 to 6 carbon atoms, including cyclopropyl, cyclobutyl, cyclopentyl, cyclohexyl, cycloheptyl, and the like. In particular, "C₃₋₇ cycloalkyl" is cyclopropyl, cyclopentyl, cyclohexyl, or the like.

The term "amino", alone or in combination, refers to a primary amino group (-NH₂), a secondary amino group (-NH-) or a tertiary amino group

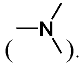

The term "C₁₋₆ alkylamino", alone or in combination, refers to an amino group as defined above, wherein the hydrogen atom(s) of the amino group is/are substituted with at least one C₁₋₆ alkyl, wherein "C₁₋₆ alkyl" is as defined above. Correspondingly, "C₁₋₆ alkylamino" includes methylamino, ethylamino, propylamino, isopropylamino, n-butylamino, isobutylamino, 2-butylamino, tert-butylamino, n-pentylamino, pentan-2-ylamino, pentan-3-ylamino, 2-methylbutan-2-ylamino, 3-methylbutan-2-ylamino, 3-methylbutan-1-ylamino, 2-methylbutan-1-ylamino, n-hexylamino, hexan-2-ylamino, hexan-3-ylamino, 2-methylpentan-2-ylamino, 3-methylpentan-2-ylamino, 4-methylpentan-2-ylamino, 3-methylpentan-3-ylamino, 2-methylpentan-3-ylamino, 2,3-dimethylbutan-2-ylamino, 3,3-dimethylbutan-2-ylamino, and the like. In particular, "C₁₋₆ alkylamino" is methylamino, ethylamino, isopropylamino, tert-butylamino, or the like.

The term "C₁₋₆ alkoxy", alone or in combination, refers to C₁₋₆ alkyl-O- group, wherein "C₁₋₆ alkyl" is as defined above. C₁₋₆ alkoxy includes (but not limited to) methoxy (-OCH₃), ethoxy (-OCH₂CH₃), n-propoxy (-OCH₂CH₂CH₃), isopropoxy (-OCH(CH₃)₂), n-butoxy (-OCH₂CH₂CH₂CH₃), sec-butoxy (-OCH(CH₃)CH₂CH₃), iso-butoxy (-OCH₂CH(CH₃)₂), tertbutoxy (-OC(CH₃)₃), n-pentoxy (-OCH₂CH₂CH₂CH₂CH₃), neopentoxy (-OCH₂C(CH₃)₃), and the like. Similarly, the term "C₁₋₃ alkoxy", alone or in combination, refers to C₁₋₃ alkyl-O- group, wherein "C₁₋₃ alkyl" is as defined above.

The term "C₃₋₇ cycloalkoxy", alone or in combination, refers to C₃₋₇ cycloalkyl-O- group, wherein C₃₋₇ cycloalkyl is as defined above.

The term "carbamoyl" refers to -C(=O)-amino or -C(=O)-linked amino.

The term "halogen", alone or in combination, refers to fluorine, chlorine, bromine or iodine, in particular, fluorine, chlorine or bromine.

The term "heterocycloalkyl" refers to a saturated or partially unsaturated (containing 1 or 2 double bonds) non-aromatic cyclic group consisting of carbon atom(s) and heteroatom(s) such as nitrogen, oxygen or sulfur, and this cyclic group may be a monocyclic or bicyclic group. In the present disclosure, the number of carbon atoms in a heterocycloalkyl is 2 to 11, the number of heteroatoms is preferably 1, 2, 3 or 4, and the nitrogen atom, carbon atom or sulfur atom in a heterocycloalkyl may be optionally oxidized. Hydrogen atoms in a "heterocycloalkyl" are independently and optionally substituted with one or more substituents described in the present disclosure. A "heterocycloalkyl" may be linked to the skeleton via any ring atom in the ring.

The term "3- to 7-membered heterocycloalkyl" refers to a monocyclic heterocycloalkyl containing 3 to 7 carbon atom(s) and heteroatom(s); for example, aziridinyl, azetidinyl, oxetanyl, pyrrolidinyl, tetrahydrofuranyl, tetrahydrothienyl, piperidinyl, morpholinyl, piperazinyl, thiomorpholinyl, tetrahydropyranyl, and 1,1-dioxo-thiomorpholinyl.

The term "C₃₋₇ cycloalkylamino", alone or in combination, refers to the amino group as defined above, wherein the hydrogen atom(s) of the amino group is/are substituted with at least one C₃₋₇ cycloalkyl and "C₃₋₇ cycloalkyl" is as defined above.

The term "3- to 7-membered heterocycloalkylamino", alone or in combination, refers to the amino group as defined above, wherein the hydrogen atom(s) of the amino group is/are substituted with at least one 3- to 7-membered heterocycloalkyl and "3- to 7-membered heterocycloalkyl" is as defined above.

The term "aryl" refers to any stable 6- to 10-membered monocyclic or bicyclic aromatic group, including phenyl, naphthyl, tetrahydronaphthyl, 2,3-dihydroindenyl, biphenyl, or the like. Hydrogen atoms in an "aryl" are independently and optionally substituted with one or more substituents described in the present disclosure.

The term "heteroaryl" refers to an aromatic cyclic group formed by substituting the carbon atom(s) in the ring with at least one heteroatom selected from sulfur, oxygen or nitrogen, and this aromatic cyclic group may be a 5- to 7-membered monocyclic group or a 7- to 12-membered bicyclic group. In the present disclosure, the number of heteroatoms in a heteroaryl is preferably 1, 2, 3 or 4, for example, thienyl, pyridinyl, pyrimidinyl, pyrazinyl, pyridazinyl, pyridin-2(1*H*)-carbonyl, pyridin-4(1*H*)-carbonyl, pyrrolyl, pyrazolyl, thiazolyl, 1,2,3-triazolyl, 1,2,4-triazolyl, imidazolyl, tetrazolyl, isothiazolyl, oxazolyl, isoxazolyl, thiadiazolyl, oxadiazolyl, naphthyl, benzothienyl, indolyl, benzimidazolyl, benzothiazolyl, benzofuranyl, quinolinyl, isoquinolinyl, quinazolinyl, and the like. Hydrogen atoms in a "heteroaryl" are independently and optionally substituted with one or more substituents described in the present disclosure.

The term "C₅₋₁₀ heteroaryl" refers to an aromatic heterocyclic ring having 5 to 10 carbon atoms, wherein the aromatic heterocyclic ring is as defined above.

The term "C₆₋₁₀ aryl" refers to an aryl having 6 to 10 carbon atoms, wherein the aryl is as defined above.

The term "C₆₋₁₀ aryloxy" refers to C₆₋₁₀ aryl-O- group, wherein C₆₋₁₀ aryl is as defined above.

The term "aryl C₁₋₆ alkylene" refers to C₁₋₆ alkyl group substituted with one or more aryl groups, and aryl and C₁₋₆ alkyl are as defined above.

The term "heteroaryl C₁₋₆ alkylene" refers to C₁₋₆ alkyl group substituted with one or more heteroaryl groups, and heteroaryl and C₁₋₆ alkyl are as defined above.

The term "cyano", alone or in combination, refers to -CN group.

The term "carboxyl", alone or in combination, refers to -COOH group.

The term "hydroxy", alone or in combination, refers to -OH group.

The term "isomer" comprises all isomeric forms, including enantiomers, diastereoisomers, tautomers, and geometric isomers (including cis/trans isomers). Therefore, both the single stereochemical isomer of the compounds involved in the present disclosure, and a mixture of the enantiomers, diastereoisomers, tautomers or geometric isomers (or cis/trans isomers) thereof fall within the scope of the present disclosure.

The term "pharmaceutically acceptable salt" means that the compounds of the present disclosure exist in the form of their pharmaceutical salts, including acid addition salts and base addition salts. Pharmaceutically acceptable salts are described in the "pharmaceutically salts" in J. Pharmaceutical Sciences (Vol 66: page 1-19, 1977) by S. M. Berge. In the present disclosure, a pharmaceutically acceptable non-toxic acid addition salt refers to a salt formed by a compound of the present disclosure and an organic or inorganic acid. The organic acid or inorganic acid includes, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. A pharmaceutically acceptable non-toxic base addition salt refers to a salt formed by a compound of the present disclosure and an organic or inorganic base, including but not limited to alkali metal salts, such as lithium salts, sodium salts or potassium salts; alkaline earth metal salts, such as calcium salts or magnesium salts; salts of organic bases, such as ammonium salts formed by an organic base having nitrogen-containing group(s) or N⁺(C₁₋₆ alkyl)₄ salts. The organic or inorganic base includes, but not limited to, preferably lithium hydroxide, sodium hydroxide, potassium hydroxide, sodium carbonate, sodium bicarbonate, potassium carbonate, potassium bicarbonate, magnesium carbonate, calcium carbonate, ammonia water, triethylamine, tetrabutylammonium hydroxide, and the like.

The term "solvate" refers to a complex formed by one or more solvent molecules and a compound of the present disclosure. Solvents forming solvates include, but not limited to, water, methanol, ethanol, isopropanol, ethyl acetate, tetrahydrofuran, *N,N*-dimethylformamide, dimethyl sulfoxide, and the like. "Pharmaceutically acceptable salts" may be synthesized by general chemical methods.

The term "ester" refers to an organic ester, including monoesters, diesters, triesters, and more generally, polyesters.

The term "hydrate" refers to a complex formed by water and a compound of the present disclosure.

The term "prodrug" refers to a chemical derivative of a compound of the present disclosure, and this derivative is converted into a compound represented by general formula I via chemical reaction(s) *in vivo.*

The term "isotopic derivative" refers to an isotopic derivative obtained by substituting the hydrogen atoms in general formula I with 1 to 6 deuterium atoms, or an isotopic derivative obtained by substituting the carbon atoms in general formula I with 1 to 3 ¹⁴C atoms.

The terms involved in the present disclosure are defined above, and those skilled in the art may also understand the above terms in combination with the prior art. The following content is further description based on the content of the present disclosure and the definitions of the terms.

In a preferred embodiment, said R is a group with substitution, said group is C₁₋₆ alkyl, amino, C₁₋₆ alkoxy, C₃₋₇ cycloalkoxy, C₆₋₁₀ aryloxy, C₃₋₇ cycloalkyl, 3- to 7-membered heterocycloalkyl, C₆₋₁₀ aryl, C₅₋₁₀ heteroaryl, or carbamoyl, and said substitution refers to being substituted with one or more of the following groups: hydroxy, hydroxy-C₁₋₆ alkylene, halogen, C₁₋₆ alkyl, C₁₋₆ alkoxy, C₃₋₇ cycloalkoxy, C₁₋₆ alkoxy-C₁₋₆ alkylene, or C₁₋₆ alkoxy substituted with at least one halogen atom.

In a more preferred embodiment, said R is a group with substitution, said group is C₁₋₆ alkyl, amino, C₁₋₆ alkoxy, C₃₋₇ cycloalkoxy, C₃₋₇ cycloalkyl, 3- to 7-membered heterocycloalkyl, C₅₋₁₀ heteroaryl, or carbamoyl, and said substitution refers to being substituted with one or more of the following groups: hydroxy, hydroxy-C₁₋₆ alkylene, halogen, C₁₋₃ alkyl, C₁₋₃ alkoxy, C₁₋₃ alkoxy-C₁₋₆ alkylene, C₃₋₇ cycloalkoxy, or C₁₋₃ alkoxy substituted with at least one halogen atom.

In a preferred embodiment, the compound of general formula I has the structure of general formula 1-1: 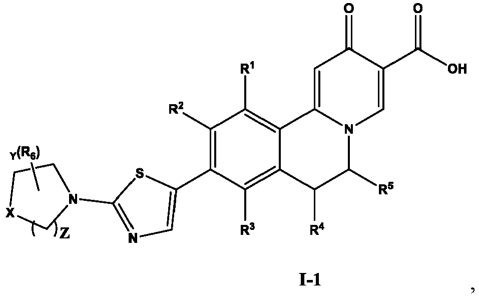 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl;
X is O, N, S, SO, SO₂, or C(R₆)₂;
Y is 0, 1, 2, 3, 4 or 5;
Z is 0, 1 or 2;
each R₆ is independently selected from hydrogen, deuterium, hydroxy, cyano, amino, C₁₋₆ alkylamino, halogen, C₁₋₆ alkyl, C₁₋₆ alkyl substituted with at least one halogen atom, C₁₋₆ alkyl substituted with hydroxy, or C₁₋₆ alkoxy;
wherein "_{Y}(R₆)" means being substituted with Y R₆ groups and said Y has the above-mentioned definition.

In a more preferred embodiment, said X is O or C(R₆)₂;
Y is 0, 1 or 2; and
R₆ is independently selected from hydrogen, deuterium, hydroxy, cyano, amino, methylamino, ethylamino, fluorine, chlorine, C₁₋₃ alkyl, C₁₋₃ alkyl substituted with at least one fluorine or chlorine, C₁₋₃ alkyl substituted with hydroxy, or C₁₋₃ alkoxy.

In a preferred embodiment, the compound of general formula I has the structure of general formula 1-2: 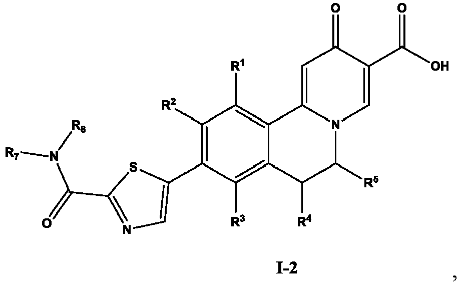 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl; and
R₇ and Rs are each independently selected from hydrogen, deuterium, C₁₋₆ alkyl, C₁₋₆ alkyl substituted with at least one halogen atom or C₁₋₆ alkoxy, or C₁₋₆ alkyl substituted with hydroxy.

In a more preferred embodiment, said R₇ and Rs are each independently selected from hydrogen, deuterium, C₁₋₆ alkyl, or C₁₋₆ alkyl substituted with at least one halogen, C₁₋₆ alkoxy or hydroxy.

In a preferred embodiment, the compound of general formula I has the structure of general formula 1-3: 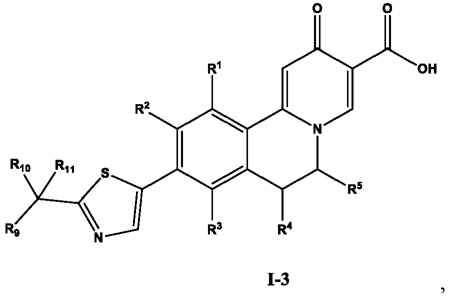 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl; and
R₉, R₁₀ and R₁₁ are each independently selected from hydrogen, deuterium, C₁₋₆ alkyl, hydroxy, C₁₋₆ alkyl substituted with at least one halogen atom or hydroxy, C₁₋₆ alkoxy unsubstituted or substituted with at least one halogen atom, and C₃₋₇ cycloalkoxy unsubstituted or substituted with at least one halogen atom or C₁₋₆ alkyl, or any two of R₉, R₁₀ and R₁₁ may form a substituted or unsubstituted C₃₋₇ cycloalkyl, and R₉, R₁₀ and R₁₁ are not hydrogen at the same time.

In a more preferred embodiment, R₉, R₁₀ and R₁₁ are each independently selected from hydrogen, deuterium, methyl, ethyl, n-propyl, isopropyl, hydroxy, methoxy, ethoxy, cyclopropoxy, and methyl, ethyl, n-propyl, isopropyl or C₁₋₃ alkoxy substituted with at least one fluorine, chlorine or hydroxy, and the substituted C₃₋₇ cycloalkyl refers to the one substituted with at least one halogen atom, C₁₋₆ alkyl or C₁₋₆ alkoxy. Preferably, the substituted C₃₋₇ cycloalkyl refers to the one substituted with 1 to 3 fluorine, chlorine, bromine, methoxy or ethoxy, and said C₃₋₇ cycloalkyl may be cyclopropyl, cyclobutyl, cyclopentyl or cyclohexyl.

In a preferred embodiment, said R is a substituted or unsubstituted group, said group is piperidinyl, pyrrolidinyl, azetidinyl, morpholinyl, or C₁₋₃ alkyl.

In a preferred embodiment, said R is a substituted or unsubstituted group, said group is piperidinyl, azetidinyl, C₁₋₆ alkoxy, cyclobutyl, C₁₋₃ alkyl, or carbamoyl.

In a more preferred embodiment, said R is substituted or unsubstituted piperidin-1-yl, substituted or unsubstituted azetidin-1-yl, cyclopropyl, substituted or unsubstituted cyclobutyl, propyl, or methyl or ethyl substituted with C₁₋₆ alkoxy, wherein the substituted piperidin-1-yl refers to the one substituted with at least one hydroxy-C₁₋₆ alkylene, C₁₋₆ alkyl or hydroxy, preferably substituted with hydroxy-C₁₋₆ alkylene or substituted with both C₁₋₆ alkyl and hydroxy; the substituted azetidin-1-yl refers to the one substituted with at least one halogen atom or C₁₋₆ alkoxy, more preferably substituted with 1 to 3 fluorine, chlorine, bromine, methoxy or ethoxy; and the substituted cyclobutyl refers to the one substituted with at least one halogen atom, C₁₋₆ alkyl or C₁₋₆ alkoxy, more preferably substituted with 1 to 3 fluorine, chlorine, bromine, methyl, ethyl, isopropyl, n-propyl, methoxy, ethoxy, n-propoxy or isopropoxy. In a more preferred embodiment, said R is substituted piperidin-1-yl, the substituted piperidin-1-yl refers to the one substituted with at least one hydroxy-C₁₋₃ alkylene, C₁₋₆ alkoxy, C₁₋₃ alkyl or hydroxy, preferably substituted with hydroxy-C₁₋₃ alkylene or substituted with both C₁₋₃ alkyl and hydroxy.

In a more preferred embodiment, said R is substituted or unsubstituted azetidin-1-yl, the substituted azetidin-1-yl refers to the one substituted with 1 to 3 halogen atoms or C₁₋₃ alkoxy.

In a more preferred embodiment, said R is methyl, ethyl or isopropyl substituted with C₁₋₆ alkoxy, preferably methyl, ethyl or isopropyl substituted with methoxy, ethoxy, propoxy or isopropoxy.

In a more preferred embodiment, said R¹ is any one of hydrogen, deuterium, fluorine, chlorine, bromine, methyl, ethyl, isopropyl, tert-butyl, methylamino, ethylamino, methoxy, ethoxy, and isopropoxy;
R² is any one of hydrogen, deuterium, fluorine, chlorine, bromine, methyl, ethyl, isopropyl, tert-butyl, trifluoromethyl, trifluoromethylmethyl, cyclopropyl, cyclopentyl, methylamino, ethylamino, methoxy, ethoxy, isopropoxy, pyrrolidinyl, and morpholinyl;
R³ is any one of hydrogen, deuterium, fluorine, chlorine, bromine, methyl, ethyl, and cyano;
R⁴ is any one of hydrogen, deuterium, methyl, and ethyl; and
R⁵ is any one of hydrogen, deuterium, methyl, ethyl, isopropyl, butyl, sec-butyl, isobutyl, tert-butyl, trifluoromethyl, trifluoromethylmethyl, and cyclopropyl.
Preferably, said R¹ is any one of hydrogen, deuterium, halogen, and C₁₋₆ alkoxy;
R² is any one of hydrogen, deuterium, halogen, C₁₋₆ alkylamino, and C₁₋₆ alkoxy;
R³ is any one of hydrogen, deuterium, and halogen;
R⁴ is hydrogen or deuterium; and
R⁵ is any one of hydrogen, deuterium, C₁₋₆ alkyl, C₁₋₆ alkyl substituted with at least one fluorine, and C₃₋₇ cycloalkyl.

More Preferably, said R¹ is hydrogen or deuterium;
R² is any one of hydrogen, deuterium, halogen, and C₁₋₆ alkoxy;
R³ is hydrogen or deuterium;
R⁴ is hydrogen or deuterium;
R⁵ is any one of hydrogen, deuterium, methyl, ethyl, isopropyl, butyl, sec-butyl, isobutyl, tert-butyl, trifluoromethyl, trifluoromethylmethyl, and cyclopropyl; and
R is a group with or without substitution, said group is piperidinyl, azetidinyl, morpholinyl, amino, cyclopropyl, propyl, C₁₋₃ alkyl, C₁₋₃ alkoxy, cyclobutyl, or carbamoyl, and said substitution refers to being substituted with one or more of the following groups: hydroxy, hydroxy-C₁₋₆ alkylene, halogen, C₁₋₃ alkyl, C₁₋₃ alkoxy, C₁₋₃ alkoxy-C₁₋₆ alkylene, C₁₋₃ alkyl substituted with at least one halogen atom, and C₃₋₇ cycloalkoxy.

In a more preferred embodiment, the compound of general formula I is any one of the following compounds:
6-tert-butyl-9-[2-(4-hydroxypiperidin-1-yl)thiazol-5-yl]-10-methoxy-2-oxo-6,7-dihydro-2*H*-pyiido[2,1-*α*]isoquinoline-3-carboxylic acid;
6-tert-butyl-9-{2-[4-(hydroxymethyl)piperidin-1-yl]thiazol-5-yl}-10-methoxy-2-oxo-6,7-dihydro-2*H*-pyrido[2,1-*α*]isoquinoline-3-carboxylicacid;
9-[2-(azetidin-1-yl)thiazol-5-yl]-6-tert-butyl-10-methoxy-2-oxo-6,7-dihydro-2*H-*pyrido[2,1-*α*]isoquinoline-3-carboxylic acid;
6-tert-butyl-9-[2-(3,3-difluoroazetidin-1-yl)thiazol-5-yl]-10-methoxy-2-oxo-6,7-dihydro-2*H*-pyiido[2,1-*α*]isoquinoline-3-carboxylic acid;
6-tert-butyl-10-methoxy-9-(2-morpholinothiazol-5-yl)-2-oxo-6,7-dihydro-2*H*-pyrido[2,1-*α*]isoquinoline-3-carboxylic acid;
6-tert-butyl-9-[2-(dimethylamino)thiazol-5-yl]-10-methoxy-2-oxo-6,7-dihydro-2*H-*pyrido[2,1-*α*]isoquinoline-3-carboxylic acid;
6-tert-butyl-9-[2-(4-hydroxy-4-methylpiperidin-1-yl)thiazol-5-yl]-10-methoxy-2-oxo-6,7-dihydro-2*H*-pyrido[2,1-*α*]isoquinoline-3-carboxylic acid;
6-tert-butyl-10-methoxy-9-[2-(4-methoxypiperidin-1-yl)thiazol-5-yl]-2-oxo-6,7-dihydro-2*H*-pyiido[2,1-*α*]isoquinoline-3-carboxylic acid;
6-tert-butyl-10-methoxy-9-[2-(3-methoxyazetidin-1-yl)thiazol-5-yl]-2-oxo-6,7-dihydro-2*H*-pyiido[2,1-*α*]isoquinoline-3-carboxylic acid;
6-tert-butyl-10-methoxy-9-[2-(methoxymethyl)thiazol-5-yl]-2-oxo-6,7-dihydro-2*H-*pyrido[2,1-*α*]isoquinoline-3-carboxylic acid;
6-tert-butyl-9-[2-(3-fluoroazetidin-1-yl)thiazol-5-yl]-10-methoxy-2-oxo-6,7-dihydro-2*H-*pyrido[2,1-*α*]isoquinoline-3-carboxylic acid;
6-tert-butyl-10-methoxy-9-(2-methoxythiazol-5-yl)-2-oxo-6,7-dihydro-2*H*-pyrido[2,1-*α*]isoquinoline-3-carboxylic acid;
6-tert-butyl-9-[2-(ethoxy)thiazol-5-yl]-10-methoxy-2-oxo-6,7-dihydro-2*H*-pyrido[2,1-*α*]isoquinoline-3-carboxylic acid;
6-tert-butyl-9-[2-(ethoxymethyl)thiazol-5-yl]-10-methoxy-2-oxo-6,7-dihydro-2*H-*pyrido[2,1-*α*]isoquinoline-3-carboxylic acid;
6-tert-butyl-10-methoxy-9-[2-(1-methoxyethyl)thiazol-5-yl]-2-oxo-6,7-dihydro-2*H-*pyrido[2,1-*α*]isoquinoline-3-carboxylic acid;
6-tert-butyl-9-[2-(1-hydroxyethyl)thiazol-5-yl]-10-methoxy-2-oxo-6,7-dihydro-2*H-*pyrido[2,1-*α*]isoquinoline-3-carboxylic acid;
6-tert-butyl-10-methoxy-9-[2-(2-methoxypropan-2-yl)thiazol-5-yl]-2-oxo-6,7-dihydro-2*H*-pyiido[2,1-*α*]isoquinoline-3-carboxylic acid;
6-tert-butyl-10-methoxy-2-oxo-9-(2-propylthiazol-5-yl)-6,7-dihydro-2*H*-pyrido[2,1-*α*] isoquinoline-3-carboxylic acid;
6-tert-butyl-10-methoxy-9-[2-(methylcarbamoyl)thiazol-5-yl]-2-oxo-6,7-dihydro-2*H-*pyrido[2,1-*α*]isoquinoline-3-carboxylic acid;
6-tert-butyl-9-[2-(ethylcarbamoyl)thiazol-5-yl]-10-methoxy-2-oxo-6,7-dihydro-2*H-*pyrido[2,1-*α*]isoquinoline-3-carboxylic acid;
6-tert-butyl-10-methoxy-9-[2-(2-methoxyethoxy)thiazol-5-yl]-2-oxo-6,7-dihydro-2*H-*pyrido[2,1-*α*]isoquinoline-3-carboxylic acid;
6-tert-butyl-10-methoxy-9-{2-[(2-methoxyethyl)carbamoyl]thiazol-5-yl}-2-oxo-6,7-dihydro-2*H*-pyrido[2,1-*α*]isoquinoline-3-carboxylic acid;
6-tert-butyl-9-{2-[(difluoromethoxy)methyl]thiazol-5-yl}-10-methoxy-2-oxo-6,7-dihydro-2*H*-pyrido[2,1-*α*]isoquinoline-3-carboxylic acid;
6-tert-butyl-9-[2-(cyclopropoxymethyl)thiazol-5-yl]-10-methoxy-2-oxo-6,7-dihydro-2*H-*pyrido[2,1-*α*]isoquinoline-3-carboxylic acid;
6-tert-butyl-9-[2-(3,3-difluorocyclobutyl)thiazol-5-yl}-10-methoxy-2-oxo-6,7-dihydro-2*H*-pyiido[2,1-*α*]isoquinoline-3-carboxylic acid;
6-tert-butyl-9-[2-(3-fluorocyclobutyl)thiazol-5-yl]-10-methoxy-2-oxo-6,7-dihydro-2*H-*pyrido[2,1-*α*]isoquinoline-3-carboxylic acid; and
6-tert-butyl-10-methoxy-9-[2-(3-methoxycyclobutyl)thiazol-5-yl]-2-oxo-6,7-dihydro-2*H-*pyrido[2,1-*α*]isoquinoline-3-carboxylic acid.

The present disclosure further provides a pharmaceutical composition, comprising any aforementioned compound of general formula I, or the pharmaceutically acceptable salt thereof, the ester thereof, the isomer thereof, the solvate thereof, the hydrate thereof, the prodrug thereof, or the isotopically-labeled compound thereof.

In some embodiments of the present disclosure, the aforementioned pharmaceutical composition further comprises a pharmaceutically acceptable carrier.

In a more preferred embodiment, the aforementioned pharmaceutical composition further comprises:
- a pharmaceutically acceptable carrier;
- an adjuvant; and/or
- an excipient.

The present disclosure further provides a pharmaceutical formulation, comprising the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, wherein the formulation is any one of a tablet, a capsule, an injection, a granule, a pulvis, a suppository, a pill, a cream, a paste, a gel, a powder, an oral solution, an inhalant, a suspension, a dry suspension, a patch, and a lotion.

The present disclosure further provides the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation, which is used for preventing and treating viral hepatitis B.

The present disclosure further provides the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation for use in the prevention and/or treatment of viral hepatitis B.

The present disclosure further provides use of the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation in the preparation of a drug for preventing and/or treating viral hepatitis B.

The present disclosure further provides a method for preventing and/or treating viral hepatitis B, comprising the following step of administering a therapeutically effective amount of the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation to a patient in need thereof.

The term "therapeutically effective amount" refers to a dose of an active pharmaceutical ingredient capable of inducing the generation of a biological or medical response in a cell, a tissue, an organ or an organism (for example, a patient).

The term "administering" refers to a process of applying an active pharmaceutical ingredient (for example, a compound of the present disclosure) or a pharmaceutical composition comprising the active pharmaceutical ingredient (for example, a pharmaceutical composition of the present disclosure) to a patient or a part thereof (for example, a cell, a tissue, an organ, or a biofluid) so as to enable the active pharmaceutical ingredient or the pharmaceutical composition to contact with the patient or a part thereof (for example, a cell, a tissue, an organ, or a biofluid). Common modes of administration include (but not limited to) oral administration, subcutaneous administration, intramuscular administration, subperitoneal administration, ocular administration, nasal administration, sublingual administration, rectal administration, vaginal administration, and the like.

The term "in need thereof' refers to a judgment made by physicians or other nursing staffs that patients need benefits from or will benefit from the prevention and/or treatment process. This judgment is based on various factors in the fields of expertise of physicians or other nursing staffs.

The term "patient" (also referred to as subject) refers to human or non-human animal (for example, a mammal).

The present disclosure further provides a pharmaceutical combination, comprising the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation, and at least one additional therapeutic agent for viral hepatitis B.

Preferably, the aforementioned pharmaceutical combination further comprises at least one of the following substances:
HBV polymerase inhibitors; interferon α-2a; interferon α-2b; pegylated interferon α-2a; ribavirin; HBV preventive vaccines; HBV therapeutic vaccines; HBV capsid inhibitors; HBV RNA replication inhibitors; siRNA; inhibitors for HBsAg production or secretion; HBV antibodies or TLR7 agonists.

The present disclosure further provides a method for preventing and/or treating viral hepatitis B, comprising the following step of administering a therapeutically effective amount of the aforementioned compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof, or the aforementioned pharmaceutical composition, or the aforementioned pharmaceutical formulation, and at least one additional therapeutic agent for viral hepatitis B to a patient in need thereof.

The present disclosure further provides a preparation method of the compound of general formula I, or a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof. The technical solutions of the present disclosure are further described by describing a typical synthetic route of the compound as represented by general formula I. Specifically, the following can be seen from the reaction route as shown below.

Compound 1 is condensed with TsNHNH₂ to afford Product 2;
Product 2 reacts with aldehyde 3 in the presence of EtONa to afford Product 4;
Product 4 and ammonium acetate are subjected to reductive amination to afford Product 5;
Product 5 is acylated with formic acid to afford Amide 6;
Amide 6 is treated with oxalyl chloride and reacted in the presence of FeCl₃, and the isolated crude product reacts with concentrated sulfuric acid in a methanol solution to afford Compound 7;
Compound 7 and ethyl 2-(ethoxymethylene)-3-oxo-butyrate 8 undergo ring-closing reaction to afford Product 9;
Product 9 is subjected to oxidative dehydrogenation with tetrachlorobenzoquinone to afford Compound 10;
Compound 10 is coupled with bis(pinacolato)diboron to afford Product 11;
Product 11 is coupled with substituted bromothiazole to afford Product 12; and the ester group of Product 12 is hydrolyzed by sodium hydroxide or lithium hydroxide to afford Product 13.

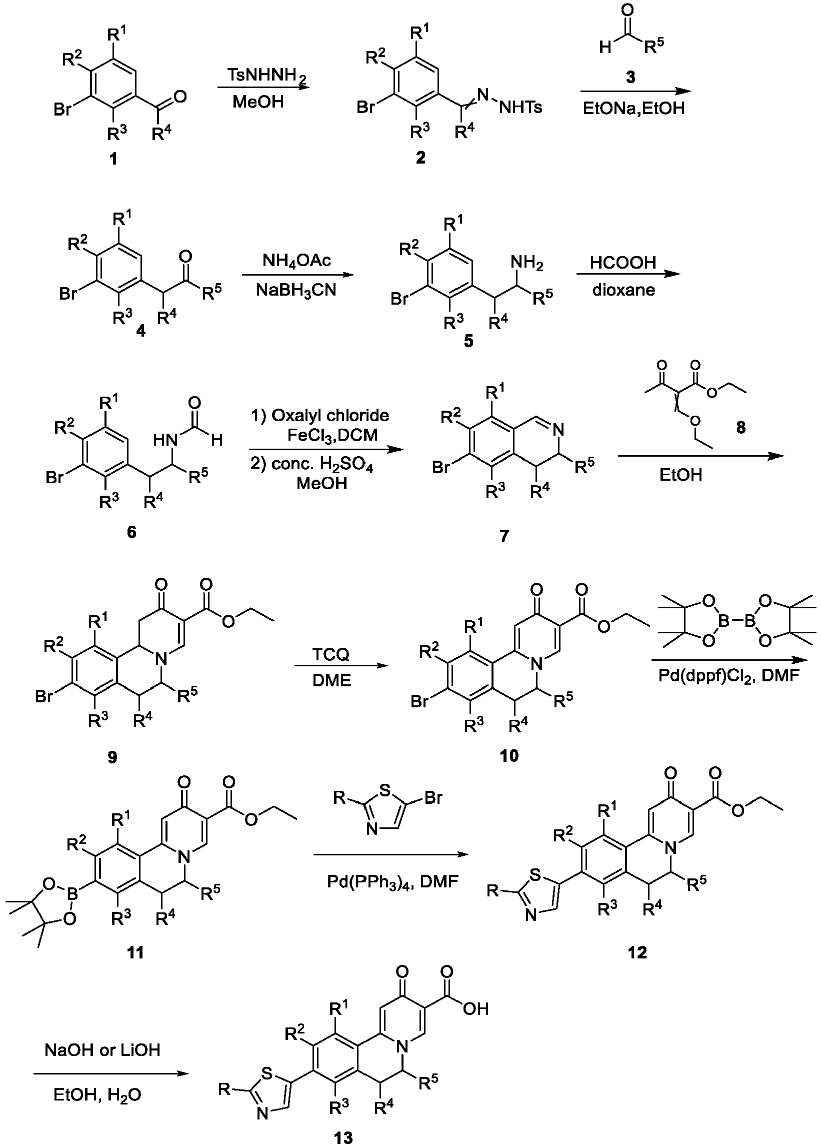

The present disclosure may be further described by the following Examples. However, these Examples should not be considered as limitations to the scope of the present disclosure.

The abbreviations used in the present disclosure are as follows:
AUC₀₋₁₂: area under the drug concentration-time curve from 0 hour to 12 hours
AUC_{INF}: area under the drug concentration-time curve from 0 hour extrapolated to infinity
CDCl₃: deuterated chloroform
dioxane: 1,4-dioxane
CL: apparent clearance
Cₘₐₓ: peak plasma drug concentration
CO₂: carbon dioxide
conc. H₂SO₄: concentrated sulfuric acid
DCM: dichloromethane
Dioxane: 1,4-dioxane
DME: ethylene glycol dimethyl ether
DMF: *N*,*N*-dimethylformamide
DMSO: dimethyl sulfoxide
DMSO-*d*₆: deuterated dimethyl sulfoxide
EtOH: ethanol
EtONa: sodium ethoxide
F: bioavailability
FeCl₃: ferric trichloride
g: gram
HCOOH: formic acid
Hz: hertz
h: hour
IC₅₀: half maximal inhibitory concentration
LiOH: lithium hydroxide
MeOH: methanol
mg: milligram
mL: milliliter
mL/hr/kg: milliliter/hour/kilogram
mL/kg: milliliter/kilogram
mmol: millimole
MHz: megahertz
NaBH₃CN: sodium cyanoborohydride
NaOH: sodium hydroxide
ng/mL^{∗}hr: nanogram/milliliter^{∗}hour
NH₄OAc: ammonium acetate
NMR: nuclear magnetic resonance
M: molar concentration
Oxalyl chloride: oxalyl chloride
PBS: phosphate-buffered saline solution
PdCl₂(dppf): [1,1'-bis(diphenylphosphino)ferrocene]palladium dichloride
Pd(PPh₃)₄: tetrakis(triphenylphosphine)palladium
t_{1/2}: elimination half-life
TCQ: tetrachlorobenzoquinone
Tₘₐₓ: time taken to reach peak plasma drug concentration
TLC: thin layer chromatography
TsNHNH₂: p-toluenesulfonyl hydrazide
µM: micromole/liter
Vss: apparent volume of distribution
µg: microgram
µL: microliter
δ: chemical shift

The general test conditions in the Examples of the present disclosure are described as below.

First, the reactions in the Examples were generally carried out under nitrogen atmosphere.

Further, the intermediates and the final products were separated and purified via a chromatographic column, a preparative chromatoplate, and an ICSO flash and preparative chromatography system.

Further, ACQUITY Arc System (Waters Co.) equipped with a QDa Detector was used as the LC-MS liquid chromatography-tandem mass spectrometer. ESI source was used in mass spectrometry (MS), and only the molecular weight M of the parent molecule was indicated and generally reported as [M+H]⁺. The injection volume was determined by the concentration of the sample; the flow rate was 1.2 mL/min; and the peak value of HPLC was recorded and read at UV-Vis wavelengths of 220 nm and 254 nm. The mobile phases were a solution of 0.01% formic acid in ultrapure water (mobile phase A) and a solution of 0.01% formic acid in acetonitrile (mobile phase B). Gradient elution conditions were as shown in the following Table 1 and Table 2.

**Table 1: Gradient elution condition 1**

| Time (min) | A (H₂O, 0.01% HCOOH) | B (CH₃CN, 0.01% HCOOH) |
|---|---|---|
| 0.0-0.3 | 95-85 | 5-15 |
| 0.3-3.2 | 85-20 | 15-80 |
| 3.2-3.8 | 20-5 | 80-95 |
| 3.8-3.81 | 5-95 | 95-5 |
| 3.81-4.0 | 95 | 5 |

**Table 2: Gradient elution condition 2**

| Time (min) | A (H₂O, 0.01% HCOOH) | B(CH₃CN, 0.01% HCOOH) |
|---|---|---|
| 0.00-5.90 | 95-5 | 5-95 |
| 5.90-5.91 | 5-95 | 95-5 |
| 5.91-6.00 | 95 | 5 |

Further, Varian 400MHz nuclear magnetic resonance spectrometer was utilized to obtain the NMR spectrum, CDCl₃ and DMSO-*d*₆ were often used as solvents, and chemical shifts were reported in ppm. Various peaks were described as follows: s (singlet), d (doublet), t (triplet), q (quartet), m (multiplet), dd (doublet of doublets). The coupling constant was expressed in Hz.

### Example 1:

6-tert-butyl-9-[2-(4-hydroxypiperidin-1-yl)thiazol-5-yl]-10-methoxy-2-oxo-6,7-dihydro-2*H*-pyiido[2,1-*α*]isoquinoline-3-carboxylic acid

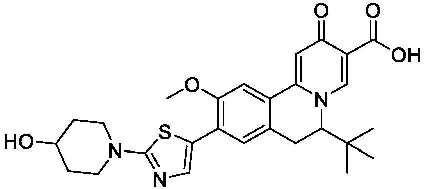

### Step 1a: Preparation of ethyl 6-tert-butyl-10-methoxy-2-oxo-9-(4,4,5,5-tetramethyl-1,3,2-dioxaborolan-2-yl)-6,7-dihydro-2H-pyrido[2,1-α]isoquinoline-3-carboxylate

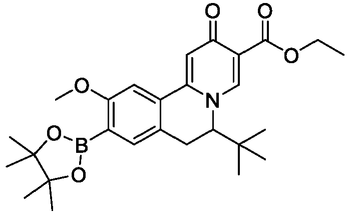

Under nitrogen atmosphere, to a solution of ethyl 9-bromo-6-tert-butyl-10-methoxy-2-oxo-6,7-dihydro-2*H*-pyrido[2,1-*α*]isoquinoline-3-carboxylate (please refer to Example 8 in WO 2018130152 for the preparation method) (3.0 g, 6.91 mmol) in 1,4-dioxane (50 mL) were added bis(pinacolato)diboron (3.5 g, 13.81 mmol), potassium acetate (1.4 g, 13.81 mmol) and [1,1'-bis(diphenylphosphino)ferrocene]palladium dichloride (505 mg, 0.69 mmol). The mixture was stirred at 100°C for 1 hour. The mixture was diluted with dichloromethane (100 mL), washed with water (100 mL) and saturated brine (100 mL), and then dried over anhydrous sodium sulfate, filtered and concentrated under reduced pressure to afford a crude product. The crude product was purified by column chromatography to afford crude ethyl 6-tert-butyl-10-methoxy-2-oxo-9-(4,4,5,5-tetramethyl-1,3,2- dioxaborolan-2-yl)-6,7-dihydro-2*H*-pyrido[2,1-*α*]isoquinoline-3-carboxylate (3 g) as black solid.

### Step 1b: Preparation of 1-(5-bromothiazol-2-yl)piperidin-4-ol

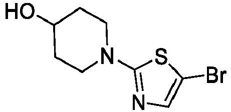

To a solution of 2,5-dibromothiazole (500 mg, 2.06 mmol) in 1,4-dioxane (30 mL) were added piperidin-4-ol (250 mg, 2.47 mmol) and *N*,*N* diisopropylethylamine (372 mg, 2.88 mmol). The mixture was stirred at 80°C for 8 hours. The mixture was diluted with ethyl acetate (100 mL), washed with water (100 mL) and saturated brine (100 mL), and then dried over anhydrous sodium sulfate, filtered and concentrated under reduced pressure to afford a crude product. The crude product was purified by column chromatography to afford 1-(5-bromothiazol-2-yl)piperidin-4-ol (310 mg) as brown solid.

### Step 1c: Preparation of ethyl 6-tert-butyl-9-[2-(4-hydroxypiperidin-1-yl)thiazol-5-yl]- 10-methoxy-2-oxo-6,7-dihydro-2H-pyrido[2,1-α]isoquinoline-3-carboxylate

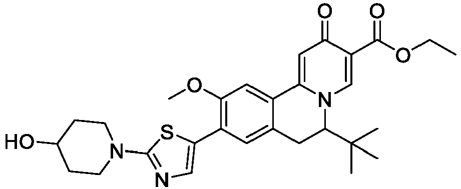

Under nitrogen atmosphere, to a solution of ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (100 mg, 0.21 mmol) in *N*,*N-*dimethylformamide (2 mL) were added 1-(5-bromothiazol-2-yl)piperidin-4-ol (82 mg, 0.31 mmol), potassium carbonate (58 mg, 0.41 mmol) and tetrakis(triphenylphosphine)palladium (48 mg, 0.04 mmol). The mixture was stirred at 90°C for 4 hours. The mixture was diluted with dichloromethane (50 mL), washed with water (50 mL) and saturated brine (50 mL), and then dried over anhydrous sodium sulfate, filtered and concentrated under reduced pressure to afford a crude product. The crude product was purified by column chromatography to afford ethyl 6-tert-butyl-9-[2-(4-hydroxypiperidin-1-yl)thiazol-5-yl]-10-methoxy-2-oxo-6,7-dihydro-2*H*-pyrido[2,1-*α*]isoquinoline-3-carboxylate (40 mg) as yellow solid.

### Step 1d: Preparation of 6-tert-butyl-9-[2-(4-hydroxypiperidin-1-yl)thiazol-5-yl] -10-methoxy-2-oxo-6,7-dihydro-2H-pyrido[2,1-a]isoquinoline-3-carboxylic acid

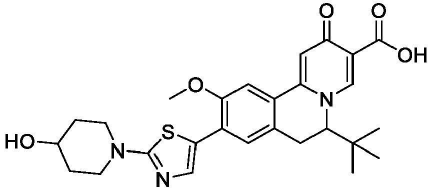

To a solution of ethyl 6-tert-butyl-9-[2-(4-hydroxypiperidin-1-yl)thiazol-5-yl] -10-methoxy-2-oxo-6,7-dihydro-2H-pyrido[2,1-*α*]isoquinoline-3-carboxylate (40 mg, 0.07 mmol) in ethanol (4 mL) was added 10% sodium hydroxide solution (1 mL). The mixture was stirred at room temperature for 2 hours. The mixture was concentrated to afford a crude product. The crude product was dissolved in water (10 mL), and the resulting solution was adjusted to pH 2 with 1 M hydrochloric acid solution and then extracted with dichloromethane (10 mL × 3). The organic phases were combined and washed with water (30 mL × 3), and then the resultant was concentrated to afford 6-tert-butyl-9-[2-(4-hydroxypiperidin-1-yl)thiazol-5-yl] -10-methoxy-2-oxo-6,7-dihydro-2*H*-pyrido[2,1-*α*]isoquinoline-3-carboxylic acid (9.9 mg) as yellow solid.

¹HNMR (DMSO-*d*₆, 400 MHz): δ 8.75 (s, 1 H), 7.89 (s, 1 H), 7.69 (s, 1 H), 7.60 (s, 2 H), 4.61 (d, *J* = 5.2 Hz, 1 H), 4.00 (s, 3 H), 3.83 - 3.72 (m, 5 H), 2.93 - 2.88 (m, 2 H), 1.86 - 1.81 (s, 2 H), 1.49 - 1.49 (m, 2 H), 0.74 (s, 9 H). MS measured value (ESI⁺) [(M+H)⁺]: 510.

### Example 2:

6-tert-butyl-9-{2-[4-(hydroxymethyl)pipendin-1-yl]thiazol-5-yl}-10-methoxy-2-oxo-6,7-dihydro-2*H*-pyrido[2,1-*α*]isoquinoline-3-carboxylicacid

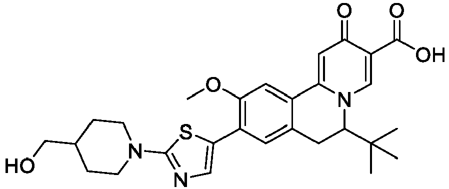

### Step 2a: Preparation of [1-(5-bromothiazol-2-yl)piperidin-4-yl]methanol

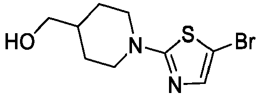

2,5-Dibromothiazole (100 mg, 0.41 mmol) and piperidin-4-ylmethanol (57 mg, 0.49 mmol) were used to prepare and afford [1-(5-bromothiazol-2-yl)piperidin-4-yl]methanol (51 mg) as white solid in accordance with a method similar to that in Step 1b of Example 1.

### Step 2b: Preparation of ethyl 6-tert-butyl-9-{2-[4-(hydroxymethyl)piperidin-1-yl] thiazol-5-yl}-10-methoxy-2-oxo-6,7-dihydro-2H-pyrido[2,1-α]isoquinoline-3-carboxylate

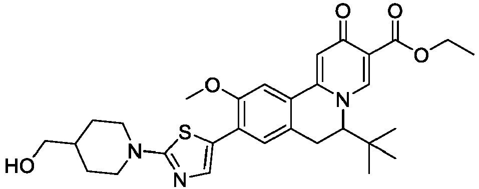

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (100 mg, 0.21 mmol) and [1-(5-bromothiazol-2-yl)piperidin-4-yl]methanol (58 mg, 0.21 mmol) were used to prepare and afford ethyl 6-tert-butyl-9-{2-[4-(hydroxymethyl)piperidin-1-yl]thiazol-5-yl}-10-methoxy -2-oxo-6,7-dihydro-2*H*-pyrido[2,1-*α*]isoquinoline-3-carboxylate (48 mg) as yellow solid in accordance with a method similar to that in Step 1c of Example 1.

### Step 2c: Preparation of 6-tert-butyl-9- {2-[4-(hydroxymethyl)piperidin-1-yl]thiazol-5-yl} - 10-methoxy-2-oxo-6,7-dihydro-2H-pyrido[2,1-α]isoquinoline-3-carboxylic acid

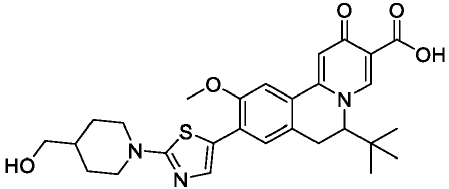

Ethyl 6-tert-butyl-9-{2-[4-(hydroxymethyl)piperidin-1-yl]thiazol-5-yl}-10-methoxy-2-oxo- 6,7-dihydro-2*H*-pyrido[2,1-*α*]isoquinoline-3-carboxylate (48 mg, 0.09 mmol) was used to prepare and afford 6-tert-butyl-9-{2-[4-(hydroxymethyl)piperidin-1-yl]thiazol-5-yl} -10-methoxy-2-oxo-6,7-dihydro-2*H*-pyrido[2,1-*α*]isoquinoline-3-carboxylic acid (11.2 mg) as yellow solid in accordance with a method similar to that in Step Id of Example 1. ¹HNMR (DMSO-*d*₆, 400 MHz): δ 8.75 (s, 1 H), 7.87 (s, 1 H), 7.67 (s, 1 H), 7.60 (s, 1 H), 7.59 (s, 1 H), 4.60 (d, *J =* 5.2 Hz, 1 H), 4.00 (s, 3 H), 3.96 (s, 1 H), 3.32 - 3.28 (m, 4 H), 3.07 (t*, J =* 12.0 Hz, 2 H), 1.77 (d, *J=* 12.0 Hz, 2 H), 1.65 (s, 1 H), 1.25 - 1.20 (m, 2 H), 0.74 (s, 9 H). MS measured value (ESI⁺) [(M+H)⁺]: 524.

### Example 3:

9-[2-(azetidin-1-yl)thiazol-5-yl]-6-tert-butyl-10-methoxy-2-oxo-6,7-dihydro-2*H-*pyrido[2,1-*α*]isoquinoline-3-carboxylic acid

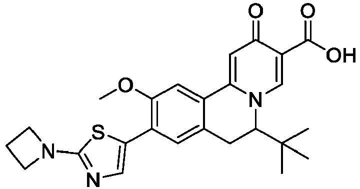

### Step 3a: Preparation of 2-(azetidin-1-yl)-5-bromothiazole

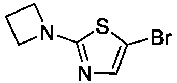

2,5-Dibromothiazole (300 mg, 1.23 mmol) and azetidine (85 mg, 1.48 mmol) were used to prepare and afford 2-(azetidin-1-yl)-5-bromothiazole (55 mg) as white solid in accordance with a method similar to that in Step 1b of Example 1.

### Step 3b: Preparation of ethyl 9-[2-(azetidin-1-yl)thiazol-5-yl]-6-tert-butyl-10-methoxy- 2-oxo-6,7-dihydro-2H-pyrido[2,1-α]isoquinoline-3-carboxylate

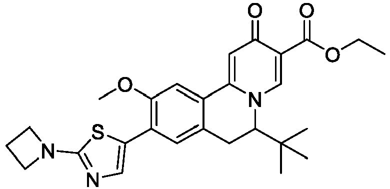

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (100 mg, 0.21 mmol) and 2-(azetidin-1-yl)-5-bromothiazole(50 mg, 0.23 mmol) were used to prepare and afford ethyl 9-[2-(azetidin-1-yl)thiazol-5-yl]-6-tert-butyl-10-methoxy-2-oxo-6,7-dihydro-2*H*-pyiido[2,1-*α*]isoquinoline-3-carboxylate (14 mg) as yellow solid in accordance with a method similar to that in Step 1c of Example 1.

### Step 3c: 9-[2-(azetidin-1-yl)thiazol-5-yl]-6-tert-butyl-10-methoxy-2-oxo-6,7-dihydro-2H-pyiido[2,1-α]isoquinoline-3-carboxylic acid

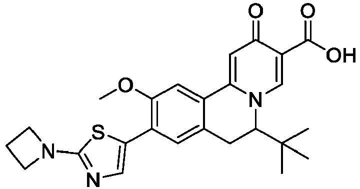

Ethyl 9-[2-(azetidin-1-yl)thiazol-5-yl]-6-tert-butyl-10-methoxy-2-oxo-6,7-dihydro-2*H-*pyrido[2,1-*α*]isoquinoline-3-carboxylate (14 mg, 0.03 mmol) was used to prepare and afford 9-[2-(azetidin-1-yl)thiazol-5-yl]-6-tert-butyl-10-methoxy-2-oxo-6,7-dihydro-2*H*-pyrido[2,1-*α*]isoquinoline-3-carboxylic acid (3.3 mg) as yellow solid in accordance with a method similar to that in Step Id of Example 1. ¹HNMR (DMSO-*d*₆, 400 MHz): δ 8.76 (s, 1 H), 7.94 (s, 1 H), 7.74 (s, 1 H), 7.63 (s, 1 H), 7.61 (s, 1 H), 4.61 (d, *J=* 6.4 Hz, 1 H), 4.16 - 4.12 (m, 4 H), 4.01 (s, 3 H), 3.42 - 3.35(m., 2 H), 2.46 (d, *J=* 6.8 Hz, 2 H), 0.74 (s, 9 H). MS measured value (ESI⁺) [(M+H)⁺]: 466.

### Example 4:

### 6-tert-butyl-9-[2-(3,3-difluoroazetidin-1-yl)thiazol-5-yl]-10-methoxy-2-oxo-6,7-dihydro-2H-pyiido[2,1-α]isoquinoline-3-carboxylic acid

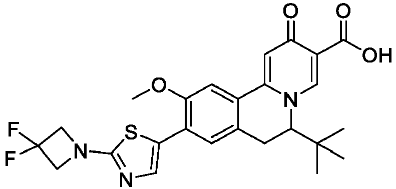

### Step 4a: Preparation of 2-(3,3-difluoroazetidin-1-yl)-5-bromothiazole

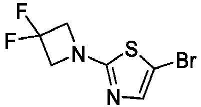

2,5-Dibromothiazole (300 mg, 1.23 mmol) and 3,3-difluoroazetidine (138 mg, 1.48 mmol) were used to prepare and afford 2-(3,3-difluoroazetidin-1-yl)-5-bromothiazole (260 mg) as white solid in accordance with a method similar to that in Step 1b of Example 1.

### Step 4b: Preparation of ethyl 6-tert-butyl-9-[2-(3,3-difluoroazetidin-1-yl)thiazol-5-yl]-10-methoxy-2-oxo-6,7-dihydro-2H-pyrido[2,1-α]isoquinoline-3-carboxylate

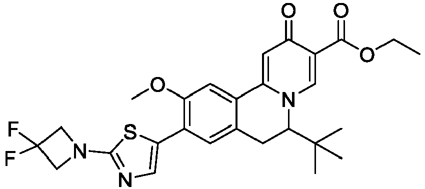

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (100 mg, 0.21 mmol) and 2-(3,3-difluoroazetidin-1-yl)-5-bromothiazole (58 mg, 0.23 mmol) were used to prepare and afford ethyl 6-tert-butyl-9-[2-(3,3-difluoroazetidin-1-yl)thiazol-5-yl]-10-methoxy-2-oxo- 6,7-dihydro-2*H*-pyrido[2,1-*α*]isoquinoline-3-carboxylate (70 mg) as yellow solid in accordance with a method similar to that in Step 1c of Example 1.

### Step 4c: Preparation of 6-tert-butyl-9-[2-(3,3-difluoroazetidin-1-yl)thiazol-5-yl]-10-methoxy-2-oxo-6,7-dihydro-2H-pyrido[2,1-α]isoquinoline-3-carboxylic acid

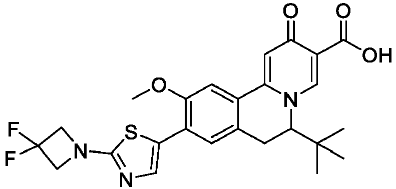

Ethyl 6-tert-butyl-9-[2-(3,3-difluoroazetidin-1-yl)thiazol-5-yl]-10-methoxy-2-oxo- 6,7-dihydro-2*H*-pyrido[2,1-*α*]isoquinoline-3-carboxylate (70 mg, 0.13 mmol) was used to prepare and afford 6-tert-butyl-9-[2-(3,3-difluoroazetidin-1-yl)thiazol-5-yl]-10-methoxy- 2-oxo-6,7-dihydro-2*H*-pyrido[2,1-*α*]isoquinoline-3-carboxylic acid (31.8 mg) as yellow solid in accordance with a method similar to that in Step Id of Example 1. ¹HNMR (DMSO-*d*₆, 400 MHz): δ 16.56 (s, 1 H), 8.76 (s, 1 H), 7.94 (s, 1 H), 7.79 (s, 1 H), 7.63 (s, 1 H), 7.61 (s, 1 H), 4.62 - 4.61 (m, 1 H), 4.58 - 4.52 (m, 4 H), 4.01 (s, 3 H) 3.38 - 3.33(m, 2 H), 0.74 (s, 9 H). MS measured value (ESI⁺) [(M+H)⁺]: 502.

### Example 5:

### 6-tert-butyl-10-methoxy-9-(2-morpholinothiazol-5-yl)-2-oxo-6,7-dihydro-2H-pyrido[2,1-α]isoquinoline-3-carboxylic acid

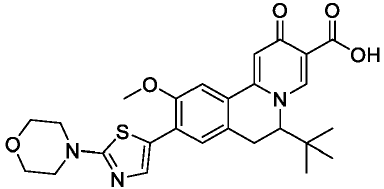

### Step 5a: Preparation of 4-(5-bromothiazol-2-yl)morpholine

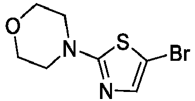

2,5-Dibromothiazole (300 mg, 1.23 mmol) and morpholine (129 mg, 1.48 mmol) were used to prepare and afford 4-(5-bromothiazol-2-yl)morpholine (300 mg) as white solid in accordance with a method similar to that in Step 1b of Example 1.

### Step 5b: Preparation of ethyl 6-tert-butyl-10-methoxy-9-(2-morpholinothiazol-5-yl) -2-oxo-6,7-dihydro-2H-pyrido[2,1-α]isoquinoline-3-carboxylate

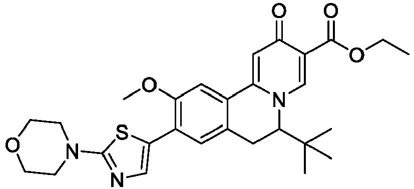

Ethyl 6-tert-butyl-10-methoxy-2-oxo-9-(4,4,5,5-tetramethyl-1,3,2-dioxaborolan-2-yl) - 6,7-dihydro-2*H*-pyrido[2,1-*α*]isoquinoline-3-carboxylate (100 mg, 0.21 mmol) and 4-(5-bromothiazol-2-yl)morpholine (62 mg, 0.23 mmol) were used to prepare and afford ethyl 6-tert-butyl-10-methoxy-9-(2-morpholinothiazol-5-yl)-2-oxo-6,7-dihydro-2*H*-pyrido[2,1-*α*]isoquinoline-3-carboxylate (100 mg) as yellow solid in accordance with a method similar to that in Step 1c of Example 1.

### Step 5c: Preparation of 6-tert-butyl-10-methoxy-9-(2-morpholinothiazol-5-yl)-2-oxo- 6,7-dihydro-2H-pyrido[2,1-α]isoquinoline-3-carboxylicacid

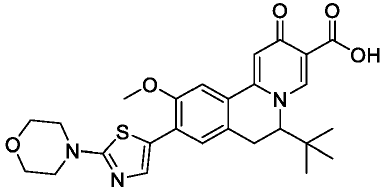

Ethyl 6-tert-butyl-10-methoxy-9-(2-morpholinothiazol-5-yl)-2-oxo-6,7-dihydro- 2H-pyrido[2,1-*α*]isoquinoline-3-carboxylate (100 mg, 0.19 mmol) was used to prepare and afford 6-tert-butyl-10-methoxy-9-(2-morpholinothiazol-5-yl)-2-oxo-6,7-dihydro-2*H*- pyrido[2,1-*α*]isoquinoline-3-carboxylic acid (29.1 mg) as yellow solid in accordance with a method similar to that in Step Id of Example 1. ¹HNMR (DMSO-*d*₆, 400 MHz): δ 8.75 (s, 1 H), 7.90 (s, 1 H), 7.70 (s, 1 H), 7.60 (s, 1 H), 7.59 (s, 1 H), 4.63 - 4.60 (m, 1 H), 4.00 (s, 3 H), 3.76 - 3.70 (m, 4 H), 3.48 - 3.42 (m, 4 H), 3.38 - 3.34 (m, 2 H), 0.74 (s, 9 H). MS measured value (ESI⁺) [(M+H)⁺]: 496.

### Example 6:

### 6-tert-butyl-9-[2-(dimethylamino)thiazol-5-yl]-10-methoxy-2-oxo-6,7-dihydro-2H-pyrido[2,1-α]isoquinoline-3-carboxylic acid

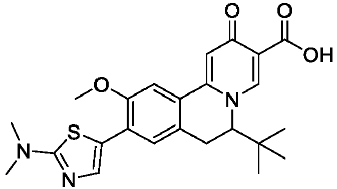

### Step 6a: Preparation of 5-bromo-2-(dimethylamino)thiazole

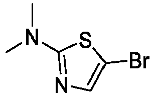

2,5-Dibromothiazole (300 mg, 2.47 mmol) and dimethylamine (1.447 g, 12.35 mmol) were used to prepare and afford 5-bromo-2-(dimethylamino)thiazole (128 mg) as white solid in accordance with a method similar to that in Step 1b of Example 1.

### Step 6b: Preparation of ethyl 6-tert-butyl-9-[2-(dimethylamino)thiazol-5-yl]-10- methoxy-2-oxo-6,7-dihydro-2H-pyrido[2,1-α]isoquinoline-3-carboxylate

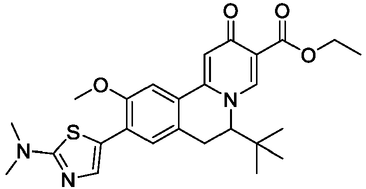

Ethyl 6-tert-butyl-10-methoxy-2-oxo-9-(4,4,5,5-tetramethyl-1,3,2-dioxaborolan-2-yl) - 6,7-dihydro-2*H*-pyrido[2,1-*α*]isoquinoline-3-carboxylate (250 mg, 0.52 mmol) and 5-bromo-2-(dimethylamino)thiazole (128 mg, 0.45 mmol) were used to prepare and afford ethyl 6-tert-butyl-9-[2-(dimethylamino)thiazol-5-yl]-10-methoxy-2-oxo-6,7-dihydro-2*H*- pyrido[2,1-*α*]isoquinoline-3-carboxylate (71 mg) as yellow solid in accordance with a method similar to that in Step 1c of Example 1.

### Step 6c: 6-tert-butyl-9-[2-(dimethylamino)thiazol-5-yl]-10-methoxy-2-oxo-6,7-dihydro-2H-pyiido[2,1-α]isoquinoline-3-carboxylic acid

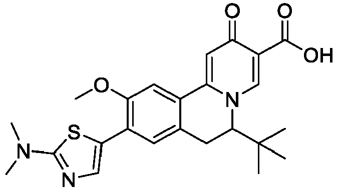

Ethyl 6-tert-butyl-9-[2-(dimethylamino)thiazol-5-yl]-10-methoxy-2-oxo-6,7-dihydro- 2H-pyrido[2,1-*α*]isoquinoline-3-carboxylate (71 mg, 0.15 mmol) was used to prepare and afford 6-tert-butyl-9-[2-(dimethylamino)thiazol-5-yl]-10-methoxy-2-oxo-6,7-dihydro-2*H*- pyrido[2,1-*α*]isoquinoline-3-carboxylic acid (30.2 mg) as yellow solid in accordance with a method similar to that in Step Id of Example 1. ¹HNMR (DMSO-*d*₆, 400 MHz): δ 16.61 (s, 1 H), 8.75 (s, 1 H), 7.87 (s, 1 H), 7.65 (s, 1 H), 7.58 (s, 2 H), 4.60 (d, *J=* 5.2 Hz, 1 H), 4.00 (s, 3 H), 3.49 (d, *J=* 5.2 Hz, 2 H), 3.10 (s, 6 H), 0.74 (s, 9 H). MS measured value (ESI⁺) [(M+H)⁺]: 454.

### Example 7:

6-tert-butyl-9-[2-(4-hydroxy-4-methylpiperidin-1-yl)thiazol-5-yl]-10-methoxy-2-oxo-6,7-dihydro-2*H*-pyrido[2,1-*α*]isoquinoline-3-carboxylic acid

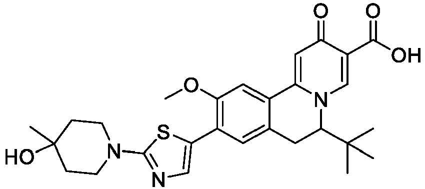

### Step 7a: Preparation of 1-(5-bromothiazol-2-yl)-4-methylpiperidin-4-ol

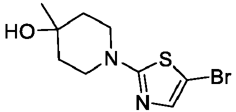

2,5-Dibromothiazole (500 mg, 2.06 mmol) and 4-methylpiperidin-4-ol (285 mg, 2.47 mmol) were used to prepare and afford 1-(5-bromothiazol-2-yl)-4-methylpiperidin-4-ol (390 mg) as white solid in accordance with a method similar to that in Step 1b of Example 1.

### Step 7b: Preparation of ethyl 6-tert-butyl-9-[2-(4-hydroxy-4-methylpiperidin-1-yl) thiazol-5-yl]-10-methoxy-2-oxo-6,7-dihydro-2H-pyrido[2,1-α]isoquinoline-3-carboxylate

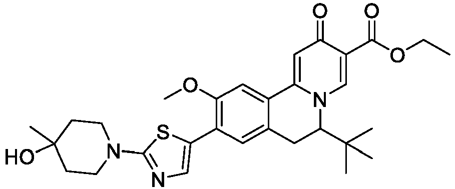

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (150 mg, 0.32 mmol) and 1-(5-bromothiazol-2-yl)-4-methylpiperidin-4-ol (104 mg, 0.37 mmol) were used to prepare and afford ethyl 6-tert-butyl-9-[2-(4-hydroxy-4-methylpiperidin-1-yl)thiazol-5-yl]-10-methoxy- 2-oxo-6,7-dihydro-2*H*-pyrido[2,1-*α*]isoquinoline-3-carboxylate (100 mg) as yellow solid in accordance with a method similar to that in Step 1c of Example 1.

### Step 7c: Preparation of 6-tert-butyl-9-[2-(4-hydroxy-4-methylpiperidin-1-yl)thiazol-5-yl] -10-methoxy-2-oxo-6,7-dihydro-2H-pyrido[2,1-α]isoquinoline-3-carboxylic acid

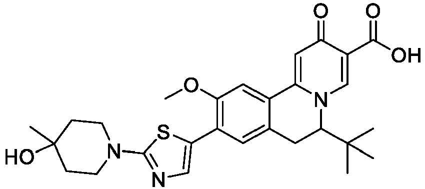

Ethyl 6-tert-butyl-9-[2-(4-hydroxy-4-methylpiperidin-1-yl)thiazol-5-yl]-10-methoxy-2-oxo-6,7-dihydro-2*H*-pyrido[2,1-*α*]isoquinoline-3-carboxylate (100 mg, 0.18 mmol) was used to prepare and afford 6-tert-butyl-9-[2-(4-hydroxy-4-methylpiperidin-1-yl)thiazol-5-yl] -10-methoxy-2-oxo-6,7-dihydro-2*H*-pyrido[2,1-*α*]isoquinoline-3-carboxylic acid (65.9 mg) as yellow solid in accordance with a method similar to that in Step Id of Example 1. ¹HNMR (DMSO-*d*₆, 400 MHz): δ 8.76 (s, 1 H), 7.90 (s, 1 H), 7.70 (s, 1 H), 7.61 (s, 1 H), 7.60 (s, 1 H), 4.61 (d*, J =* 5.6 Hz, 1 H), 4.01 (s, 3 H), 3.71 - 3.70 (m, 2 H), 3.50 - 3.32 (m, 4 H), 1.62 - 1.56 (m., 4 H), 1.18 (s, 3 H), 0.74 (s, 9 H). MS measured value (ESI⁺) [(M+H)⁺]: 524.

### Example 8:

### 6-tert-butyl-10-methoxy-9-[2-(4-methoxypiperidin-1-yl)thiazol-5-yl]-2-oxo-6,7-dihydro-2H-pyiido[2,1-α]isoquinoline-3-carboxylic acid

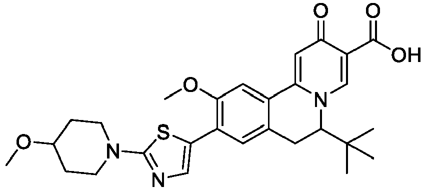

### Step 8a: Preparation of 5-bromo-2-(4-methoxypiperidin-1-yl)thiazole

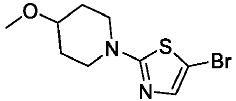

2,5-Dibromothiazole (500 mg, 2.06 mmol) and 4-methoxypiperidine (285 mg, 2.47 mmol) were used to prepare and afford 5-bromo-2-(4-methoxypiperidin-1-yl)thiazole (390 mg) as white solid in accordance with a method similar to that in Step 1b of Example 1.

### Step 8b: Preparation of ethyl 6-tert-butyl-10-methoxy-9-[2-(4-methoxypiperidin-1-yl)thiazol-5-yl]-2-oxo-6,7-dihydro-2H-pyrido[2,1-α]isoquinoline-3-carboxylate

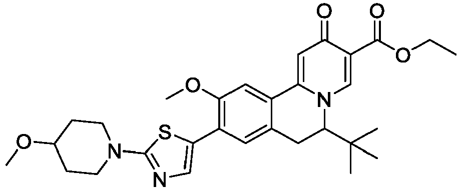

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (150 mg, 0.32 mmol) and 5-bromo-2-(4-methoxypiperidin-1-yl)thiazole (104 mg, 0.37 mmol) were used to prepare and afford ethyl 6-tert-butyl-10-methoxy-9-[2-(4-methoxypiperidin-1-yl)thiazol-5-yl] -2-oxo-6,7-dihydro-2*H-*pyrido[2,1-*α*]isoquinoline-3-carboxylate (100 mg) as yellow solid in accordance with a method similar to that in Step 1c of Example 1.

### Step 8c: Preparation of 6-tert-butyl-10-methoxy-9-[2-(4-methoxypiperidin-1-yl)thiazol- 5-yl]-2-oxo-6,7-dihydro-2H-pyrido[2,1-α]isoquinoline-3-carboxylic acid

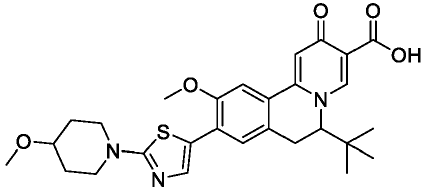

Ethyl 6-tert-butyl-10-methoxy-9-[2-(4-methoxypiperidin-1-yl)thiazol-5-yl]-2-oxo-6,7-dihydro-2*H*-pyrido[2,1-*α*]isoquinoline-3-carboxylate (100 mg, 0.18 mmol) was used to prepare and afford 6-tert-butyl-10-methoxy-9-[2-(4-methoxypiperidin-1-yl)thiazol-5-yl] -2-oxo-6,7-dihydro-2*H*-pyrido[2,1-*α*]isoquinoline-3-carboxylic acid (72.1 mg) as yellow solid in accordance with a method similar to that in Step Id of Example 1. ¹HNMR (DMSO-*d*₆, 400 MHz): δ 8.75 (s, 1 H), 7.88 (s, 1 H), 7.68 (s, 1 H), 7.60 (s, 1 H), 7.59 (s, 1 H), 4.61 (d, *J=* 4.8 Hz, 1 H), 4.00 (s, 3 H), 3.76 - 3.75 (m, 4 H), 3.48 - 3.45 (m, 1 H), 3.36 - 3.33 (m, 2 H), 3.29 (s, 3 H), 1.95 - 1.92 (m., 2 H), 1.56 - 1.54 (m, 2 H), 0.74 (s, 9 H). MS measured value (ESI⁺) [(M+H)⁺]: 524.

### Example 9:

### 6-tert-butyl-10-methoxy-9-[2-(3-methoxyazetidin-l-yl)thiazol-5-yl]-2-oxo-6,7-dihydro-2H-pyiido[2,1-α]isoquinoline-3-carboxylic acid

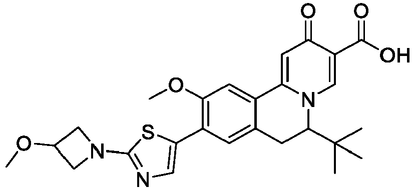

### Step 9a: Preparation of 5-bromo-2-(3-methoxyazetidin-1-yl)thiazole

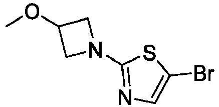

2,5-Dibromothiazole (500 mg, 2.06 mmol) and 3-methoxy azetidine (215 mg, 2.47 mmol) were used to prepare and afford 5-bromo-2-(3-methoxyazetidin-1-yl)thiazole (370 mg) as white solid in accordance with a method similar to that in Step 1b of Example 1.

### Step 9b: Preparation of ethyl 6-tert-butyl-10-methoxy-9-[2-(3-methoxyazetidin-1-yl)thiazol-5-yl]-2-oxo-6,7-dihydro-2H-pyrido[2,1-α]isoquinoline-3-carboxylate

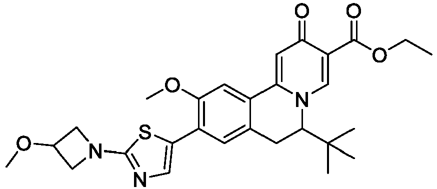

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (150 mg, 0.32 mmol) and 5-bromo-2-(3-methoxyazetidin-1-yl)thiazole (93 mg, 0.37 mmol) were used to prepare and afford ethyl 6-tert-butyl-10-methoxy-9-[2-(3-methoxyazetidin-1-yl)thiazol-5-yl]-2- oxo-6,7-dihydro-2*H-*pyrido[2,1-*α*]isoquinoline-3-carboxylate (100 mg) as yellow solid in accordance with a method similar to that in Step 1c of Example 1.

### Step 9c: Preparation of 6-tert-butyl-10-methoxy-9-[2-(3-methoxyazetidin-1-yl)thiazol- 5-yl]-2-oxo-6,7-dihydro-2H-pyrido[2,1-α]isoquinoline-3-carboxylic acid

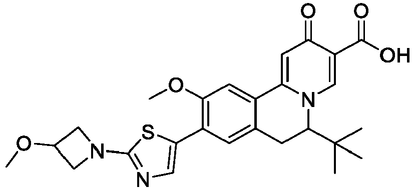

Ethyl 6-tert-butyl-10-methoxy-9-[2-(3-methoxyazetidin-1-yl)thiazol-5-yl]-2-oxo-6,7-dihydro-2*H*-pyrido[2,1-*α*]isoquinoline-3-carboxylate (100 mg, 0.18 mmol) was used to prepare and afford 6-tert-butyl-10-methoxy-9-[2-(3-methoxyazetidin-1-yl)thiazol-5-yl]-2-oxo- 6,7-dihydro-2*H*-pyrido[2,1-*α*]isoquinoline-3-carboxylic acid (30.9 mg) as yellow solid in accordance with a method similar to that in Step Id of Example 1. ¹HNMR (DMSO-*d*₆, 400 MHz): δ 16.59 (s, 1 H), 8.75 (s, 1 H), 7.87 (s, 1 H), 7.71 (s, 1 H), 7.60 (s, 1 H), 7.59 (s, 1 H), 4.61 (d, *J=* 6.0 Hz, 1 H), 4.42 - 4.38 (m, 1 H), 4.28 - 4.23 (m, 2 H), 4.00 (s, 3 H), 3.90 - 3.88 (m, 2 H), 3.38 - 3.34 (m, 2 H), 3.26 (s, 3 H), 0.74 (s, 9 H). MS measured value (ESI⁺) [(M+H)⁺]: 496.

### Example 10:

### 6-tert-butyl-10-methoxy-9-[2-(methoxymethyl)thiazol-5-yl]-2-oxo-6,7-dihydro-2H-pyrido[2,1-α]isoquinoline-3-carboxylic acid

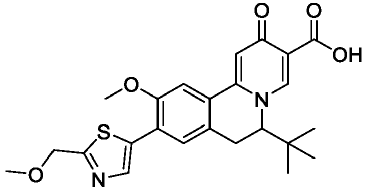

### Step 10a: Preparation of thiazol-2-ylmethanol

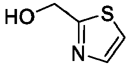

Sodium borohydride (800 mg, 21.21 mmol) was added to a solution of thiazole-2-carboxaldehyde (1.0 g, 8.84 mmol) in methanol (10 mL) at 0°C. The mixture was stirred at room temperature for 2 hours. The mixture was quenched with water (10 mL), and ethyl acetate (50 mL) was added for dilution. Afterwards, the resultant was washed with water (50 mL) and saturated brine (50 mL), and then dried over anhydrous sodium sulfate, filtered and concentrated under reduced pressure to afford a crude product. The crude product was purified by column chromatography to afford thiazol-2-ylmethanol (1.0 g) as white solid.

### Step 10b: Preparation of 2-(methoxymethyl)thiazole

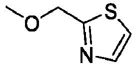

60% Sodium hydride (521 mg, 13.30 mmol) was added to a solution of thiazol-2-ylmethanol (1.0 g, 8.68 mmol) in tetrahydrofuran (10 mL) at 0°C. The mixture was stirred at room temperature for half an hour. Iodomethane (0.6 mL, 9.55 mmol) was then added to the solution. The mixture was stirred at room temperature for 2 hours. The mixture was quenched with water (10 mL), and ethyl acetate (50 mL) was added for dilution. Afterwards, the resultant was washed with water (50 mL) and saturated brine (50 mL), and then dried over anhydrous sodium sulfate, filtered and concentrated under reduced pressure to afford a crude product. The crude product was purified by column chromatography to afford 2-(methoxymethyl)thiazole (920 mg) as colorless liquid.

### Step 10c: Preparation of 5-bromo-2-(methoxymethyl)thiazole

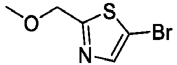

To a solution of 2-(methoxymethyl)thiazole (920 mg, 7.12 mmol) in *N,N-*dimethylformamide (5 mL) was added N-bromosuccinimide (1.9 g, 10.68 mmol). The mixture was stirred at room temperature for 1 hour. The mixture was diluted with ethyl acetate (50 mL), washed with water (50 mL) and saturated brine (50 mL), and then dried over anhydrous sodium sulfate, filtered and concentrated under reduced pressure to afford a crude product. The crude product was purified by column chromatography to afford 5-bromo-2-(methoxymethyl)thiazole (900 mg) as white solid.

### Step 10d: Preparation of ethyl 6-tert-butyl-10-methoxy-9-[2-(methoxymethyl)thiazol- 5-yl]-2-oxo-6,7-dihydro-2H-pyrido[2,1-α]isoquinoline-3-carboxylate

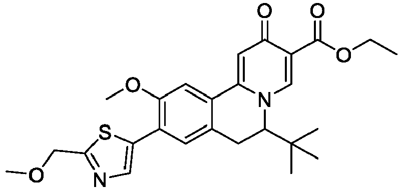

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (150 mg, 0.32 mmol) and 5-bromo-2-(methoxymethyl)thiazole (78 mg, 0.37 mmol) were used to prepare and afford ethyl 6-tert-butyl-10-methoxy-9-[2-(methoxymethyl)thiazol-5-yl]-2-oxo-6,7-dihydro-2*H*-pyrido[2,1-*α*]isoquinoline-3-carboxylate (150 mg) as yellow solid in accordance with a method similar to that in Step 1c of Example 1.

### Step 10e: Preparation of 6-tert-butyl-10-methoxy-9-[2-(methoxymethyl)thiazol-5-yl]-2-oxo-6,7-dihydro-2H-pyrido[2,1-α]isoquinoline-3-carboxylic acid

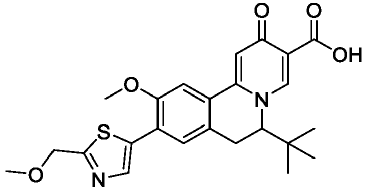

Ethyl 6-tert-butyl-10-methoxy-9-[2-(methoxymethyl)thiazol-5-yl]-2-oxo-6,7-dihydro-2*H*-pyrido[2,1-*α*]isoquinoline-3-carboxylate (150 mg, 0.31 mmol) was used to prepare and afford 6-tert-butyl-10-methoxy-9-[2-(methoxymethyl)thiazol-5-yl]-2-oxo-6,7-dihydro-2*H-*pyrido[2,1-*α*]isoquinoline-3-carboxylic acid (49.8 mg) as yellow solid in accordance with a method similar to that in Step Id of Example 1. ¹HNMR (DMSO-*d*₆, 400 MHz): δ 8.78 (s, 1 H), 8.38 (s, 1 H), 7.95 (s, 1 H), 7.70 (s, 1 H), 7.67 (s, 1 H), 4.74 (s, 2 H), 4.64 (d, *J=* 5.6 Hz, 1 H), 4.05 (s, 3 H), 3.42 (s, 3 H), 3.39 - 3.32 (m, 2 H), 0.74 (s, 9 H). MS measured value (ESI⁺)

[(M+H)⁺]: 455.

### Example 11:

### 6-tert-butyl-9-[2-(3-fluoroazetidin-1-yl)thiazol-5-yl]-10-methoxy-2-oxo-6,7-dihydro-2H-pyrido[2,1-α]isoquinoline-3-carboxylic acid

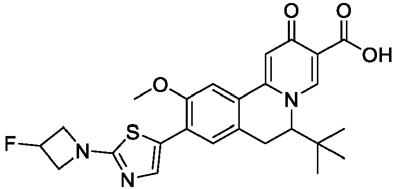

### Step 11a: Preparation of 5-bromo-2-(3-fluoroazetidin-1-yl)thiazole

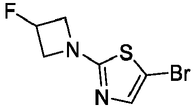

2,5-Dibromothiazole (500 mg, 2.06 mmol) and 3-fluoroazetidine (185 mg, 2.47 mmol) were used to prepare and afford 5-bromo-2-(3-fluoroazetidin-1-yl)thiazole (210 mg) as white solid in accordance with a method similar to that in Step 1b of Example 1.

### Step 11b: Preparation of ethyl 6-tert-butyl-9-[2-(3-fluoroazetidin-1-yl)thiazol-5-yl]-10-methoxy-2-oxo-6,7-dihydro-2H-pyrido[2,1-α]isoquinoline-3-carboxylate

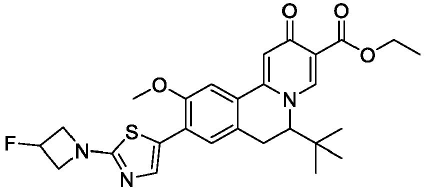

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*α*]isoquinoline-3-carboxylate (150 mg, 0.32 mmol) and 5-bromo-2-(3-fluoroazetidin-1-yl)thiazole(89 mg, 0.37 mmol) were used to prepare and afford ethyl 6-tert-butyl-9-[2-(3-fluoroazetidin-1-yl)thiazol-5-yl]-10-methoxy-2-oxo-6,7-dihydro- 2*H*-pyrido[2,1-*α*]isoquinoline-3-carboxylate (100 mg) as yellow solid in accordance with a method similar to that in Step 1c of Example 1.

### Step 11c: Preparation of 6-tert-butyl-9-[2-(3-fluoroazetidin-l-yl)thiazol-5-yl]-10-methoxy-2-oxo-6,7-dihydro-2H-pyrido[2,1-α]isoquinoline-3-carboxylic acid

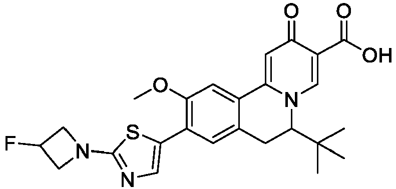

Ethyl 6-tert-butyl-9-[2-(3-fluoroazetidin-1-yl)thiazol-5-yl]-10-methoxy-2-oxo-6,7-dihydro-2*H*-pyrido[2,1-*α*]isoquinoline-3-carboxylate (100 mg, 0.19 mmol) was used to prepare and afford ethyl 6-tert-butyl-9-[2-(3-fluoroazetidin-1-yl)thiazol-5-yl]-10- methoxy-2-oxo-6,7-dihydro-2*H*-pyrido[2,1-*α*]isoquinoline-3-carboxylate (61.9 mg) as yellow solid in accordance with a method similar to that in Step Id of Example 1. ¹HNMR (DMSO-*d*₆, 400 MHz): δ 16.57 (s, 1 H), 8.75 (s, 1 H), 7.89 (s, 1 H), 7.73 (s, 1 H), 7.61 (s, 1 H), 7.60 (s, 1 H), 5.56 (d, *J=* 56.0 Hz, 1 H), 4.61 (d*, J* = 5.2 Hz, 1 H), 4.44 - 4.34 (m, 2 H), 4.21 - 4.12 (m, 2 H), 4.00 (s, 3 H), 3.42 - 3.33 (m, 2 H), 0.74 (s, 9 H). MS measured value (ESI⁺) [(M+H)⁺]: 484.

### Example 12:

### 6-tert-butyl-10-methoxy-9-(2-methoxythiazol-5-yl)-2-oxo-6,7-dihydro-2H-pyrido[2,1-α]isoquinoline-3-carboxylic acid

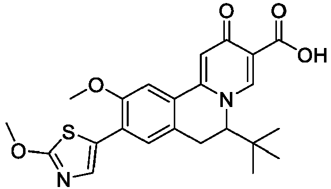

### Step 12a: Preparation of 5-bromo-2-methoxythiazole

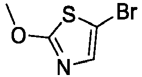

To a solution of 2,5-dibromothiazole (500 mg, 2.06 mmol) in anhydrous methanol (5 mL) was added sodium methoxide (333.6 mg, 6.17 mmol). The reaction mixture was heated to 30°C and was stirred at 30°C for 12 hours. The reaction was quenched with 5 mL of water, and then 20 mL of water was added. The organic phase was separated, and the aqueous phase was then extracted with 20 mL of ethyl acetate three times. The combined organic phase was concentrated and then subjected to column chromatography to afford 5-bromo-2-methoxythiazole (230 mg) as colorless oil.

### Step 12b: Preparation of ethyl 6-tert-butyl-10-methoxy-9-(2-methoxythiazol-5-yl)-2- oxo-6,7-dihydro-2H-pyrido[2,1-α]isoquinoline-3-carboxylate

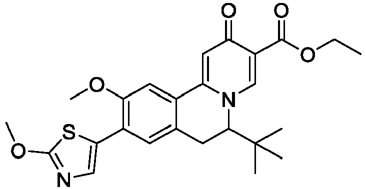

Ethyl 6-tert-butyl-10-methoxy-2-oxo-9-(4,4,5,5-tetramethyl-1,3,2-dioxaborolan-2-yl) - 6,7-dihydro-2*H*-pyrido[2,1-*α*]isoquinoline-3-carboxylate (150 mg, 0.375 mmol) and 5-bromo-2-methoxythiazole (73 mg, 0.375 mmol) were used to prepare and afford ethyl 6-tert-butyl-10-methoxy-9-(2-methoxythiazol-5-yl)-2-oxo-6,7-dihydro-2*H*-pyrido[2,1-*α*]isoquinoline-3-carboxylate (110 mg) as yellow solid in accordance with a method similar to that in Step 1c of Example 1.

### Step 12c: Preparation of 6-tert-butyl-10-methoxy-9-(2-methoxythiazol-5-yl)-2-oxo- 6,7-dihydro-2H-pyrido[2,1-α]isoquinoline-3-carboxylicacid

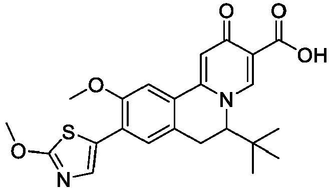

Ethyl 6-tert-butyl-10-methoxy-9-(2-methoxythiazol-5-yl)-2-oxo-6,7-dihydro-2*H-*pyrido[2,1-*α*]isoquinoline-3-carboxylate (110 mg, 0.234 mmol) was used to prepare and afford 6-tert-butyl-10-methoxy-9-(2-methoxythiazol-5-yl)-2-oxo-6,7-dihydro-2*H*- pyrido[2,1-*α*]isoquinoline-3-carboxylic acid (40 mg) as yellowish solid in accordance with a method similar to that in Step Id of Example 1. ¹H NMR (DMSO-*d*₆, 400 MHz): δ 8.77 (s, 1 H), 7.88 (s, 1 H), 7.82 (s, 1 H), 7.66 (s, 1 H), 7.64 (s, 1 H), 4.63 - 4.62 (m, 1 H), 4.06 (s, 3 H), 4.02 (s, 3 H), 3.39 - 3.34 (m, 2 H), 0.74 (s, 9 H). MS measured value (ESI⁺) [(M+H)⁺]: 441.

### Example 13:

### 6-tert-butyl-9-[2-(ethoxy)thiazol-5-yl]-10-methoxy-2-oxo-6,7-dihydro-2H-pyrido[2,1-α]isoquinoline-3-carboxylic acid

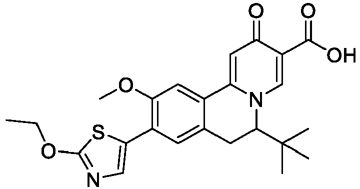

### Step 13a: Preparation of 5-bromo-2-ethoxythiazole

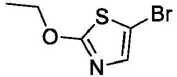

2,5-Dibromothiazole (500 mg, 2.06 mmol) and sodium ethoxide (420.21 mg, 6.17 mmol) were used to prepare and afford 5-bromo-2-ethoxythiazole (95 mg) as colorless oily liquid in accordance with a method similar to that in Step 12a of Example 12.

### Step 13b: Preparation of ethyl 6-tert-butyl-9-[2-(ethoxy)thiazol-5-yl]-10-methoxy-2- oxo-6,7-dihydro-2H-pyrido[2,1-α]isoquinoline-3-carboxylate

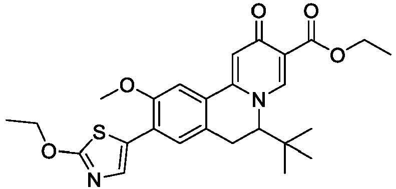

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (180 mg, 0.45 mmol) and 5-bromo-2-ethoxythiazole (94 mg, 0.45 mmol) were used to prepare and afford ethyl 6-tert-butyl-9-[2-(ethoxy)thiazol-5-yl]-10-methoxy-2-oxo-6,7-dihydro-2*H*-pyrido[2,1-*a*]isoquinoline-3-carboxylate (120 mg) as yellow solid in accordance with a method similar to that in Step 1c of Example 1.

### Step 13c: Preparation of 6-tert-butyl-9-[2-(ethoxy)thiazol-5-yl]-10-methoxy-2-oxo- 6,7-dihydro-2H-pyrido[2,1-a]isoquinoline-3-carboxylic acid

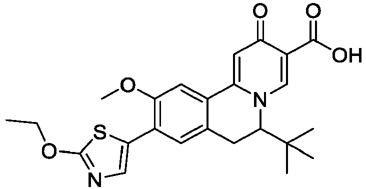

Ethyl 6-tert-butyl-9-[2-(ethoxy)thiazol-5-yl]-10-methoxy-2-oxo-6,7-dihydro-2*H-*pyrido[2,1-*a*]isoquinoline-3-carboxylate (120 mg, 0.25 mmol) was used to prepare and afford 6-tert-butyl-9-[2-(ethoxy)thiazol-5-yl]-10-methoxy-2-oxo-6,7-dihydro-2*H*- pyrido[2,1-*a*]isoquinoline-3-carboxylic acid (50 mg) as yellowish solid in accordance with a method similar to that in Step Id of Example 1. ¹H NMR (DMSO-*d₆*, 400 MHz): δ 8.77 (s, 1 H), 7.86 (s, 1 H), 7.80 (s, 1 H), 7.65 (s, 1 H), 7.63 (s, 1 H), 4.62 - 4.61 (m, 1 H), 4.48 - 4.43 (m, 2 H), 4.01 (s, 3 H), 3.46 - 3.38 (m, 2 H), 1.38 (t, *J* = 8.0 Hz, 3 H), 0.74 (s, 9 H). MS measured value (ESI⁺) [(M+H)⁺]: 455.

### Example 14:

### 6-tert-butyl-9-[2-(ethoxymethyl)thiazol-5-yl]-10-methoxy-2-oxo-6,7-dihydro-2H-pyrido[2,1-a]isoquinoline-3-carboxylic acid

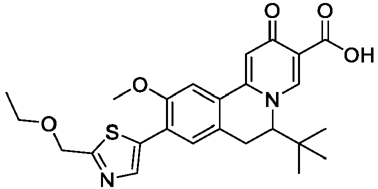

### Step 14a: Preparation of 2-ethoxymethylthiazole

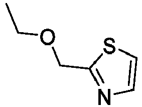

2-Thiazolemethanol (450 mg, 3.91 mmol) and iodoethane (914.3 mg, 5.86 mmol) were used to prepare and afford 2-ethoxymethylthiazole (330 mg) as colorless oily liquid in accordance with a method similar to that in Step 10b of Example 10.

### Step 14b: Preparation of 5-bromo-2-ethoxymethylthiazole

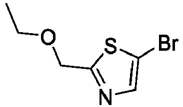

2-Ethoxymethylthiazole (0.33 g, 2.30 mmol) was used to prepare and afford 5-bromo-2-ethoxymethylthiazole (110 mg) as white solid in accordance with a method similar to that in Step 10c of Example 10.

### Step 14c: Preparation of ethyl 6-tert-butyl-9-[2-(ethoxymethyl)thiazol-5-yl]-10-methoxy-2-oxo-6,7-dihydro-2H-pyrido[2,1-a]isoquinoline-3-carboxylate

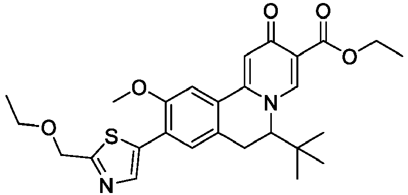

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (180 mg, 0.45 mmol) and 5-bromo-2-ethoxymethylthiazole (110 mg, 0.50 mmol) were used to prepare and afford ethyl 6-tert-butyl-9-[2-(ethoxymethyl)thiazol-5-yl]-10-methoxy-2-oxo-6,7-dihydro-2*H*-pyrido[2,1-*a*]isoquinoline-3-carboxylate (80 mg) as yellow solid in accordance with a method similar to that in Step 1c of Example 1.

### Step 14d: Preparation of 6-tert-butyl-9-[2-(ethoxymethyl)thiazol-5-yl]-10-methoxy-2-oxo-6,7-dihydro-2H-pyrido[2,1-a]isoquinoline-3-carboxylic acid

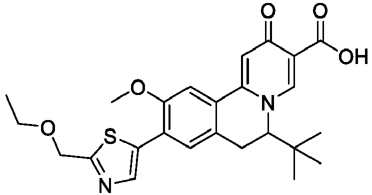

Ethyl 6-tert-butyl-9-[2-(ethoxymethyl)thiazol-5-yl]-10-methoxy-2-oxo-6,7-dihydro-2*H-*pyrido[2,1-*a*]isoquinoline-3-carboxylate (80 mg, 0.16 mmol) was used to prepare and afford 6-tert-butyl-9-[2-(ethoxymethyl)thiazol-5-yl]-10-methoxy-2-oxo-6,7-dihydro-2*H*-pyrido[2,1-*a*]isoquinoline-3-carboxylic acid (30 mg) as white solid in accordance with a method similar to that in Step Id of Example 1. ¹H NMR (DMSO-*d₆*, 400 MHz): δ 8.78 (s, 1 H), 8.37 (s, 1 H), 7.92 (s, 1 H), 7.69 (s, 1 H), 7.66 (s, 1 H), 4.77 (s, 2 H), 4.64 - 4.63 (m, 1 H), 4.05 (s, 3 H), 3.65 - 3.60 (m, 2 H), 3.43 - 3.38 (m, 2 H), 1.19 (t, *J* = 8.0 Hz, 3 H), 0.74 (s, 9 H). MS measured value (ESI⁺) [(M+H)⁺]: 469.

### Example 15:

### 6-tert-butyl-10-methoxy-9-[2-(1-methoxyethyl)thiazol-5-yl]-2-oxo-6,7-dihydro-2H-pyrido[2,1-a]isoquinoline-3-carboxylic acid

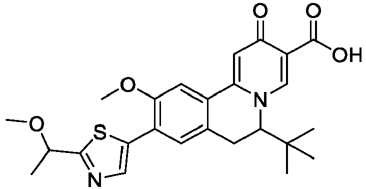

### Step 15a: Preparation of 1-(thiazol-2-yl)ethan-1-ol

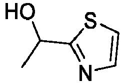

1-(Thiazol-2-yl)ethan-1-one (1.0 g, 7.86 mmol) was used to prepare and afford 1-(thiazol-2-yl)ethan-1-ol (1.0 g) as colorless oily liquid in accordance with a method similar to that in Step 10a of Example 10.

### Step 15b: Preparation of 1-(5-bromothiazol-2-yl)ethan-1-ol

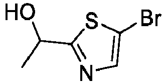

1-(Thiazol-2-yl)ethan-1-ol (1.0 g, 7.74 mmol) was used to prepare and afford 1-(5-bromothiazol-2-yl)ethan-1-ol (1.0 g) as white solid in accordance with a method similar to that in Step 10c of Example 10.

### Step 15c: Preparation of 5-bromo-2-(1-methoxyethyl)thiazole

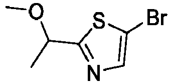

1-(5-Bromothiazol-2-yl)ethan-1-ol (500 mg, 2.4 mmol) was used to prepare and afford 5-bromo-2-(1-methoxyethyl)thiazole (100 mg) as white solid in accordance with a method similar to that in Step 10b of Example 10.

### Step 15d: Preparation of ethyl 6-tert-butyl-10-methoxy-9-[2-(1-methoxyethyl)thiazol- 5-yl]-2-oxo-6,7-dihydro-2H-pyrido[2,1-a]isoquinoline-3-carboxylate

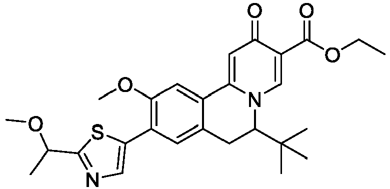

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (150 mg, 0.32 mmol) and 5-bromo-2-(1-methoxyethyl)thiazole (83 mg, 0.37 mmol) were used to prepare and afford ethyl 6-tert-butyl-10-methoxy-9-[2-(1-methoxyethyl)thiazol-5-yl]-2-oxo-6,7-dihydro-2*H*- pyrido[2,1-*a*]isoquinoline-3-carboxylate (100 mg) as yellow solid in accordance with a method similar to that in Step 1c of Example 1.

### Step 15e: Preparation of 6-tert-butyl-10-methoxy-9-[2-(1-methoxyethyl)thiazol-5-yl] -2-oxo-6,7-dihydro-2H-pyrido[2,1-a]isoquinoline-3-carboxylic acid

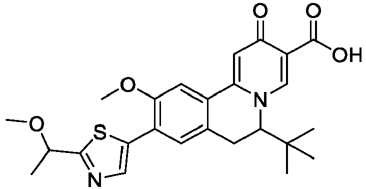

Ethyl 6-tert-butyl-10-methoxy-9-[2-(1-methoxyethyl)thiazol-5-yl]-2-oxo-6,7-dihydro-2*H*-pyrido[2,1-*a*]isoquinoline-3-carboxylate (100 mg, 0.20 mmol) was used to prepare and afford 6-tert-butyl-10-methoxy-9-[2-(1-methoxyethyl)thiazol-5-yl]-2-oxo-6,7-dihydro-2*H-*pyrido[2,1-*a*]isoquinoline-3-carboxylic acid (26.4 mg) as yellow solid in accordance with a method similar to that in Step Id of Example 1. ¹HNMR (DMSO-*d₆*, 400 MHz): δ 16.51 (s, 1 H), 8.78 (s, 1 H), 8.35 (s, 1 H), 7.92 (s, 1 H), 7.70 (s, 1 H), 7.67 (s, 1 H), 4.72 - 4.63 (m, 2 H), 4.05 (s, 3 H), 3.41 - 3.37 (m, 2 H), 3.36 (s, 3 H), 1.50 (d, *J* = 5.6 Hz, 3 H), 0.74 (s, 9 H). MS measured value (ESI⁺) [(M+H)⁺]: 469.

### Example 16:

### 6-tert-butyl-9-[2-(1-hydroxyethyl)thiazol-5-yl]-10-methoxy-2-oxo-6,7-dihydro-2H-pyrido[2,1-a]isoquinoline-3-carboxylic acid

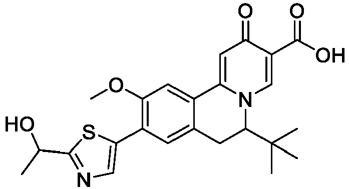

### Step 16a: Preparation of ethyl 6-tert-butyl-9-[2-(1-hydroxyethyl)thiazol-5-yl]-10-methoxy-2-oxo-6,7-dihydro-2H-pyrido[2,1-a]isoquinoline-3-carboxylate

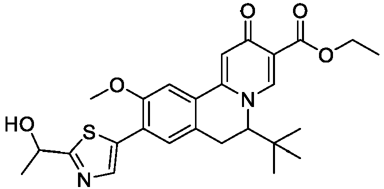

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (150 mg, 0.32 mmol) and 1-(5-bromothiazol-2-yl)ethan-1-ol (78 mg, 0.37 mmol) were used to prepare and afford ethyl 6-tert-butyl-9-[2-(1-hydroxyethyl)thiazol-5-yl]-10-methoxy-2-oxo-6,7-dihydro-2*H*-pyrido[2,1-*a*]isoquinoline-3-carboxylate (80 mg) as yellow solid in accordance with a method similar to that in Step 1c of Example 1.

### Step 16b: Preparation of 6-tert-butyl-9-[2-(1-hydroxyethyl)thiazol-5-yl]-10-methoxy- 2-oxo-6,7-dihydro-2H-pyrido[2,1-a]isoquinoline-3-carboxylic acid

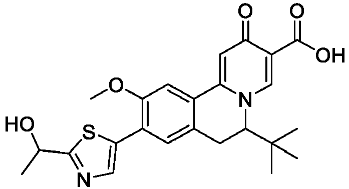

Ethyl 6-tert-butyl-9-[2-(1-hydroxyethyl)thiazol-5-yl]-10-methoxy-2-oxo-6,7-dihydro-2*H-*pyrido[2,1-*a*]isoquinoline-3-carboxylate (80 mg, 0.16 mmol) were used to prepare and afford 6-tert-butyl-9-[2-(1-hydroxyethyl)thiazol-5-yl]-10-methoxy-2-oxo-6,7-dihydro-2*H*-pyrido[2,1-*a*]isoquinoline-3-carboxylic acid (20.8 mg) as yellow solid in accordance with a method similar to that in Step Id of Example 1. ¹HNMR (DMSO-*d₆*, 400 MHz): δ 16.52 (s, 1 H), 8.78 (s, 1 H), 8.29 (s, 1 H), 7.88 (s, 1 H), 7.69 (s, 1 H), 7.66 (s, 1 H), 6.14 (d, *J* = 5.2 Hz, 1 H), 4.98 - 4.94 (m, 1 H), 4.63 (d, *J* = 5.2 Hz, 1 H), 4.04 (s, 3 H), 3.40 - 3.35 (m, 2 H), 1.47 (d, *J* = 7.0 Hz, 3 H), 0.74 (s, 9 H). MS measured value (ESI⁺) [(M+H)⁺]: 455.

### Example 17:

### 6-tert-butyl-10-methoxy-9-[2-(2-methoxypropan-2-yl)thiazol-5-yl]-2-oxo-6,7-dihydro-2H-pyrido[2,1-a]isoquinoline-3-carboxylic acid

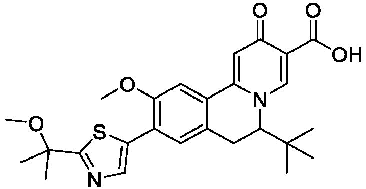

### Step 17a: Preparation of 2-(thiazol-2-yl)propan-2-ol

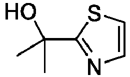

Under nitrogen atmosphere, to a solution of 1-(thiazol-2-yl)ethan-1-one (2.0 g, 15.73 mmol) in tetrahydrofuran (20 mL) was added dropwise methylmagnesium bromide (8.0 mL, 24.00 mmol) at -78°C. The mixture was stirred at -78°C for 2 hours. The mixture was quenched with saturated aqueous ammonium chloride solution (10 mL), and ethyl acetate (50 mL) was added for dilution. Afterwards, the resultant was washed with water (50 mL) and saturated brine (50 mL), and then dried over anhydrous sodium sulfate, filtered and concentrated under reduced pressure to afford a crude product. The crude product was purified by column chromatography to afford 2-(thiazol-2-yl)propan-2-ol (1.6 g) as white solid.

### Step 17b: Preparation of 2-(5-bromothiazol-2-yl)propan-2-ol

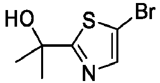

2-(Thiazol-2-yl)propan-2-ol (1.6 g, 11.17 mmol) was used to prepare and afford 2-(5-bromothiazol-2-yl)propan-2-ol (1.6 g) as white solid in accordance with a method similar to that in Step 10c of Example 10.

### Step 17c: Preparation of 5-bromo-2-(2-methoxypropan-2-yl)thiazole

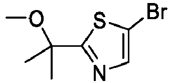

2-(5-Bromothiazol-2-yl)propan-2-ol (500 mg, 2.25 mmol) was used to prepare and afford 5-bromo-2-(2-methoxypropan-2-yl)thiazole (300 mg) as white solid in accordance with a method similar to that in Step 10b of Example 10.

### Step 17d: Preparation of ethyl 6-tert-butyl-10-methoxy-9-[2-(2-methoxypropan-2-yl)thiazol-5-yl]-2-oxo-6,7-dihydro-2H-pyrido[2,1-a]isoquinoline-3-carboxylate

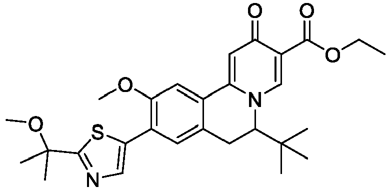

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (150 mg, 0.32 mmol) and 5-bromo-2-(2-methoxypropan-2-yl)thiazole (88 mg, 0.37 mmol) were used to prepare and afford ethyl 6-tert-butyl-10-methoxy-9-[2-(2-methoxypropan-2-yl)thiazol-5-yl]-2-oxo- 6,7-dihydro-2H-pyrido[2,1-*a*]isoquinoline-3-carboxylate (120 mg) as yellow solid in accordance with a method similar to that in Step 1c of Example 1.

### Step 17e: Preparation of 6-tert-butyl-10-methoxy-9-[2-(2-methoxypropan-2-yl)thiazol- 5-yl]-2-oxo-6,7-dihydro-2H-pyrido[2,1-a]isoquinoline-3-carboxylic acid

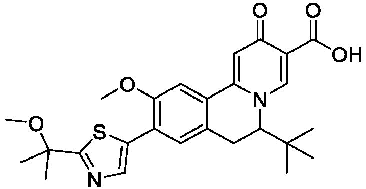

Ethyl 6-tert-butyl-10-methoxy-9-[2-(2-methoxypropan-2-yl)thiazol-5-yl]-2-oxo-6,7-dihydro-2*H*-pyrido[2,1-*a*]isoquinoline-3-carboxylate (120 mg, 0.23 mmol) was used to prepare and afford 6-tert-butyl-10-methoxy-9-[2-(2-methoxypropan-2-yl)thiazol-5-yl]-2-oxo-6,7-dihydro-2*H*-pyrido[2,1-*a*]isoquinoline-3-carboxylic acid (26.4 mg) as yellow solid in accordance with a method similar to that in Step Id of Example 1. ¹HNMR (DMSO-*d₆*, 400 MHz): δ 16.51 (s, 1 H), 8.78 (s, 1 H), 8.31 (s, 1 H), 7.90 (s, 1 H), 7.69 (s, 1 H), 7.67 (s, 1 H), 4.64 (s, 1 H), 4.04 (s, 3 H), 3.40 - 3.35 (m, 2 H), 3.20 (s, 3 H), 1.57 (s, 6 H), 0.74 (s, 9 H). MS measured value (ESI⁺) [(M+H)⁺]: 483.

### Example 18:

### 6-tert-butyl-10-methoxy-2-oxo-9-(2-propylthiazol-5-yl)-6,7-dihydro-2H-pyrido[2,1-a]isoquinoline-3-carboxylic acid

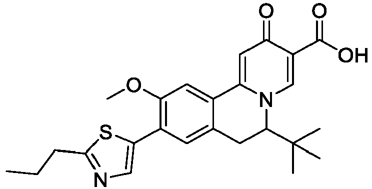

### Step 18a: Preparation of 5-bromo-2-propylthiazole

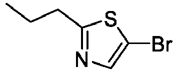

2-Propylthiazole (264 mg, 2 mmol) was used to prepare and afford 5-bromo-2-propylthiazole (320 mg) in accordance with a method similar to that in Step 10c of Example 10.

### Step 18b: Preparation of ethyl 6-tert-butyl-10-methoxy-2-oxo-9-(2-propylthiazol-5-yl)-6,7-dihydro-2H-pyrido[2,1-a]isoquinoline-3-carboxylate

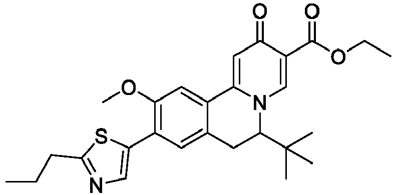

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (193 mg, 0.4 mmol) and 5-bromo-2-propylthiazole (107 mg, 0.52 mmol) were used to prepare and afford ethyl 6-tert-butyl-10-methoxy-2-oxo-9-(2-propylthiazol-5-yl)-6,7-dihydro-2*H*-pyrido[2,1-*a*]isoquinoline-3-carboxylate (73 mg) in accordance with a method similar to that in Step 1c of Example 1.

### Step 18c: Preparation of 6-tert-butyl-10-methoxy-2-oxo-9-(2-propylthiazol-5-yl)-6,7-dihydro-2H-pyrido[2,1-a]isoquinoline-3-carboxylic acid

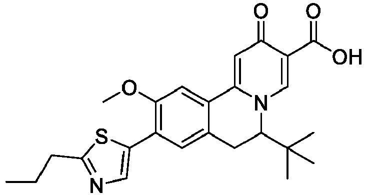

Ethyl 6-tert-butyl-10-methoxy-2-oxo-9-(2-propylthiazol-5-yl)-6,7-dihydro-2*H-*pyrido[2,1-*a*]isoquinoline-3-carboxylate (73 mg, 0.152 mmol) were used to prepare and afford 6-tert-butyl-10-methoxy-2-oxo-9-(2-propylthiazol-5-yl)-6,7-dihydro-2*H*- pyrido[2,1-*a*]isoquinoline-3-carboxylic acid (53.5 mg) as yellow solid in accordance with a method similar to that in Step Id of Example 1. ¹H NMR (DMSO-*d₆*, 400 MHz): δ 16.48 (s, 1 H), 8.76 (s, 1 H), 8.28 (s, 1 H), 7.88 (s, 1 H), 7.67 (s, 1 H), 7.64 (s, 1 H), 4.62 (s, 1 H), 4.03 (s, 3 H), 3.63 - 3.51 (m, 2 H), 2.96 (t, *J* = 7.2 Hz, 2 H), 1.79 - 1.74 (m, 2 H), 0.96 (t, *J* = 7.2 Hz, 3 H), 0.74 (s, 9 H). MS measured value (ESI⁺) [(M+H)⁺]: 453.

### Example 19:

### 6-tert-butyl-10-methoxy-9-[2-(methylcarbamoyl)thiazol-5-yl]-2-oxo-6,7-dihydro-2H-pyrido[2,1-a]isoquinoline-3-carboxylic acid

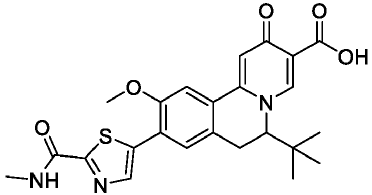

### Step 19a: Preparation of 5-bromo-N-methylthiazol-2-carboxamide

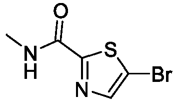

To a solution of 5-bromothiazol-2-carboxylic acid (200 mg, 0.96 mmol) in dichloromethane (5 mL) were added one drop of *N*,*N-*dimethylformamide and oxalyl chloride (246 µL, 2.88 mmol). After the mixture was stirred at room temperature for one hour, the resultant was concentrated under reduced pressure, the residue was dissolved in a solution of tetrahydrofuran (5 mL), and then a solution of methylamine in tetrahydrofuran (2 M, 960 µL) was slowly added dropwise under ice bath condition. The mixture was stirred at room temperature for 6 h. After the completion of the reaction, the resultant was concentrated under reduced pressure, and a solution of dichloromethane (10 mL) was added thereto. The resulting mixture was respectively washed twice with water and saturated sodium chloride solution. The organic layer was dried over anhydrous sodium sulfate and concentrated under reduced pressure to afford a crude product. The crude product was purified by flash column chromatography to afford 5-bromo-*N*-methylthiazol-2-carboxamide (180 mg) as white solid.

### Step 19b: Preparation of ethyl 6-tert-butyl-10-methoxy-9-[2-(methylcarbamoyl)thiazol- 5-yl]-2-oxo-6,7-dihydro-2H-pyrido[2,1-a]isoquinoline-3-carboxylate

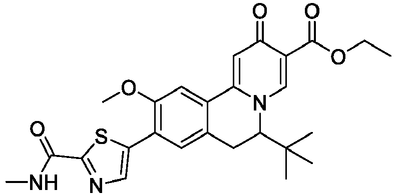

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (193 mg, 0.4 mmol) and 5-bromo-*N-*methylthiazol-2-carboxamide (115 mg, 0.52 mmol) were used to prepare and afford ethyl 6-tert-butyl-10-methoxy-9-[2-(methylcarbamoyl)thiazol-5-yl]-2-oxo-6,7- dihydro-2*H*-pyrido[2,1-*a*]isoquinoline-3-carboxylate (42 mg) in accordance with a method similar to that in Step 1c of Example 1.

### Step 19c: Preparation of 6-tert-butyl-10-methoxy-9-[2-(methylcarbamoyl)thiazol-5-yl]- 2-oxo-6,7-dihydro-2H-pyrido[2,1-a]isoquinoline-3-carboxylic acid

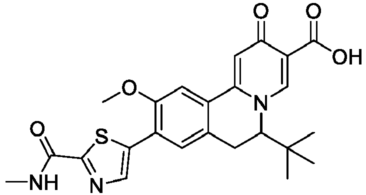

Ethyl 6-tert-butyl-10-methoxy-9-[2-(methylcarbamoyl)thiazol-5-yl]-2-oxo-6,7-dihydro-2*H*-pyrido[2,1-*a*]isoquinoline-3-carboxylate (42 mg, 0.085 mmol) were used to prepare and afford 6-tert-butyl-10-methoxy-9-[2-(methylcarbamoyl)thiazol-5-yl]-2-oxo-6,7-dihydro-2*H-*pyrido[2,1-*a*]isoquinoline-3-carboxylic acid (33 mg) as yellow solid in accordance with a method similar to that in Step Id of Example 1. ¹H NMR (DMSO-*d₆*, 400 MHz): δ 8.81 (s, 1 H), 8.59 (s, 1 H), 8.51 (s, 1 H), 8.04 (s, 1 H), 7.61 (s, 1 H), 7.18 (s, 1 H), 4.38 (s, 1 H), 4.08 (s, 3 H), 3.33 - 3.32 (m, 2 H), 2.81 (d, *J* = 4.4Hz, 3 H), 0.74 (s, 9H). MS measured value (ESI⁺) [(M+H)⁺]: 468.

### Example 20:

### 6-tert-butyl-9-[2-(ethylcarbamoyl)thiazol-5-yl]-10-methoxy-2-oxo-6,7-dihydro-2H-pyrido[2,1-a]isoquinoline-3-carboxylic acid

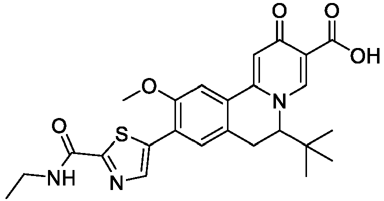

### Step 20a: Preparation of 5-bromo-N-ethylthiazol-2-carboxamide

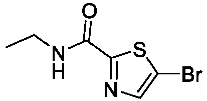

5-Bromothiazol-2-carboxylic acid (200 mg, 0.96 mmol) and a solution of ethylamine in tetrahydrofuran (2 M, 960 µL) were used to prepare and afford 5-bromo-*N*-ethylthiazol-2-carboxamide (210 mg) in accordance with a method similar to that in Step 19a of Example 19.

### Step 20b: Preparation of ethyl 6-tert-butyl-9-[2-(ethylcarbamoyl)thiazol-5-yl]-10-methoxy-2-oxo-6,7-dihydro-2H-pyrido[2,1-a]isoquinoline-3-carboxylate

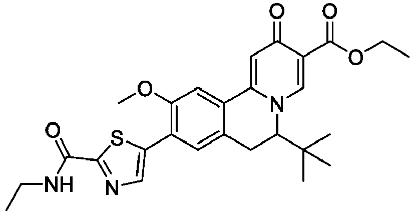

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (193 mg, 0.4 mmol) and 5-bromo-*N-*ethylthiazol-2-carboxamide (122 mg, 0.52 mmol) were used to prepare and afford ethyl 6-tert-butyl-9-[2-(ethylcarbamoyl)thiazol-5-yl]-10-methoxy-2-oxo-6,7-dihydro-2*H*- pyrido[2,1-*a*]isoquinoline-3-carboxylate (15 mg) in accordance with a method similar to that in Step 1c of Example 1.

### Step 20c: Preparation of 6-tert-butyl-9-[2-(ethylcarbamoyl)thiazol-5-yl]-10-methoxy-2-oxo-6,7-dihydro-2H-pyrido[2,1-a]isoquinoline-3-carboxylic acid

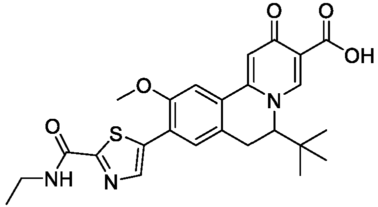

Ethyl 6-tert-butyl-9-[2-(ethylcarbamoyl)thiazol-5-yl]-10-methoxy-2-oxo-6,7-dihydro-2*H*-pyrido[2,1-*a*]isoquinoline-3-carboxylate (15 mg, 0.029 mmol) was used to prepare and afford 6-tert-butyl-9-[2-(ethylcarbamoyl)thiazol-5-yl]-10-methoxy-2-oxo-6,7-dihydro-2*H-*pyrido[2,1-*a*]isoquinoline-3-carboxylic acid (8.6 mg) as yellow solid in accordance with a method similar to that in Step Id of Example 1. ¹H NMR (DMSO-*d₆*, 400 MHz): δ 16.42 (s, 1 H), 8.89 (t, *J* = 5.2 Hz, 1 H), 8.78 (s, 1 H), 8.61 (s, 1 H), 8.09 (s, 1 H), 7.73 (s, 1 H), 7.69 (s, 1 H), 4.64 (d, *J* = 2.4 Hz, 1 H), 4.09 (s, 3 H), 3.46 - 3.43 (m, 2 H), 3.30 (d, *J* = 6.8 Hz, 2 H), 1.13 (t, *J* = 7.2 Hz, 3 H), 0.75 (s, 9 H). MS measured value (ESI⁺) [(M+H)⁺]: 482.

### Example 21:

### 6-tert-butyl-10-methoxy-9-[2-(2-methoxyethoxy)thiazol-5-yl]-2-oxo-6,7-dihydro-2H-pyrido[2,1-a]isoquinoline-3-carboxylic acid

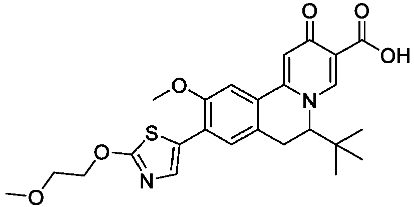

### Step 21a: Preparation of 5-bromo-2-(2-methoxyethoxy)thiazole

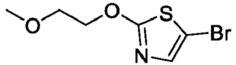

2,5-Dibromothiazole (200 mg, 0.82 mmol) and ethylene glycol monomethyl ether (6 mL) were used to prepare and afford 5-bromo-2-(2-methoxyethoxy)thiazole (90 mg) as white solid in accordance with a method similar to that in Step 12a of Example 12.

### Step 21b: Preparation of ethyl 6-tert-butyl-10-methoxy-9-[2-(2-methoxyethoxy)thiazol-5-yl]-2-oxo-6,7-dihydro-2H-pyrido[2,1-a]isoquinoline-3-carboxylate

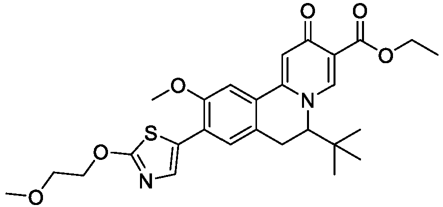

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (146 mg, 0.30 mmol) and 5-bromo-2-(methoxyethoxy)thiazole (90 mg, 0.38 mmol) were used to prepare and afford ethyl 6-tert-butyl-10-methoxy-9-[2-(2-methoxyethoxy)thiazol-5-yl]-2-oxo-6,7-dihydro-2*H*-pyrido[2,1-*a*]isoquinoline-3-carboxylate (140 mg) as yellow solid in accordance with a method similar to that in Step 1c of Example 1.

### Step 21c: 6-tert-butyl-10-methoxy-9-[2-(2-methoxyethoxy)thiazol-5-yl]-2-oxo-6,7-dihydro-2H-pyrido[2,1-a]isoquinoline-3-carboxylic acid

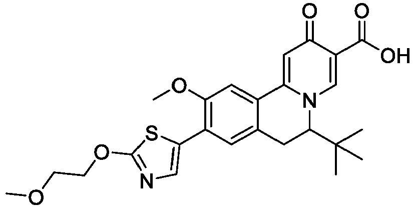

Ethyl 6-tert-butyl-10-methoxy-9-[2-(2-methoxyethoxy)thiazol-5-yl]-2-oxo-6,7-dihydro-2*H*-pyrido[2,1-*a*]isoquinoline-3-carboxylate (140 mg, 0.27 mmol) was used to prepare and afford 6-tert-butyl-10-methoxy-9-[2-(2-methoxyethoxy)thiazol-5-yl]-2-oxo-6,7-dihydro-2*H-*pyrido[2,1-*a*]isoquinoline-3-carboxylic acid (47 mg) as yellow solid in accordance with a method similar to that in Step Id of Example 1. ¹HNMR (DMSO-*d₆*, 400 MHz): δ 8.75 (s, 1 H), 7.85 (s, 1 H), 7.80 (s, 1 H), 7.65 (s, 1 H),7.60 (s, 1 H), 4.62 (d, *J* = 4.4 Hz, 1 H), 4.53 (t, *J* = 4.0 Hz, 2 H), 4.00 (s, 3 H), 3.72 - 3.67 (m, 2 H), 3.45 - 3.35 (m, 2 H), 3.33 (s, 3 H), 0.74 (s, 9 H). MS measured value (ESI⁺) [(M+H)⁺]: 485.

### Example 22:

### 6-tert-butyl-10-methoxy-9-{2-[(2-methoxyethyl)carbamoyl]thiazol-5-yl}-2-oxo-6,7-dihydro-2H-pyrido[2,1-a]isoquinoline-3-carboxylic acid

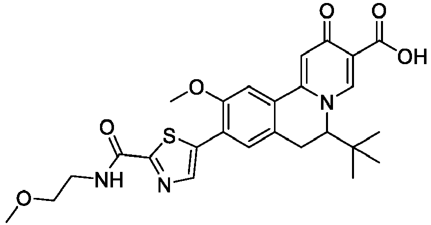

### Step 22a: Preparation of 5-bromo-N-(2-methoxyethyl)thiazole-2-carboxamide

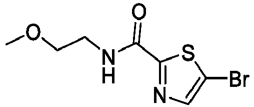

5-Bromothiazol-2-carboxylic acid (200 mg, 0.96 mmol) and 2-methoxyethylamine (144 mg, 1.92 mmol) were used to prepare and afford 5-bromo-*N*-(2-methoxyethyl)thiazole-2-carboxamide (192 mg) in accordance with a method similar to that in Step 19a of Example 19.

### Step 22b: Preparation of ethyl 6-tert-butyl-10-methoxy-9-{2-[(2-methoxyethyl) carbamoyl]thiazol-5-yl}-2-oxo-6,7-dihydro-2H-pyrido[2,1-a]isoquinoline-3-carboxylate

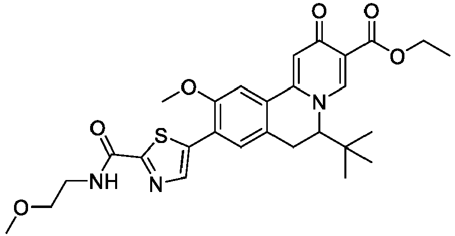

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (193 mg, 0.4 mmol) and 5-bromo-*N-*(2-methoxyethyl)thiazole-2-carboxamide (138 mg, 0.52 mmol) were used to prepare and afford ethyl 6-tert-butyl-10-methoxy-9-{2-[(2-methoxyethyl)carbamoyl]thiazol- 5-yl}-2-oxo-6,7-dihydro-2*H*-pyrido[2,1-*a*]isoquinoline-3-carboxylate (77 mg) in accordance with a method similar to that in Step 1c of Example 1.

### Step 22c: Preparation of 6-tert-butyl-10-methoxy-9-{2-[(2-methoxyethyl) carbamoyl]thiazol-5-yl}-2-oxo-6,7-dihydro-2H-pyrido[2,1-a]isoquinoline-3-carboxylic acid

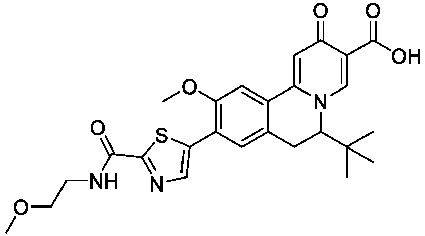

Ethyl 6-tert-butyl-10-methoxy-9-{2-[(2-methoxyethyl)carbamoyl]thiazol-5-yl}-2-oxo-6,7-dihydro-2*H*-pyrido[2,1-*a*]isoquinoline-3-carboxylate (77 mg, 0.143 mmol) was used to prepare and afford 6-tert-butyl-10-methoxy-9-{2-[(2-methoxyethyl) carbamoyl]thiazol-5-yl}-2-oxo-6,7-dihydro-2*H*-pyrido[2,1-*a*]isoquinoline-3-carboxylic acid (57.5 mg) as yellow solid in accordance with a method similar to that in Step Id of Example 1. ¹H NMR (DMSO-*d₆*, 400 MHz): δ 16.48 (s, 1 H), 8.80 (s, 1 H), 8.77 (s, 1 H), 8.62 (s, 1 H), 8.10 (s, 1 H), 7.75 (s, 1 H), 7.70 (s, 1 H), 4.65 (d, *J* = 5.6 Hz, 1 H), 4.10 (s, 3 H), 3.48 - 3.44 (m, 4 H), 3.43 - 3.33 (m, 2 H), 3.27 (s, 3 H), 0.75 (s, 9 H).MS measured value (ESI⁺) [(M+H)⁺]: 512.

### Example 23:

### 6-tert-butyl-9-{2-[(difluoromethoxy)methyl]thiazol-5-yl}-10-methoxy-2-oxo-6,7-dihydro-2H-pyrido[2,1-a]isoquinoline-3-carboxylic acid

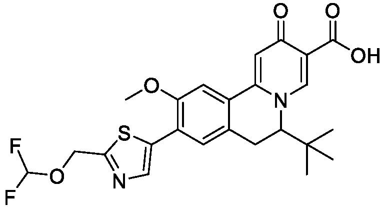

### Step 23a: Preparation of 2-[(difluoromethoxy)methyl]thiazole

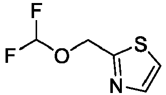

Under nitrogen atmosphere, to a solution of thiazol-2-ylmethanol (2.0 g, 17.37 mmol) and cuprous iodide (662 mg, 3.47 mmol) in acetonitrile (20 mL) was added dropwise 2,2-difluoro-2-(fluorosulfonyl)acetic acid (3.1 g, 17.37 mmol) at 50°C. The mixture was stirred at 50°C for 8 hours. Ethyl acetate (50 mL) was added to the mixture for dilution. Afterwards, the resultant was washed with water (50 mL) and saturated brine (50 mL), and then dried over anhydrous sodium sulfate, filtered and concentrated under reduced pressure to afford a crude product. The crude product was purified by column chromatography to afford 2-[(difluoromethoxy)methyl]thiazole (310 mg) as white solid.

### Step 23b: Preparation of 5-bromo-2-[(difluoromethoxy)methyl]thiazole

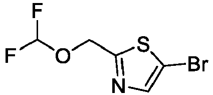

2-[(Difluoromethoxy)methyl]thiazole (310 mg, 1.88 mmol) was used to prepare and afford 5-bromo-2-[(difluoromethoxy)methyl]thiazole (211 mg) as white solid in accordance with a method similar to that in Step 10c of Example 10.

### Step 23c: Preparation of ethyl 6-tert-butyl-9-{2-[(difluoromethoxy)methyl]thiazol- 5-yl}-10-methoxy-2-oxo-6,7-dihydro-2H-pyrido[2,1-a]isoquinoline-3-carboxylate

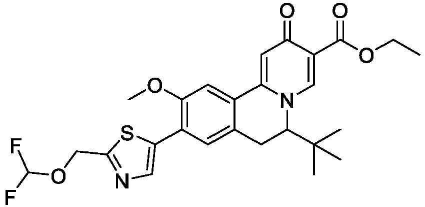

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (150 mg, 0.32 mmol) and 5-bromo-2-[(difluoromethoxy)methyl]thiazole (91 mg, 0.37 mmol) were used to prepare and afford ethyl 6-tert-butyl-9-{2-[(difluoromethoxy)methyl]thiazol-5-yl}-10-methoxy-2-oxo- 6,7-dihydro-2*H-*pyrido[2,1-*a*]isoquinoline-3-carboxylate (120 mg) as yellow solid in accordance with a method similar to that in Step 1c of Example 1.

### Step 23d: Preparation of 6-tert-butyl-9-{2-[(difluoromethoxy)methyl]thiazol-5-yl} -10-methoxy-2-oxo-6,7-dihydro-2H-pyrido[2,1-a]isoquinoline-3-carboxylic acid

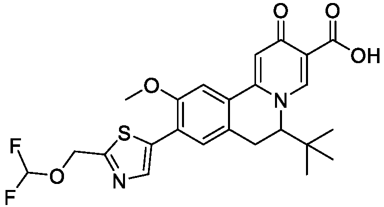

Ethyl 6-tert-butyl-9-{2-[(difluoromethoxy)methyl]thiazol-5-yl}-10-methoxy-2-oxo-6,7-dihydro-2*H*-pyrido[2,1-*a*]isoquinoline-3-carboxylate (120 mg, 0.23 mmol) was used to prepare and afford 6-tert-butyl-9-{2-[(difluoromethoxy)methyl]thiazol-5-yl}-10-methoxy -2-oxo-6,7-dihydro-2*H*-pyrido[2,1-*a*]isoquinoline-3-carboxylic acid (35.2 mg) as yellow solid in accordance with a method similar to that in Step Id of Example 1. ¹HNMR (DMSO-*d₆*, 400 MHz): δ 8.79 (s, 1 H), 8.45 (s, 1 H), 7.99 (s, 1 H), 7.72 (s, 1 H), 7.69 (s, 1 H), 6.91 (t, J = 74.0 Hz, 1 H), 5.26 (s, 2 H), 4.64 (s, 1 H), 4.06 (s, 3 H), 3.42 - 3.36 (m, 2 H), 0.74 (s, 9 H). MS measured value (ESI⁺) [(M+H)⁺]: 491.

### Example 24:

### 6-tert-butyl-9-[2-(cyclopropoxymethyl)thiazol-5-yl]-10-methoxy-2-oxo-6,7-dihydro-2H-pyrido[2,1-a]isoquinoline-3-carboxylic acid

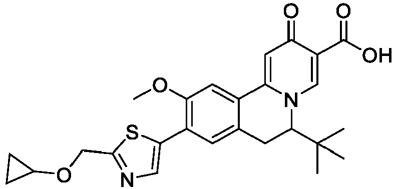

### Step 24a: Preparation of 5-bromo-2-(bromomethyl)thiazole

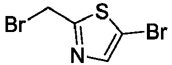

5-Bromo-2-methylthiazole (1 g, 5.62 mmol) and benzoyl peroxide (14 mg, 0.11 mmol) were added to a solution of *N*-bromosuccinimide (1.2 g, 6.74 mmol) in tetrachloromethane (10 mL) at room temperature, and then the mixture was heated to 80°C and stirred at 80°C for 6 hours. The resultant was diluted with water and extracted with dichloromethane (50 mL × 3). The organic phases were collected, washed with saturated brine and dried over anhydrous sodium sulfate. A red oily crude product was afforded after drying via rotary evaporation. The crude product was purified by flash column chromatography to afford 5-bromo-2-(bromomethyl)thiazole (1 g) as yellow liquid.

### Step 24b: Preparation of 5-bromo-2-(cyclopropoxymethyl)thiazole

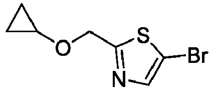

At 0°C and under nitrogen atmosphere, a solution of cyclopropanol (0.03 mg, 0.43 mmol) in tetrahydrofuran (5 mL) was added to sodium hydride (43 mg,1.17 mmol), and the resulting mixture was stirred for 30 minutes. Then, 5-bromo-2-(bromomethyl)thiazole (100 mg, 0.39 mmol) was added to the solution, and the mixture was heated to room temperature and stirred at room temperature for 4 hours. The reaction was quenched with water, and the resultant was extracted with ethyl acetate (30 mL × 3). The organic phases were collected, washed with saturated brine and dried over anhydrous sodium sulfate. A yellowish oily crude product was afforded after drying via rotary evaporation. The crude product was purified by flash column chromatography to afford 5-bromo-2-(cyclopropoxymethyl)thiazole (80 mg) as yellow solid.

### Step 24c: Preparation of ethyl 6-tert-butyl-9-[2-(cyclopropoxymethyl)thiazol-5-yl]-10-methoxy-2-oxo-6,7-dihydro-2H-pyrido[2,1-a]isoquinoline-3-carboxylate

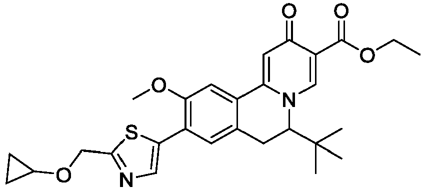

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (250 mg, 0.52 mmol) and 5-bromo-2-(cyclopropoxymethyl)thiazole (80 mg, 0.34 mmol) were used to prepare and afford ethyl 6-tert-butyl-9-[2-(cyclopropoxymethyl)thiazol-5-yl]-10-methoxy-2-oxo-6,7-dihydro- 2*H-*pyrido[2,1-*a*]isoquinoline-3-carboxylate (110 mg) as yellow solid in accordance with a method similar to that in Step 1c of Example 1.

### Step 24d: 6-tert-butyl-9-[2-(cyclopropoxymethyl)thiazol-5-yl]-10-methoxy-2-oxo-6,7-dihydro-2H-pyrido[2,1-a]isoquinoline-3-carboxylic acid

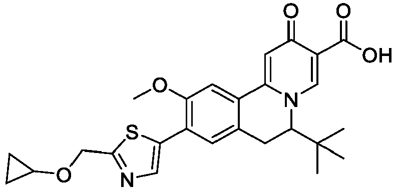

Ethyl 6-tert-butyl-9-[2-(cyclopropoxymethyl)thiazol-5-yl]-10-methoxy-2-oxo-6,7-dihydro-2*H*-pyrido[2,1-*a*]isoquinoline-3-carboxylate (110 mg, 0.21 mmol) was used to prepare and afford 6-tert-butyl-9-[2-(cyclopropoxymethyl)thiazol-5-yl]-10-methoxy-2-oxo-6,7-dihydro-2*H*-pyrido[2,1-*a*]isoquinoline-3-carboxylic acid (58.1 mg) as yellow solid in accordance with a method similar to that in Step Id of Example 1. ¹HNMR (CDCl₃, 400 MHz): δ 8.50 (s, 1 H), 8.21 (s, 1 H), 7.50 (s, 1 H), 7.27 (s, 1 H), 7.20 (s, 1 H), 4.88 (s, 2 H), 4.12 - 4.07 (m, 1 H), 4.03 (s, 3 H), 3.58 - 3.52 (m, 1 H), 3.35 - 3.26 (m, 2 H), 0.85 (s, 9 H), 0.76 - 0.72 (m, 2 H), 0.61 - 0.55 (m, 2 H). MS measured value (ESI⁺) [(M+H)⁺]: 481.

### Example 25:

### 6-tert-butyl-9-[2-(3,3-difluorocyclobutyl)thiazol-5-yl]-10-methoxy-2-oxo-6,7-dihydro-2H-pyrido[2,1-a]isoquinoline-3-carboxylic acid

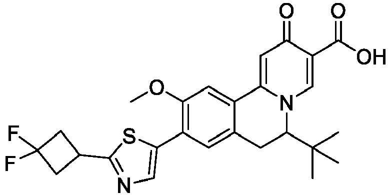

### Step 25a: Preparation of 3,3-difluorocyclobutane-1-thioformamide

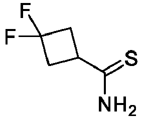

To a solution of 3,3-difluorocyclobutane-1-carboxamide (2.0 g, 14.80 mmol) in 1,2-dichloroethane (20 mL) was added Lawesson's reagent (3.0 g, 7.40 mmol). The mixture was stirred at 85°C for 2 hours. The mixture was concentrated under reduced pressure to afford a crude product. The crude product was purified by column chromatography to afford 3,3-difluorocyclobutane-1-thioformamide (2.0 g) as yellow solid.

### Step 25b: Preparation of 2-(3,3-difluorocyclobutyl)thiazole

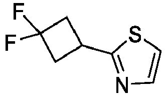

To a solution of 3,3-difluorocyclobutane-1-thioformamide (2.0 g, 12.23 mmol) in ethanol (10 mL) was added 40% aqueous solution of chloroacetaldehyde (7.8 mL, 39.69 mmol). The mixture was stirred at 100°C for four hours. The mixture was concentrated under reduced pressure to afford a crude product. The crude product was purified by column chromatography to afford 2-(3,3-difluorocyclobutyl)thiazole (340 mg) as white solid.

### Step 25c: Preparation of 5-bromo-2-(3,3-difluorocyclobutyl)thiazole

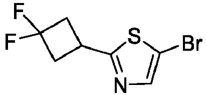

2-(3,3-Difluorocyclobutyl)thiazole (340 mg, 1.94 mmol) and *N*-bromosuccinimide (380 mg, 2.13 mmol) were used to prepare and afford 5-bromo-2-(3,3-difluorocyclobutyl)thiazole (300 mg) as white solid in accordance with a method similar to that in Step 10c of Example 10.

### Step 25d: Preparation of ethyl 6-tert-butyl-9-[2-(3,3-difluorocyclobutyl)thiazol-5-yl]-10-methoxy-2-oxo-6,7-dihydro-2H-pyrido[2,1-a]isoquinoline-3-carboxylate

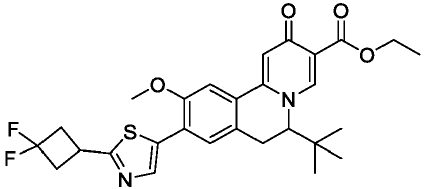

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (150 mg, 0.32 mmol) and 5-bromo-2-(3,3-difluorocyclobutyl)thiazole (95 mg, 0.37 mmol) were used to prepare and afford ethyl 6-tert-butyl-9-[2-(3,3-difluorocyclobutyl)thiazol-5-yl]-10-methoxy-2-oxo-6,7- dihydro-2H-pyrido[2,1-*a*]isoquinoline-3-carboxylate (100 mg) as brown solid in accordance with a method similar to that in Step 1c of Example 1.

### Step 25e: Preparation of 6-tert-butyl-9-[2-(3,3-difluorocyclobutyl)thiazol-5-yl]-10-methoxy-2-oxo-6,7-dihydro-2H-pyrido[2,1-a]isoquinoline-3-carboxylic acid

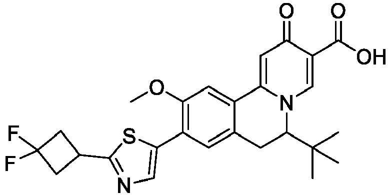

Ethyl 6-tert-butyl-9-[2-(3,3-difluorocyclobutyl)thiazol-5-yl]-10-methoxy-2-oxo-6,7-dihydro-2*H*-pyrido[2,1-*a*]isoquinoline-3-carboxylate (100 mg, 0.19 mmol) was used to prepare and afford 6-tert-butyl-9-[2-(3,3-difluorocyclobutyl)thiazol-5-yl]-10-methoxy- 2-oxo-6,7-dihydro-2*H*-pyrido[2,1-*a*]isoquinoline-3-carboxylic acid (2.1 mg) as yellow solid in accordance with a method similar to that in Step Id of Example 1. ¹HNMR (DMSO-*d₆*, 400 MHz) δ 8.79 (s, 1 H), 8.37 (s, 1 H), 7.93 (s, 1 H), 7.70 (s, 1 H), 7.67 (s, 1 H), 4.64 (d, *J* = 5.2 Hz, 1 H), 4.04 (s, 3 H), 3.88 - 3.84 (m, 1 H), 3.45 - 3.38 (m, 2 H). 3.16 - 3.09 (m, 2 H), 2.98 - 2.91 (m, 2 H), 0.74 (s, 9 H). MS measured value (ESI⁺) [(M+H)⁺]: 501.

### Example 26:

### 6-tert-butyl-9-[2-(3-fluorocyclobutyl)thiazol-5-yl]-10-methoxy-2-oxo-6,7-dihydro-2H-pyrido[2,1-a]isoquinoline-3-carboxylic acid

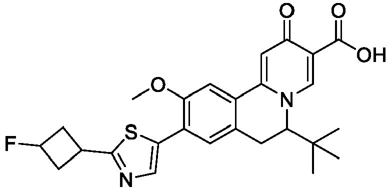

### Step 26a: Preparation of 5-bromo-2-(3-fluorocyclobutyl)thiazole

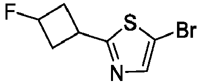

2-(3-Fluorocyclobutyl)thiazole (100 mg, 0.64 mmol) and *N*-bromosuccinimide (124 mg, 0.70 mmol) were used to prepare and afford 5-bromo-2-(3-fluorocyclobutyl)thiazole (100 mg) as white solid in accordance with a method similar to that in Step 10c of Example 10.

### Step 26b: Preparation of ethyl 6-tert-butyl-9-[2-(3-fluorocyclobutyl)thiazol-5-yl]-10-methoxy-2-oxo-6,7-dihydro-2H-pyrido[2,1-a]isoquinoline-3-carboxylate

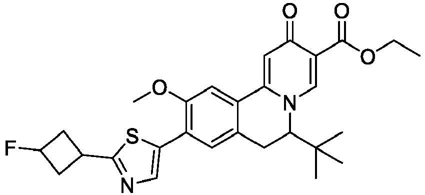

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (226 mg, 0.47 mmol) and 5-bromo-2-(3-fluorocyclobutyl)thiazole (100 mg, 0.42 mmol) were used to prepare and afford ethyl 6-tert-butyl-9-[2-(3-fluorocyclobutyl)thiazol-5-yl]-10-methoxy-2-oxo-6,7-dihydro-2*H*- pyrido[2,1-*a*]isoquinoline-3-carboxylate (65 mg) as yellow solid in accordance with a method similar to that in Step 1c of Example 1.

### Step 26c: Preparation of 6-tert-butyl-9-[2-(3-fluorocyclobutyl)thiazol-5-yl]-10- methoxy-2-oxo-6,7-dihydro-2H-pyrido[2,1-a]isoquinoline-3-carboxylic acid

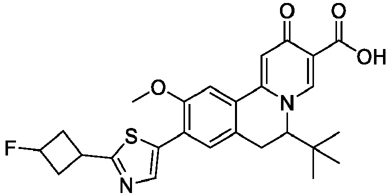

Ethyl 6-tert-butyl-9-[2-(3-fluorocyclobutyl)thiazol-5-yl]-10-methoxy-2-oxo-6,7-dihydro-2*H*-pyrido[2,1-*a*]isoquinoline-3-carboxylate (65 mg, 0.13 mmol) was used to prepare and afford 6-tert-butyl-9-[2-(3-fluorocyclobutyl)thiazol-5-yl]-10-methoxy-2-oxo-6,7-dihydro-2*H-*pyrido[2,1-*a*]isoquinoline-3-carboxylic acid (22.3 mg) as yellow solid in accordance with a method similar to that in Step Id of Example 1. ¹HNMR (DMSO-*d₆*, 400 MHz) δ 16.57 (s, 1 H), 8.79 (s, 1 H), 8.37 (s, 1 H), 7.65 (s, 1 H), 7.61 (s, 1 H), 7.59 (s, 1 H), 5.26 (d, *J=* 56.0 Hz, 1 H), 4.64 (d, *J=* 5.2 Hz, 1 H), 4.04 (s, 3 H), 3.88 - 3.84 (m, 1 H), 3.43 - 3.35 (m, 2 H), 2.82 - 2.76 (m, 2 H). 2.55 - 2.48 (m, 2 H), 0.74 (s, 9 H). MS measured value (ESI⁺) [(M+H)⁺]: 483.

### Example 27:

### 6-tert-butyl-10-methoxy-9-[2-(3-methoxycyclobutyl)thiazol-5-yl]-2-oxo-6,7-dihydro-2H-pyrido[2,1-a]isoquinoline-3-carboxylic acid

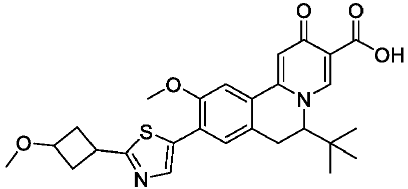

### Step 27a: Preparation of 5-bromo-2-(3-methoxycyclobutyl)thiazole

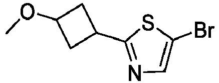

2-(3-Methoxycyclobutyl)thiazole (70 mg, 0.41 mmol) and *N*-bromosuccinimide (80 mg, 0.45 mmol) were used to prepare and afford 5-bromo-2-(3-methoxycyclobutyl)thiazole (32 mg) as white solid in accordance with a method similar to that in Step 10c of Example 10.

### Step 27b: Preparation of ethyl 6-tert-butyl-10-methoxy-9-[2-(3-methoxycyclobutyl) thiazol-5-yl]-2-oxo-6,7-dihydro-2H-pyrido[2,1-a]isoquinoline-3-carboxylate

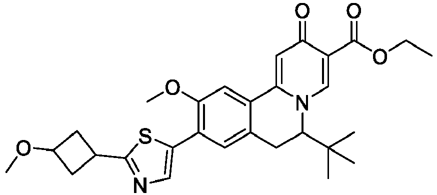

Ethyl 6-tert-butyl-10-methoxy-2-oxo-9-(4,4,5,5-tetramethyl-1,3,2-dioxaborolan-2-yl)-6,7-dihydro-2*H*-pyrido[2,1-*a*]isoquinoline-3-carboxylate (67 mg, 0.14 mmol) and 5-bromo-2-(3-methoxycyclobutyl)thiazole (32 mg, 0.13 mmol) were used to prepare and afford ethyl 6-tert-butyl-10-methoxy-9-[2-(3-methoxycyclobutyl)thiazol-5-yl]-2-oxo-6,7-dihydro- 2*H*-pyrido[2,1-*a*]isoquinoline-3-carboxylate (20 mg) as brown solid in accordance with a method similar to that in Step 1c of Example 1.

### Step 27c: Preparation of 6-tert-butyl-10-methoxy-9-[2-(3-methoxycyclobutyl)thiazol- 5-yl]-2-oxo-6,7-dihydro-2H-pyrido[2,1-a]isoquinoline-3-carboxylic acid

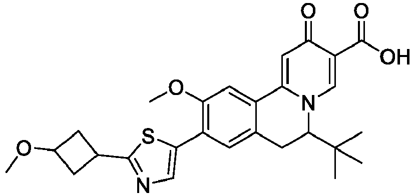

Ethyl 6-tert-butyl-10-methoxy-9-[2-(3-methoxycyclobutyl)thiazol-5-yl]-2-oxo-6,7-dihydro-2*H*-pyrido[2,1-*a*]isoquinoline-3-carboxylate (20 mg, 0.038 mmol) was used to prepare and afford 6-tert-butyl-10-methoxy-9-[2-(3-methoxycyclobutyl)thiazol-5-yl]- 2-oxo-6,7-dihydro-2*H*-pyrido[2,1-*a*]isoquinoline-3-carboxylic acid (5.1 mg) as yellow solid in accordance with a method similar to that in Step Id of Example 1. ¹HNMR (DMSO-*d₆*, 400 MHz) δ 16.56 (s, 1 H), 8.77 (s, 1 H), 7.90 (s, 1 H), 7.73 (s, 1 H), 7.63 (s, 1 H), 7.62 (s, 1 H), 4.61 (d, *J* = 6.0 Hz, 1 H), 4.00 (s, 3 H), 3.90 - 3.83 (m, 1 H), 3.38 - 3.34 (m, 2 H), 3.29 (s, 3 H), 2.74 - 2.68 (m, 2 H), 2.48 - 2.40 (m, 2 H), 0.75 (s, 9 H). MS measured value (ESI⁺) [(M+H)⁺]: 495.

The biological experiment data were specifically described below, so as to further illustrate the embodiments of the present disclosure.

### Materials and Methods

### HBV-producing cell line:

The full genome of HBV was inserted into an HepG2.2.15 cell, which could therefore express HBV continuously (Sells et al, Proc Natl Acad Sci U S A. 1987 Feb; 84(4): 1005-9). The culture condition was DMEM medium containing 10% serum and 400 µg/mg G418. Cells were placed in an incubator containing 5% CO₂ and cultured at 37°C.

Determination of hepatitis B surface antigen (HBsAg):
HepG2.2.15 cells were seeded into a 96-well culture plate at a density of 3 × 10⁴ cells/well. On the next day, the drug dissolved in DMSO was subjected to a 5-fold dilution and then added to the cells. 0.5% DMSO was added separately as control. The supernatant was collected after 4 days of drug action, and the content of hepatitis B surface antigen was determined.

The determination of hepatitis B surface antigen was carried out by using a kit for quantitative determination of hepatitis B virus surface antigen (chemiluminescence method). The specific operations were as follows. 50 µL cell culture supernatant was taken and transferred to the assay plate. 50 µL of enzyme-linked reagent was added thereto and the assay plate was sealed. Incubation was carried out at room temperature for 1 hour. Afterwards, the assay plate was washed six times with PBS, and finally, the residual liquid was removed by shaking the plate. 25 µL of substrates A and B were added respectively, and the determination was conducted by using a microplate reader (Tecan Infinite® F200) after ten minutes of reaction. IC₅₀ was calculated after fitting the dose-response curve.

### Determination results

The HBsAg inhibitory activities of the compounds involved in Example 1 to Example 27 of the present disclosure were determined according to the above-mentioned method, and the results were summarized in Table 3.

**Table 3: Data indicating the HBsAg inhibitory activities of the compounds of the Examples**

| Example | IC₅₀ (nM) | Example | IC₅₀ (nM) |
|---|---|---|---|
| 1 | 0.33 | 2 | 0.06 |
| 3 | 0.07 | 4 | 0.045 |
| 5 | 0.3 | 6 | 0.3 |
| 7 | 0.08 | 8 | 0.2 |
| 9 | 0.03 | 10 | 0.2 |
| 11 | 0.09 | 12 | 0.45 |
| 13 | 0.2 | 14 | 0.2 |
| 15 | 0.2 | 16 | 0.1 |
| 17 | 0.3 | 18 | 0.09 |
| 19 | 0.3 | 20 | 0.3 |
| 21 | 0.3 | 22 | 0.4 |
| 23 | 0.2 | 24 | 0.2 |
| 25 | 0.24 | 26 | 0.1 |
| 27 | 0.1 | | |

Determination of HBV DNA:
HepG2.2.15 cells were seeded into a 96-well culture plate at a density of 4 × 10⁴ cells/well. After 4 hours, the drug dissolved in DMSO was subjected to a 4-fold dilution and then added to cells. 0.5% DMSO was added separately as control. On the 4th day of drug action, the original supernatant was removed and then the drug was added thereto again. The supernatant was collected after a total of 7 days of drug action and treated with lysis buffer. The determination of hepatitis B DNA was carried out by real-time fluorescence-based quantitative polymerase chain reaction (qPCR). After the completion of the qPCR, data were exported from the instrument and analyzed, and IC₅₀ indicating antiviral activity was calculated after fitting the dose-response curve.

### Determination results

The inhibitory activities of the compounds involved in the present disclosure against HBV DNA were determined according to the above-mentioned method, and the results were summarized in Table 4.

**Table 4: Data indicating the inhibitory activities of the compounds of the Examples against HBV DNA**

| Example | IC₅₀ (nM) | Example | IC₅₀ (nM) |
|---|---|---|---|
| 2 | 0.11 | 4 | 0.26 |
| 7 | 0.15 | 9 | 0.10 |
| 10 | 0.13 | 11 | 0.13 |
| 13 | 0.31 | 16 | 0.41 |
| 18 | 0.06 | 23 | 0.84 |
| 24 | 0.45 | | |

Multiple compounds in the Examples of the present disclosure were administered to ICR mice to conduct pharmacokinetic experiments. A single intravenous injection (with a dose of 2 mg/kg) and a single oral administration (with a dose of 10 mg/kg) were administered respectively. Blood was respectively collected prior to administration and 5 minutes, 15 minutes, 30 minutes, 1 hour, 2 hours, 4 hours, 8 hours, 12 hours and 24 hours after administration, and the concentration of the test compound in plasma was determined. Plasma pharmacokinetic data of the control compound (obtained from Example 28 in patent WO2018130152 A1) and the compounds of Examples 10 and 19 in the present disclosure in mice were as shown in the following Table 5 to Table 7.

**Table 5: PK parameters of the control compound in mice**

| | Intravenous injection | Oral administration |
|---|---|---|
| Tₘₐₓ (hr) | 0.08 | 0.25 |
| Cmax (ng/mL) | 78.7 | 110.6 |
| AUC₀₋₁₂ (ng/mL^{∗}hr) | 102.8 | 203.2 |
| AUC_{INF} (ng/mL^{∗}hr) | 111.9 | 209.0 |
| t_{1/2} (hr) | 3.7 | 1.5 |
| CL (mL/hr/kg) | 17878.0 | / |
| Vss (mL/kg) | 71068.8 | / |
| F | 39.55% | |

**Table 6: PK parameters of the compound of Example 10 in mice**

| | Intravenous injection | Oral administration |
|---|---|---|
| Tₘₐₓ (hr) | 0.08 | 0.50 |
| Cₘₐₓ (ng/mL) | 423.3 | 458.0 |
| AUC₀₋₁₂ (ng/mL^{∗}hr) | 796.2 | 1348.0 |
| AUC_{INF} (ng/mL^{∗}hr) | 804.3 | 1395.8 |
| t_{1/2} (hr) | 1.9 | 2.6 |
| CL (mL/hr/kg) | 2486.6 | / |
| V_{SS} (mL/kg) | 5417.8 | / |
| F | 33.86% | |

**Table 7: PK parameters of the compound of Example 19 in mice**

| | Intravenous injection | Oral administration |
|---|---|---|
| Tₘₐₓ (hr) | 0.08 | 0.50 |
| Cₘₐₓ (ng/mL) | 1016.0 | 2423.3 |
| AUC₀₋₁₂ (ng/mL^{∗}hr) | 1196.9 | 5209.1 |
| AUC_{INF} (ng/mL^{∗}hr) | 1207.3 | 5289.8 |
| t_{1/2} (hr) | 1.3 | 2.5 |
| CL (mL/hr/kg) | 1656.6 | / |
| V_{SS} (mL/kg) | 2270.3 | / |
| F | 87.04% | |

As could be seen from the above tables, under the same dose, the plasma exposure of the compounds of Examples 10 and 19 in mice were significantly higher than that of the control compound after intravenous injection and oral administration. This result indicated that the compounds of the present disclosure could be administered at a lower dose for treating or preventing HBV infection as compared with the control compound.

Further, those skilled in the art understand that compounds as represented by general formula I mentioned above, different embodiments of compounds as represented by general formula I and all compounds involved in specific examples of compounds as represented by general formula I may be prepared into corresponding isomers, solvates, hydrates, prodrugs, stable isotopic derivatives and pharmaceutically acceptable salts. Preferably, said compounds are prepared into pharmaceutically acceptable derivatives, said derivative is any one of a prodrug, a salt, an ester, an amide, a salt of ester, a salt of amide, and a metabolite.

Further, pharmaceutically acceptable salts include conventional non-toxic salts obtained and formed by an inorganic acid (for example, hydrochloric acid, hydrobromic acid, hydroiodic acid, perchloric acid, sulfuric acid, phosphoric acid, or the like) or an organic acid (for example, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like) and any compound involved in the present disclosure. As for the review of suitable pharmaceutical salts, please refer to Berge S.M et al, J.Pharm.Sci. 1977, 66, 1-19; Gould P.L.Int.J.Pharm 1986, 33, 201-277 and Bighley et al, Encyclopedia of Pharmaceutical Technology, Marcel Dekker Inc, New York 1996, Vol 13, page 453-497. Pharmaceutically acceptable salts further include a salt formed by a compound of the present disclosure and an organic or inorganic base, including but not limited to alkali metal salts, such as lithium salts, sodium salts or potassium salts; alkaline earth metal salts, such as calcium salts or magnesium salts; salts of organic bases, such as ammonium salts formed by an organic base having nitrogen-containing group(s) or N⁺(C₁₋₆ alkyl)₄ salts. The organic or inorganic base includes, but not limited to, preferably lithium hydroxide, sodium hydroxide, potassium hydroxide, sodium carbonate, sodium bicarbonate, potassium carbonate, potassium bicarbonate, magnesium carbonate, calcium carbonate, ammonia water, triethylamine, tetrabutylammonium hydroxide, and the like.

Further, stable isotopic derivatives may be prepared by incorporating an isotope into any compound involved in the present disclosure, and the incorporated isotope may be ²H, ³H, ¹³C, ¹⁴C, ¹⁵N, ¹⁷O, ¹⁸O, ³¹P, ³²P, ³⁵S, ¹⁸F, or ³⁶Cl. Specific isotopic derivative may be prepared by conventional techniques.

Further, as an actual product, the compound may also be prepared into any one of a tablet, a capsule, an injection, a granule, a pulvis, a suppository, a pill, a cream, a paste, a gel, a powder, an oral solution, an inhalant, a suspension, a dry suspension, a patch, and a lotion.

Further, based on those mentioned above, the compound may also form a mixture with any one of the following substances, i.e., a pharmaceutically acceptable carrier, an adjuvant, or an excipient.

Further, based on those mentioned above, the compound may also form a composition with any one of the following substances, i.e., HBV polymerase inhibitors; interferonα-2a; interferonα-2b; pegylated interferon α-2a; ribavirin; HBV preventive vaccines; HBV therapeutic vaccines; HBV capsid inhibitors; HBV RNA replication inhibitors; siRNA; inhibitors for HBsAg production or secretion; HBV antibodies; TLR7 agonists.

All compounds involved in the present disclosure and mixtures, compositions and the like comprising the compounds of the present disclosure may be administered into an organism via any route of administration. The route of administration may be oral administration, intravenous injection, intramuscular injection, subcutaneous injection, rectal administration, vaginal administration, sublingual administration, nasal inhalation, oral inhalation, eye-drop administration, or local or systemic transdermal administration.

All compounds involved in the present disclosure and mixtures, compositions and the like comprising the compounds of the present disclosure may be prepared into a single dose, in which an active compound of the present disclosure, a carrier, an excipient and the like are contained. The dosage form may be a tablet, a capsule, an injection, a granule, a pulvis, a suppository, a pill, a cream, a paste, a gel, a powder, an oral solution, an inhalant, a suspension, a dry suspension, a patch, a lotion, or the like. These dosage forms may contain ingredients commonly used in a pharmaceutical preparation, such as a diluent, an absorbent, a wetting agent, a binder, a disintegrant, a colorant, a pH adjusting agent, an antioxidant, a bacteriostatic agent, an isotonic regulator, an antisticking agent, and the like.

Suitable formulations for various dosage forms mentioned above may be obtained from public sources, for example, Remington: The Science and Practice of Pharmacy, 21st edition, Lippincott Williams & Wilkins (published in 2006) and Rowe, Raymond C. Handbook of Pharmaceutical Excipients, Chicago, Pharmaceutical Press (published in 2005). Therefore, the dosage forms may be readily prepared by those skilled in the art.

Different dosages of administration may be selected according to factors such as the nature and extent of the diseases suffering by different individuals, the age, gender and body weight of the patients, and routes of administration. The administered dosage of the compound of the present disclosure may be 0.01 to 500 mg/kg/day, preferably, the daily dose is 1 to 100 mg/kg. The administration may be single or multiple administrations.

Those skilled in the art understand that, the typical uses of all compounds involved in the present disclosure and mixtures, compositions and the like comprising the compounds of the present disclosure are medical uses, in particular for preventing or treating hepatitis B virus infection. Specific indications are as follows.

The novel compound is capable of inhibiting the production or secretion of hepatitis B surface antigen (HBsAg), and this novel compound may be used for treating and preventing HBV infection.

The novel compound may be used for inhibiting the production or secretion of hepatitis B surface antigen (HBsAg).

The novel compound may be used for treating and preventing HBV infection.

Further, the compound of general formula I of the present disclosure may be used in combination with other drug(s), including HBV polymerase inhibitors, such as lamivudine, telbivudine, tenofovir disoproxil fumarate, adefovir dipivoxil, entecavir or tenofovir alafenamide fumarate; interferonα-2a; interferonα-2b; pegylated interferonα-2a; ribavirin; HBV preventive vaccines; HBV therapeutic vaccines; HBV capsid inhibitors; HBV RNA replication inhibitors; siRNA; inhibitors for HBsAg production or secretion; HBV antibodies; and TLR 7 agonists.

Specific examples of the present disclosure are described above. It needs to be understood that the present disclosure is not limited to the above specific embodiments, and those skilled in the art can make various variations or modifications within the scope of claims, which does not affect the essential contents of the present disclosure.

## Claims

1. A compound of general formula I, a pharmaceutically acceptable salt thereof, an ester thereof, an isomer thereof, a solvate thereof, a hydrate thereof, a prodrug thereof, or an isotopically-labeled compound thereof, the compound of general formula I having a structure as follows: 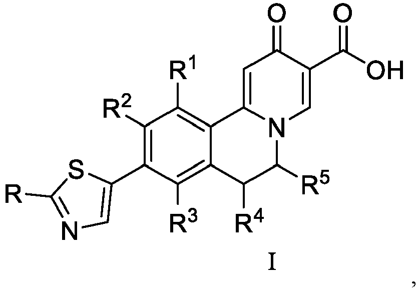 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one fluorine, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one fluorine, and C₃₋₇ cycloalkyl; and
R is a substituted or unsubstituted group, said group is C₁₋₆ alkyl, amino, C₁₋₆ alkoxy, C₃₋₇ cycloalkoxy, C₆₋₁₀ aryloxy, C₃₋₇ cycloalkyl, 3- to 7-membered heterocycloalkyl, C₆₋₁₀ aryl, C₅₋₁₀ heteroaryl, or carbamoyl, and R is not an unsubstituted methyl.

2. The compound of general formula I, the pharmaceutically acceptable salt thereof, the ester thereof, the isomer thereof, the solvate thereof, the hydrate thereof, the prodrug thereof, or the isotopically-labeled compound thereof of claim 1, wherein said R is a group with substitution, said group is C₁₋₆ alkyl, amino, C₁₋₆ alkoxy, C₃₋₇ cycloalkoxy, C₆₋₁₀ aryloxy, C₃₋₇ cycloalkyl, 3-to 7-membered heterocycloalkyl, C₆₋₁₀ aryl, C₅₋₁₀ heteroaryl, or carbamoyl, and said substitution refers to being substituted with one or more of the following groups: hydroxy, hydroxy-C₁₋₆ alkylene, halogen, C₁₋₆ alkyl, C₁₋₆ alkoxy, C₃₋₇ cycloalkoxy, C₁₋₆ alkoxy-C₁₋₆ alkylene, and C₁₋₆ alkoxy substituted with at least one halogen atom.

3. The compound of general formula I, the pharmaceutically acceptable salt thereof, the ester thereof, the isomer thereof, the solvate thereof, the hydrate thereof, the prodrug thereof, or the isotopically-labeled compound thereof of claim 1 or 2, wherein said R is a group with substitution, said group is C₁₋₆ alkyl, amino, C₁₋₆ alkoxy, C₃₋₇ cycloalkoxy, C₃₋₇ cycloalkyl, 3- to 7-membered heterocycloalkyl, C₅₋₁₀ heteroaryl, or carbamoyl, and said substitution refers to being substituted with one or more of the following groups: hydroxy, hydroxy-C₁₋₆ alkylene, halogen, C₁₋₃ alkyl, C₁₋₃ alkoxy, C₁₋₃ alkoxy-C₁₋₆ alkylene, C₃₋₇ cycloalkoxy, and C₁₋₃ alkoxy substituted with at least one halogen atom.

4. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein the compound of general formula I has a structure of general formula 1-1: 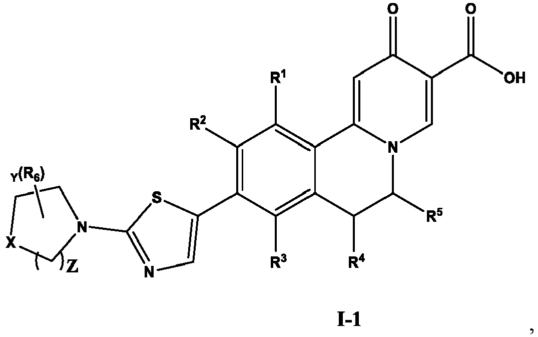 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl;
X is O, N, S, SO, SO₂, or C(R₆)₂;
Y is 0, 1,2, 3, 4 or 5;
Z is 0, 1 or 2; and
each R₆ is independently selected from hydrogen, deuterium, hydroxy, cyano, amino, C₁₋₆ alkylamino, halogen, C₁₋₆ alkyl, C₁₋₆ alkyl substituted with at least one halogen atom, C₁₋₆ alkyl substituted with hydroxy, or C₁₋₆ alkoxy.

5. The compound of general formula I, the pharmaceutically acceptable salt thereof, the ester thereof, the isomer thereof, the solvate thereof, the hydrate thereof, the prodrug thereof, or the isotopically-labeled compound thereof of claim 4, wherein
X is O or C(R₆)₂;
Y is 0, 1 or 2; and
R₆ is independently selected from hydrogen, deuterium, hydroxy, cyano, amino, methylamino, ethylamino, fluorine, chlorine, methyl, ethyl, n-propyl, isopropyl, methoxy, ethoxy, and methyl, ethyl, n-propyl and isopropyl substituted with at least one fluorine, chlorine or hydroxy.

6. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein the compound of general formula I has a structure of general formula 1-2: 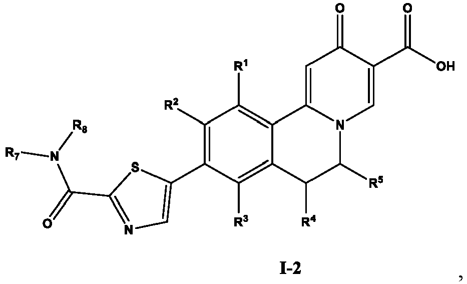 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl; and
R₇ and R₈ are each independently selected from hydrogen, deuterium, C₁₋₆ alkyl, and C₁₋₆ alkyl substituted with at least one halogen atom, C₁₋₆ alkoxy or hydroxy.

7. The compound of general formula I, the pharmaceutically acceptable salt thereof, the ester thereof, the isomer thereof, the solvate thereof, the hydrate thereof, the prodrug thereof, or the isotopically-labeled compound thereof of claim 6, wherein said R₇ and R₈ are each independently selected from hydrogen, deuterium, methyl, ethyl, n-propyl, isopropyl, methoxyethyl, and methyl, ethyl, n-propyl and isopropyl substituted with at least one fluorine, chlorine or hydroxy.

8. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein the compound of general formula I has a structure of general formula 1-3: 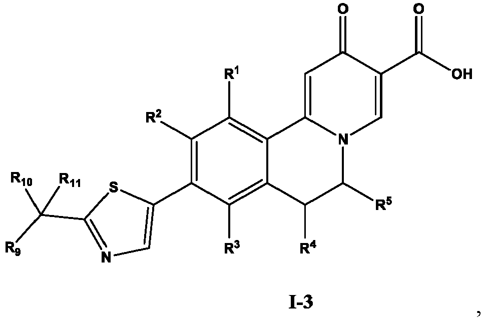 wherein
R¹ is any one of hydrogen, deuterium, halogen, C₁₋₆ alkyl, C₁₋₆ alkylamino, and C₁₋₆ alkoxy;
R² is any one of hydrogen, deuterium, halogen, C₁₋₆ alkyl, C₁₋₆ alkyl substituted with at least one halogen, C₃₋₇ cycloalkyl, C₁₋₆ alkylamino, C₁₋₆ alkoxy, and heterocycloalkyl;
R³ is any one of hydrogen, deuterium, halogen, cyano, C₁₋₆ alkyl, C₃₋₇ cycloalkyl, C₁₋₆ alkylamino, C₁₋₆ alkoxy, and heterocycloalkyl;
R⁴ is any one of hydrogen, deuterium, and C₁₋₆ alkyl;
R⁵ is any one of hydrogen, deuterium, C₁₋₆ alkyl, C₁₋₆ alkyl substituted with at least one halogen, and C₃₋₇ cycloalkyl;
R₉, R₁₀ and R₁₁ are each independently selected from hydrogen, deuterium, C₁₋₆ alkyl, hydroxy, C₁₋₆ alkyl substituted with at least one halogen atom or hydroxy, C₁₋₆ alkoxy unsubstituted or substituted with at least one halogen atom, and C₃₋₇ cycloalkoxy unsubstituted or substituted with at least one halogen atom or C₁₋₆ alkyl, or any two of R₉, R₁₀ and R₁₁ may form a substituted or unsubstituted C₃₋₇ cycloalkyl, and R₉, R₁₀ and R₁₁ are not hydrogen at the same time.

9. The compound of general formula I, the pharmaceutically acceptable salt thereof, the ester thereof, the isomer thereof, the solvate thereof, the hydrate thereof, the prodrug thereof, or the isotopically-labeled compound thereof of claim 8, wherein R₉, R₁₀ and R₁₁ are each independently selected from hydrogen, deuterium, methyl, ethyl, n-propyl, isopropyl, hydroxy, methoxy, ethoxy, cyclopropoxy, and methyl, ethyl, n-propyl, isopropyl or C₁₋₃ alkoxy substituted with at least one fluorine, chlorine or hydroxy, and the substituted C₃₋₇ cycloalkyl refers to the one substituted with at least one halogen atom, C₁₋₆ alkyl or C₁₋₆ alkoxy.

10. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein said R is a substituted or unsubstituted group, said group is piperidinyl, pyrrolidinyl, azetidinyl, morpholinyl, or C₁₋₃ alkyl.

11. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein said R is a substituted or unsubstituted group, said group is piperidinyl, azetidinyl, C₁₋₆ alkoxy, cyclobutyl, propyl, C₁₋₃ alkyl, or carbamoyl.

12. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein said R is substituted or unsubstituted piperidin-1-yl, substituted or unsubstituted azetidin-1-yl, cyclopropyl, substituted or unsubstituted cyclobutyl, propyl, or methyl or ethyl substituted with C₁₋₆ alkoxy, wherein the substituted piperidin-1-yl refers to the one substituted with at least one hydroxy-C₁₋₆ alkylene, C₁₋₆ alkyl or hydroxy, preferably substituted with hydroxy-C₁₋₆ alkylene or substituted with both C₁₋₆ alkyl and hydroxy; the substituted azetidin-1-yl refers to the one substituted with at least one halogen atom or C₁₋₆ alkoxy; and the substituted cyclobutyl refers to the one substituted with at least one halogen atom, C₁₋₆ alkyl or C₁₋₆ alkoxy.

13. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein said R is substituted piperidin-1-yl, the substituted piperidin-1-yl refers to the one substituted with at least one hydroxy-C₁₋₃ alkylene, C₁₋₆ alkoxy, C₁₋₃ alkyl or hydroxy, preferably substituted with hydroxy-C₁₋₃ alkylene or substituted with both C₁₋₃ alkyl and hydroxy.

14. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein said R is substituted or unsubstituted azetidin-1-yl, the substituted azetidin-1-yl refers to the one substituted with 1 to 3 halogen atoms or C₁₋₃ alkoxy.

15. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein said R is methyl, ethyl or isopropyl substituted with C₁₋₆ alkoxy, preferably methyl, ethyl or isopropyl substituted with methoxy, ethoxy, propoxy or isopropoxy.

16. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 15, wherein
R¹ is any one of hydrogen, deuterium, fluorine, chlorine, bromine, methyl, ethyl, isopropyl, tert-butyl, methylamino, ethylamino, methoxy, ethoxy, and isopropoxy;
R² is any one of hydrogen, deuterium, fluorine, chlorine, bromine, methyl, ethyl, isopropyl, tert-butyl, trifluoromethyl, trifluoromethylmethyl, cyclopropyl, cyclopentyl, methylamino, ethylamino, methoxy, ethoxy, isopropoxy, pyrrolidinyl, and morpholinyl;
R³ is any one of hydrogen, deuterium, fluorine, chlorine, bromine, methyl, ethyl, and cyano;
R⁴ is any one of hydrogen, deuterium, methyl, and ethyl; and
R⁵ is any one of hydrogen, deuterium, methyl, ethyl, isopropyl, butyl, sec-butyl, isobutyl, tert-butyl, trifluoromethyl, trifluoromethylmethyl, and cyclopropyl.

17. The compound of general formula I, the pharmaceutically acceptable salt thereof, the ester thereof, the isomer thereof, the solvate thereof, the hydrate thereof, the prodrug thereof, or the isotopically-labeled compound thereof of claim 16, wherein
R¹ is any one of hydrogen, deuterium, halogen, and C₁₋₆ alkoxy;
R² is any one of hydrogen, deuterium, halogen, C₁₋₆ alkylamino, and C₁₋₆ alkoxy;
R³ is any one of hydrogen, deuterium, and halogen;
R⁴ is hydrogen or deuterium; and
R⁵ is any one of hydrogen, deuterium, C₁₋₆ alkyl, C₁₋₆ alkyl substituted with at least one fluorine, and C₃₋₇ cycloalkyl.

18. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 3, wherein
R¹ is hydrogen or deuterium;
R² is any one of hydrogen, deuterium, halogen, and C₁₋₆ alkoxy;
R³ is hydrogen or deuterium;
R⁴ is hydrogen or deuterium;
R⁵ is any one of hydrogen, deuterium, methyl, ethyl, isopropyl, butyl, sec-butyl, isobutyl, tert-butyl, trifluoromethyl, trifluoromethylmethyl, and cyclopropyl;
R is a group with or without substitution, said group is piperidinyl, azetidinyl, morpholinyl, amino, cyclopropyl, propyl, C₁₋₃ alkyl, C₁₋₃ alkoxy, cyclobutyl, or carbamoyl, and said substitution refers to being substituted with one or more of the following groups: hydroxy, hydroxy-C₁₋₆ alkylene, halogen, C₁₋₃ alkyl, C₁₋₃ alkoxy, C₁₋₃ alkoxy-C₁₋₆ alkylene, C₁₋₃ alkoxy substituted with at least one halogen atom, or C₃₋₇ cycloalkoxy.

19. The compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 18, wherein the compound of general formula I is any one of the following compounds:
6-tert-butyl-9-[2-(4-hydroxypiperidin-1-yl)thiazol-5-yl]-10-methoxy-2-oxo-6,7-dihydro-2*H*-pyrido[2,1-*a*]isoquinoline-3-carboxylic acid;
6-tert-butyl-9-{2-[4-(hydroxymethyl)piperidin-1-yl]thiazol-5-yl}-10-methoxy-2-oxo-6,7-dihydro-2*H*-pyrido[2,1-*a*]isoquinoline-3-carboxylic acid;
9-[2-(azetidin-1-yl)thiazol-5-yl]-6-tert-butyl-10-methoxy-2-oxo-6,7-dihydro-2*H-*pyrido[2,1-*a*]isoquinoline-3-carboxylic acid;
6-tert-butyl-9-[2-(3,3-difluoroazetidin-1-yl)thiazol-5-yl]-10-methoxy-2-oxo-6,7-dihydro-2*H*-pyrido[2,1-*a*]isoquinoline-3-carboxylic acid;
6-tert-butyl-10-methoxy-9-(2-morpholinothiazol-5-yl)-2-oxo-6,7-dihydro-2*H*-pyrido[2,1-*a*]isoquinoline-3-carboxylic acid;
6-tert-butyl-9-[2-(dimethylamino)thiazol-5-yl]-10-methoxy-2-oxo-6,7-dihydro-2*H-*pyrido[2,1-*a*]isoquinoline-3-carboxylic acid;
6-tert-butyl-9-[2-(4-hydroxy-4-methylpiperidin-1-yl)thiazol-5-yl]-10-methoxy-2-oxo-6,7-dihydro-2*H*-pyrido[2,1-*a*]isoquinoline-3-carboxylic acid;
6-tert-butyl-10-methoxy-9-[2-(4-methoxypiperidin-1-yl)thiazol-5-yl]-2-oxo-6,7-dihydro-2*H*-pyrido[2,1-*a*]isoquinoline-3-carboxylic acid;
6-tert-butyl-10-methoxy-9-[2-(3-methoxyazetidin-1-yl)thiazol-5-yl]-2-oxo-6,7-dihydro-2*H*-pyrido[2,1-*a*]isoquinoline-3-carboxylic acid;
6-tert-butyl-10-methoxy-9-[2-(methoxymethyl)thiazol-5-yl]-2-oxo-6,7-dihydro-2*H-*pyrido[2,1-*a*]isoquinoline-3-carboxylic acid;
6-tert-butyl-9-[2-(3-fluoroazetidin-1-yl)thiazol-5-yl]-10-methoxy-2-oxo-6,7-dihydro-2*H-*pyrido[2,1-*a*]isoquinoline-3-carboxylic acid;
6-tert-butyl-10-methoxy-9-(2-methoxythiazol-5-yl)-2-oxo-6,7-dihydro-2*H*-pyrido[2,1-*a*]isoquinoline-3-carboxylic acid;
6-tert-butyl-9-[2-(ethoxy)thiazol-5-yl]-10-methoxy-2-oxo-6,7-dihydro-2*H*-pyrido[2,1-*a*]isoquinoline-3-carboxylic acid;
6-tert-butyl-9-[2-(ethoxymethyl)thiazol-5-yl]-10-methoxy-2-oxo-6,7-dihydro-2*H-*pyrido[2,1-*a*]isoquinoline-3-carboxylic acid;
6-tert-butyl-10-methoxy-9-[2-(1-methoxyethyl)thiazol-5-yl]-2-oxo-6,7-dihydro-2*H-*pyrido[2,1-*a*]isoquinoline-3-carboxylic acid;
6-tert-butyl-9-[2-(1-hydroxyethyl)thiazol-5-yl]-10-methoxy-2-oxo-6,7-dihydro-2*H-*pyrido[2,1-*a*]isoquinoline-3-carboxylic acid;
6-tert-butyl-10-methoxy-9-[2-(2-methoxypropan-2-yl)thiazol-5-yl]-2-oxo-6,7-dihydro-2*H*-pyrido[2,1-*a*]isoquinoline-3-carboxylic acid;
6-tert-butyl-10-methoxy-2-oxo-9-(2-propylthiazol-5-yl)-6,7-dihydro-2*H*-pyrido[2,1-*a*] isoquinoline-3-carboxylic acid;
6-tert-butyl-10-methoxy-9-[2-(methylcarbamoyl)thiazol-5-yl]-2-oxo-6,7-dihydro-2*H-*pyrido[2,1-*a*]isoquinoline-3-carboxylic acid;
6-tert-butyl-9-[2-(ethylcarbamoyl)thiazol-5-yl]-10-methoxy-2-oxo-6,7-dihydro-2*H-*pyrido[2,1-*a*]isoquinoline-3-carboxylic acid;
6-tert-butyl-10-methoxy-9-[2-(2-methoxyethoxy)thiazol-5-yl]-2-oxo-6,7-dihydro-2*H-*pyrido[2,1-*a*]isoquinoline-3-carboxylic acid;
6-tert-butyl-10-methoxy-9-{2-[(2-methoxyethyl)carbamoyl]thiazol-5-yl}-2-oxo-6,7-dihydro-2*H*-pyrido[2,1-*a*]isoquinoline-3-carboxylic acid;
6-tert-butyl-9-{2-[(difluoromethoxy)methyl]thiazol-5-yl}-10-methoxy-2-oxo-6,7-dihydro-2*H*-pyrido[2,1-*a*]isoquinoline-3-carboxylic acid;
6-tert-butyl-9-[2-(cyclopropoxymethyl)thiazol-5-yl]-10-methoxy-2-oxo-6,7-dihydro-2*H-*pyrido[2,1-*a*]isoquinoline-3-carboxylic acid;
6-tert-butyl-9-[2-(3,3-difluorocyclobutyl)thiazol-5-yl}-10-methoxy-2-oxo-6,7-dihydro-2*H*-pyrido[2,1-*a*]isoquinoline-3-carboxylic acid;
6-tert-butyl-9-[2-(3-fluorocyclobutyl)thiazol-5-yl]-10-methoxy-2-oxo-6,7-dihydro-2*H-*pyrido[2,1-*a*]isoquinoline-3-carboxylic acid; and
6-tert-butyl-10-methoxy-9-[2-(3-methoxycyclobutyl)thiazol-5-yl]-2-oxo-6,7-dihydro-2*H-*pyrido[2,1-*a*]isoquinoline-3-carboxylic acid.

20. A pharmaceutical composition, comprising the compound of general formula I, the pharmaceutically acceptable salt thereof, the ester thereof, the isomer thereof, the solvate thereof, the hydrate thereof, the prodrug thereof, or the isotopically-labeled compound thereof of any one of claims 1 to 19.

21. A pharmaceutical formulation, comprising the compound of general formula I, a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, wherein the formulation is any one of a tablet, a capsule, an injection, a granule, a pulvis, a suppository, a pill, a cream, a paste, a gel, a powder, an oral solution, an inhalant, a suspension, a dry suspension, a patch, and a lotion.

22. The compound of general formula I, a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, or the pharmaceutical formulation of claim 21, which is used for preventing and treating viral hepatitis B.

23. The compound of general formula I, a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, or the pharmaceutical formulation of claim 21 for use in the prevention and/or treatment of viral hepatitis B.

24. Use of the compound of general formula I, a pharmaceutically acceptable salt thereof, an ester thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, or the pharmaceutical formulation of claim 21 in the preparation of a drug for preventing and/or treating viral hepatitis B.

25. A method for preventing and/or treating viral hepatitis B, comprising the following step of administering a therapeutically effective amount of the compound of general formula I, a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, or the pharmaceutical formulation of claim 21 to a patient in need thereof.

26. A pharmaceutical combination, comprising the compound of general formula I, a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, or the pharmaceutical formulation of claim 21, and at least one additional therapeutic agent for viral hepatitis B.

27. A method for preventing and/or treating viral hepatitis B, comprising the following step of administering a therapeutically effective amount of the compound of general formula I, a pharmaceutically acceptable salt thereof, a hydrate thereof, a solvate thereof, a stereoisomer thereof, a tautomer thereof, a cis/trans isomer thereof, an isotopically-labeled compound thereof, or a prodrug thereof of any one of claims 1 to 19, or the pharmaceutical composition of claim 20, or the pharmaceutical formulation of claim 21, and at least one additional therapeutic agent for viral hepatitis B to a patient in need thereof.
